# EUROPEAN PATENT SPECIFICATION

(11) **EP 1 947 185 B1**
(45) Date of publication and mention of the grant of the patent: **20.07.2011**
(21) Application number: 07020248.6
(22) Date of filing: 15.08.2001
(51) Int. Cl.: C12N 15/40, C07K 14/18, C12Q 1/68, C12Q 1/70, G01N 33/576, A61K 39/29, C12P 21/00

(54) **HCV NS3/4A encoding nucleic acid**
HCV NS3/4A kodierende Nukleinsäure
Acide nucléique codant pour HCV NS3/4A

(30) Priority: 17.08.2000 US 225767 P; 29.08.2000 US 229175 P; 03.11.2000 US 705547
(43) Date of publication of application: 23.07.2008
(62) Divisional of application: 01972379.0
(73) Proprietor: TRIPEP AB, 141 57 Huddinge (SE)
(72) Inventor: Sallberg, Matti, 125 33 Alvsjo (SE); Hultgren, Catharina, 117 34 Stockholm (SE)
(74) Representative: Fleuchaus, Andrea

(56) References cited:
- EP-A- 0 318 216
- WO-A-98/37180
- WO-A-99/04008
- DATABASE EMBL 8 November 1999 (1999-11-08), "HCV J1 NS3-NS4 DOMAIN C200 REGION CONSENSUS DNA SEQUENCE" XP002483281 Database accession no. AAZ07660 & EP 0 939 128 A (OYA AKIRA DR DIRECTOR GENERAL [JP]; CHIRON CORP [US] OYA AKIRA DR [JP]) 1 September 1999 (1999-09-01)
- DATABASE EMBL 16 August 2000 (2000-08-16), "HEPATITIS C VIRUS GENOMIC RNA FOR POLYPROTEIN GENE" XP002483282 Database accession no. AJ278830 & JOURNAL OF VIRAL HEPATITIS, vol. 7, November 2000 (2000-11), pages 459-465,
- DATABASE EMBL 11 February 2000 (2000-02-11), "NUCLEOTIDE SEQUENCE OF THE MTNFH6NS3 CLONE B9 FUSION PROTEIN" XP002483283 & WO 99/54735 A (INNOGENETICS NV [BE]; MAERTENS GEERT [BE]; LOUWAGIE JOOST [BE]; BOSMAN) 28 October 1999 (1999-10-28)

### SEQUENCE LISTING

<110> TRIPEP AB
   Matti SALLBERG
   Catharina HULTGREN
<120> VACCINES CONTAINING RIBAVIRIN AND METHODS OF USE THEREOF
<130> TRIPEP.023VPC
<150> US 09/705,547
   <151> 2000-11-03
<150> US 60/229,175
   <151> 2000-08-29
<150> US 60/225,767
   <151> 2000-08-17
<160> 49
<170> FastSEQ for Windows Version 4.0
<210> 1
   <211> 3011
   <212> PRT
   <213> Artificial Sequence
<220>
   <223> Hepatitis C virus sequence
<400> 1
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Thr Trp Ala Gln Pro Gly
   65 70 75 80
   Tyr Pro Trp Pro Leu Tyr Gly Asn Glu Gly Cys Gly Trp Ala Gly Trp
   85 90 95
   Leu Leu Ser Pro Arg Gly Ser Arg Pro Ser Trp Gly Pro Thr Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
   130 135 140
   Gly Gly Ala Ala Arg Ala Leu Ala His Gly Val Arg Val Leu Glu Asp
   145 150 155 160
   Gly Val Asn Tyr Ala Thr Gly Asn Leu Pro Gly Cys Ser Phe Ser Ile
                   165                 170                 175
   Phe Leu Leu Ala Leu Leu Ser Cys Leu Thr Val Pro Ala Ser Ala Tyr
               180                 185                 190
   Gln Val Arg Asn Ser Ser Gly Leu Tyr His Val Thr Asn Asp Cys Pro
           195                 200                 205
   Asn Ser Ser Val Val Tyr Glu Ala Ala Asp Ala Ile Leu His Thr Pro
       210                 215                 220
   Gly Cys Val Pro Cys Val Arg Glu Gly Asn Ala Ser Arg Cys Trp Val
   225                 230                 235                 240
   Ala Val Thr Pro Thr Val Ala Thr Arg Asp Gly Lys Leu Pro Thr Thr
                   245                 250                 255
   Gln Leu Arg Arg His Ile Asp Leu Leu Val Gly Ser Ala Thr Leu Cys
               260                 265                 270
   Ser Ala Leu Tyr Val Gly Asp Leu Cys Gly Ser Val Phe Leu Val Gly
           275                 280                 285
   Gln Leu Phe Thr Phe Ser Pro Arg His His Trp Thr Thr Gln Asp Cys
       290                 295                 300
   Asn Cys Ser Ile Tyr Pro Gly His Ile Thr Gly His Arg Met Ala Trp
   305                 310                 315                 320
   Asn Met Met Met Asn Trp Ser Pro Thr Ala Ala Leu Val Val Ala Gln
                   325                 330                 335
   Leu Leu Arg Ile Pro Gln Ala Ile Met Asp Met Ile Ala Gly Ala His
               340                 345                 350
   Trp Gly Val Leu Ala Gly Ile Lys Tyr Phe Ser Met Val Gly Asn Trp
           355                 360                 365
   Ala Lys Val Leu Val Val Leu Leu Leu Phe Ala Gly Val Asp Ala Glu
       370                 375                 380
   Thr His Val Thr Gly Gly Asn Ala Gly Arg Thr Thr Ala Gly Leu Val
   385                 390                 395                 400
   Gly Leu Leu Thr Pro Gly Ala Lys Gln Asn Ile Gln Leu Ile Asn Thr
                   405                 410                 415
   Asn Gly Ser Trp His Ile Asn Ser Thr Ala Leu Asn Cys Asn Glu Ser
               420                 425                 430
   Leu Asn Thr Gly Trp Leu Ala Gly Leu Phe Tyr Gln His Lys Phe Asn
           435                 440                 445
   Ser Ser Gly Cys Pro Glu Arg Leu Ala Ser Cys Arg Arg Leu Thr Asp
       450                 455                 460
   Phe Ala Gln Gly Trp Gly Pro Ile Ser Tyr Ala Asn Gly Ser Gly Leu
   465                 470                 475                 480
   Asp Glu Arg Pro Tyr Cys Trp His Tyr Pro Pro Arg Pro Cys Gly Ile
                   485                 490                 495
   Val Pro Ala Lys Ser Val Cys Gly Pro Val Tyr Cys Phe Thr Pro Ser
               500                 505                 510
   Pro Val Val Val Gly Thr Thr Asp Arg Ser Gly Ala Pro Thr Tyr Ser
           515                 520                 525
   Trp Gly Ala Asn Asp Thr Asp Val Phe Val Leu Asn Asn Thr Arg Pro
       530                 535                 540
   Pro Leu Gly Asn Trp Phe Gly Cys Thr Trp Met Asn Ser Thr Gly Phe
   545                 550                 555                 560
   Thr Lys Val Cys Gly Ala Pro Pro Cys Val Ile Gly Gly Val Gly Asn
                   565                 570                 575
   Asn Thr Leu Leu Cys Pro Thr Asp Cys Phe Arg Lys Tyr Pro Glu Ala
               580                 585                 590
   Thr Tyr Ser Arg Cys Gly Ser Gly Pro Arg Ile Thr Pro Arg Cys Met
               595                 600                 605
   Val Asp Tyr Pro Tyr Arg Leu Trp His Tyr Pro Cys Thr Ile Asn Tyr
       610                 615                 620
   Thr Ile Phe Lys Val Arg Met Tyr Val Gly Gly Val Glu His Arg Leu
   625                 630                 635                 640
   Glu Ala Ala Cys Asn Trp Thr Arg Gly Glu Arg Cys Asp Leu Glu Asp
                   645                 650                 655
   Arg Asp Arg Ser Glu Leu Ser Pro Leu Leu Leu Ser Thr Thr Gln Trp
               660                 665                 670
   Gln Val Leu Pro Cys Ser Phe Thr Thr Leu Pro Ala Leu Ser Thr Gly
           675                 680                 685
   Leu Ile His Leu His Gln Asn Ile Val Asp Val Gln Tyr Leu Tyr Gly
       690                 695                 700
   Val Gly Ser Ser Ile Ala Ser Trp Ala Ile Lys Trp Glu Tyr Val Val
   705                 710                 715                 720
   Leu Leu Phe Leu Leu Leu Ala Asp Ala Arg Val Cys Ser Cys Leu Trp
                   725                 730                 735
   Met Met Leu Leu Ile Ser Gln Ala Glu Ala Ala Leu Glu Asn Leu Val
               740                 745                 750
   Ile Leu Asn Ala Ala Ser Leu Ala Gly Thr His Gly Leu Val Ser Phe
           755                 760                 765
   Leu Val Phe Phe Cys Phe Ala Trp Tyr Leu Lys Gly Arg Trp Val Pro
       770                 775                 780
   Gly Ala Val Tyr Ala Leu Tyr Gly Met Trp Pro Leu Leu Leu Leu Leu
   785                 790                 795                 800
   Leu Ala Leu Pro Gln Arg Ala Tyr Ala Leu Asp Thr Glu Val Ala Ala
                   805                 810                 815
   Ser Cys Gly Gly Val Val Leu Val Gly Leu Met Ala Leu Thr Leu Ser
               820                 825                 830
   Pro Tyr Tyr Lys Arg Tyr Ile Ser Trp Cys Met Trp Trp Leu Gln Tyr
           835                 840                 845
   Phe Leu Thr Arg Val Glu Ala Gln Leu His Val Trp Val Pro Pro Leu
       850                 855                 860
   Asn Val Arg Gly Gly Arg Asp Ala Val Ile Leu Leu Thr Cys Val Val
   865                 870                 875                 880
   His Pro Ala Leu Val Phe Asp Ile Thr Lys Leu Leu Leu Ala Ile Phe
                   885                 890                 895
   Gly Pro Leu Trp Ile Leu Gln Ala Ser Leu Leu Lys Val Pro Tyr Phe
               900                 905                 910
   Val Arg Val Gln Gly Leu Leu Arg Ile Cys Ala Leu Ala Arg Lys Ile
           915                 920                 925
   Ala Gly Gly His Tyr Val Gln Met Ala Ile Ile Lys Leu Gly Ala Leu
       930                 935                 940
   Thr Gly Thr Cys Val Tyr Asn His Leu Ala Pro Leu Arg Asp Trp Ala
   945                 950                 955                 960
   His Asn Gly Leu Arg Asp Leu Ala Val Ala Val Glu Pro Val Val Phe
                   965                 970                 975
   Ser Arg Met Glu Thr Lys Leu Ile Thr Trp Gly Ala Asp Thr Ala Ala
               980                 985                 990
   Cys Gly Asp Ile Ile Asn Gly Leu Pro Val Ser Ala Arg Arg Gly Gln
           995                 1000                1005
   Glu Ile Leu Leu Gly Pro Ala Asp Gly Met Val Ser Lys Gly Trp Arg
       1010                1015                1020
   Leu Leu Ala Pro Ile Thr Ala Tyr Ala Gln Gln Thr Arg Gly Leu Leu
   1025                1030                1035                1040
   Gly Cys Ile Ile Thr Ser Leu Thr Gly Arg Asp Lys Asn Gln Val Glu
   1045 1050 1055
   Gly Glu Val Gln Ile Val Ser Thr Ala Thr Gln Thr Phe Leu Ala Thr
   1060 1065 1070
   Cys Ile Asn Gly Val Cys Trp Thr Val Tyr His Gly Ala Gly Thr Arg
   1075 1080 1085
   Thr Ile Ala Ser Pro Lys Gly Pro Val Ile Gln Thr Tyr Thr Asn Val
   1090 1095 1100
   Asp Gln Asp Leu Val Gly Trp Pro Ala Pro Gln Gly Ser Arg Ser Leu
   1105 1110 1115 1120
   Thr Pro Cys Thr Cys Gly Ser Ser Asp Leu Tyr Leu Val Thr Arg His
   1125 1130 1135
   Ala Asp Val Ile Pro Val Arg Arg Arg Gly Asp Ser Arg Gly Ser Leu
   1140 1145 1150
   Leu Ser Pro Arg Pro Ile Ser Tyr Leu Lys Gly Ser Ser Gly Gly Pro
   1155 1160 1165
   Leu Leu Cys Pro Thr Gly His Ala Val Gly Leu Phe Arg Ala Ala Val
   1170 1175 1180
   Cys Thr Arg Gly Val Ala Lys Ala Val Asp Phe Ile Pro Val Glu Asn
   1185 1190 1195 1200
   Leu Glu Thr Thr Met Arg Ser Pro Val Phe Thr Asp Asn Ser Ser Pro
   1205 1210 1215
   Pro Ala Val Pro Gln Ser Phe Gln Val Ala His Leu His Ala Pro Thr
   1220 1225 1230
   Gly Ser Gly Lys Ser Thr Lys Val Pro Ala Ala Tyr Ala Ala Lys Gly
   1235 1240 1245
   Tyr Lys Val Leu Val Leu Asn Pro Ser Val Ala Ala Thr Leu Gly Phe
   1250 1255 1260
   Gly Ala Tyr Met Ser Lys Ala His Gly Val Asp Pro Asn Ile Arg Thr
   1265 1270 1275 1280
   Gly Val Arg Thr Ile Thr Thr Gly Ser Pro Ile Thr Tyr Ser Thr Tyr
   1285 1290 1295
   Gly Lys Phe Leu Ala Asp Ala Gly Cys Ser Gly Gly Ala Tyr Asp Ile
   1300 1305 1310
   Ile Ile Cys Asp Glu Cys His Ser Thr Asp Ala Thr Ser Ile Ser Gly
   1315 1320 1325
   Ile Gly Thr Val Leu Asp Gln Ala Glu Thr Ala Gly Ala Arg Leu Val
   1330 1335 1340
   Val Leu Ala Thr Ala Thr Pro Pro Gly Ser Val Thr Val Ser His Pro
   1345 1350 1355 1360
   Asn Ile Glu Glu Val Ala Leu Ser Thr Thr Gly Glu Ile Pro Phe Tyr
   1365 1370 1375
   Gly Lys Ala Ile Pro Leu Glu Val Ile Lys Gly Gly Arg His Leu Ile
   1380 1385 1390
   Phe Cys His Ser Lys Lys Lys Cys Asp Glu Leu Ala Ala Lys Leu Val
   1395 1400 1405
   Ala Leu Gly Ile Asn Ala Val Ala Tyr Tyr Arg Gly Leu Asp Val Ser
   1410 1415 1420
   Val Ile Pro Thr Ser Gly Asp Val Val Val Val Ser Thr Asp Ala Leu
   1425 1430 1435 1440
   Met Thr Gly Phe Thr Gly Asp Phe Asp Ser Val Ile Asp Cys Asn Thr
   1445 1450 1455
   Cys Val Thr Gln Thr Val Asp Phe Ser Leu Asp Pro Thr Phe Thr Ile
   1460 1465 1470
   Glu Thr Thr Thr Leu Pro Gln Asp Ala Val Ser Arg Thr Gln Arg Arg
   1475 1480 1485
   Gly Arg Thr Gly Arg Gly Lys Pro Gly Ile Tyr Arg Phe Val Ala Pro
   1490 1495 1500
   Gly Glu Arg Pro Ser Gly Met Phe Asp Ser Ser Val Leu Cys Glu Cys
   1505 1510 1515 1520
   Tyr Asp Ala Gly Cys Ala Trp Tyr Glu Leu Thr Pro Ala Glu Thr Thr
   1525 1530 1535
   Val Arg Leu Arg Ala Tyr Met Asn Thr Pro Gly Leu Pro Val Cys Gln
   1540 1545 1550
   Asp His Leu Gly Phe Trp Glu Gly Val Phe Thr Gly Leu Thr His Ile
   1555 1560 1565
   Asp Ala His Phe Leu Ser Gln Thr Lys Gln Ser Gly Glu Asn Phe Pro
   1570 1575 1580
   Tyr Leu Val Ala Tyr Gln Ala Thr Val Cys Ala Arg Ala Gln Ala Pro
   1585 1590 1595 1600
   Pro Pro Ser Trp Asp Gln Met Arg Lys Cys Leu Ile Arg Leu Lys Pro
   1605 1610 1615
   Thr Leu His Gly Pro Thr Pro Leu Leu Tyr Arg Leu Gly Ala Val Gln
   1620 1625 1630
   Asn Glu Val Thr Leu Thr His Pro Ile Thr Lys Tyr Ile Met Thr Cys
   1635 1640 1645
   Met Ser Ala Asp Leu Glu Val Val Thr Ser Thr Trp Val Leu Val Gly
   1650 1655 1660
   Gly Val Leu Ala Ala Leu Ala Ala Tyr Cys Leu Ser Thr Gly Cys Val
   1665 1670 1675 1680
   Val Ile Val Gly Arg Ile Val Leu Ser Gly Lys Pro Ala Ile Ile Pro
   1685 1690 1695
   Asp Arg Glu Val Leu Tyr Gln Glu Phe Asp Glu Met Glu Glu Cys Ser
   1700 1705 1710
   Gln His Leu Pro Tyr Ile Glu Gln Gly Met Met Leu Ala Glu Gln Phe
   1715 1720 1725
   Lys Gln Lys Ala Leu Gly Leu Leu Gln Thr Ala Ser Arg His Ala Glu
   1730 1735 1740
   Val Ile Thr Pro Ala Val Gln Thr Asn Trp Gln Lys Leu Glu Val Phe
   1745 1750 1755 1760
   Trp Ala Lys His Met Trp Asn Phe Ile Ser Gly Ile Gln Tyr Leu Ala
   1765 1770 1775
   Gly Leu Ser Thr Leu Pro Gly Asn Pro Ala Ile Ala Ser Leu Met Ala
   1780 1785 1790
   Phe Thr Ala Ala Val Thr Ser Pro Leu Thr Thr Gly Gln Thr Leu Leu
   1795 1800 1805
   Phe Asn Ile Leu Gly Gly Trp Val Ala Ala Gln Leu Ala Ala Pro Gly
   1810 1815 1820
   Ala Ala Thr Ala Phe Val Gly Ala Gly Leu Ala Gly Ala Ala Leu Asp
   1825 1830 1835 1840
   Ser Val Gly Leu Gly Lys Val Leu Val Asp Ile Leu Ala Gly Tyr Gly
   1845 1850 1855
   Ala Gly Val Ala Gly Ala Leu Val Ala Phe Lys Ile Met Ser Gly Glu
   1860 1865 1870
   Val Pro Ser Thr Glu Asp Leu Val Asn Leu Leu Pro Ala Ile Leu Ser
   1875 1880 1885
   Pro Gly Ala Leu Ala Val Gly Val Val Phe Ala Ser Ile Leu Arg Arg
   1890 1895 1900
   Arg Val Gly Pro Gly Glu Gly Ala Val Gln Trp Met Asn Arg Leu Ile
   1905 1910 1915 1920
   Ala Phe Ala Ser Arg Gly Asn His Val Ser Pro Thr His Tyr Val Pro
   1925 1930 1935
   Glu Ser Asp Ala Ala Ala Arg Val Thr Ala Ile Leu Ser Ser Leu Thr
   1940 1945 1950
   Val Thr Gln Leu Leu Arg Arg Leu His Gln Trp Ile Ser Ser Glu Cys
   1955 1960 1965
   Thr Thr Pro Cys Ser Gly Ser Trp Leu Arg Asp Ile Trp Asp Trp Ile
   1970 1975 1980
   Cys Glu Val Leu Ser Asp Phe Lys Thr Trp Leu Lys Ala Lys Leu Met
   1985 1990 1995 2000
   Pro Gln Leu Pro Gly Ile Pro Phe Val Ser Cys Gln Arg Gly Tyr Arg
   2005 2010 2015
   Gly Val Trp Arg Gly Asp Gly Ile Met His Thr Arg Cys His Cys Gly
   2020 2025 2030
   Ala Glu Ile Thr Gly His Val Lys Asn Gly Thr Met Arg Ile Val Gly
   2035 2040 2045
   Pro Arg Thr Cys Lys Asn Met Trp Ser Gly Thr Phe Phe Ile Asn Ala
   2050 2055 2060
   Tyr Thr Thr Gly Pro Cys Thr Pro Leu Pro Ala Pro Asn Tyr Lys Phe
   2065 2070 2075 2080
   Ala Leu Trp Arg Val Ser Ala Glu Glu Tyr Val Glu Ile Arg Arg Val
   2085 2090 2095
   Gly Asp Phe His Tyr Val Ser Gly Met Thr Thr Asp Asn Leu Lys Cys
   2100 2105 2110
   Pro Cys Gln Ile Pro Ser Pro Glu Phe Phe Thr Glu Leu Asp Gly Val
   2115 2120 2125
   Arg Leu His Arg Phe Ala Pro Pro Cys Lys Pro Leu Leu Arg Glu Glu
   2130 2135 2140
   Val Ser Phe Arg Val Gly Leu His Glu Tyr Pro Val Gly Ser Gln Leu
   2145 2150 2155 2160
   Pro Cys Glu Pro Glu Pro Asp Val Ala Val Leu Thr Ser Met Leu Thr
   2165 2170 2175
   Asp Pro Ser His Ile Thr Ala Glu Ala Ala Gly Arg Arg Leu Ala Arg
   2180 2185 2190
   Gly Ser Pro Pro Ser Met Ala Ser Ser Ser Ala Ser Gln Leu Ser Ala
   2195 2200 2205
   Pro Ser Leu Lys Ala Thr Cys Thr Ala Asn His Asp Ser Pro Asp Ala
   2210 2215 2220
   Glu Leu Ile Glu Ala Asn Leu Leu Trp Arg Gln Glu Met Gly Gly Asn
   2225 2230 2235 2240
   Ile Thr Arg Val Glu Ser Glu Asn Lys Val Val Ile Leu Asp Ser Phe
   2245 2250 2255
   Asp Pro Leu Val Ala Glu Glu Asp Glu Arg Glu Val Ser Val Pro Ala
   2260 2265 2270
   Glu Ile Leu Arg Lys Ser Arg Arg Phe Ala Pro Ala Leu Pro Val Trp
   2275 2280 2285
   Ala Arg Pro Asp Tyr Asn Pro Leu Leu Val Glu Thr Trp Lys Lys Pro
   2290 2295 2300
   Asp Tyr Glu Pro Pro Val Val His Gly Cys Pro Leu Pro Pro Pro Arg
   2305 2310 2315 2320
   Ser Pro Pro Val Pro Pro Pro Arg Lys Lys Arg Thr Val Val Leu Thr
   2325 2330 2335
   Glu Ser Thr Leu Pro Thr Ala Leu Ala Glu Leu Ala Thr Lys Ser Phe
   2340 2345 2350
   Gly Ser Ser Ser Thr Ser Gly Ile Thr Gly Asp Asn Thr Thr Thr Ser
   2355 2360 2365
   Ser Glu Pro Ala Pro Ser Gly Cys Pro Pro Asp Ser Asp Val Glu Ser
   2370 2375 2380
   Tyr Ser Ser Met Pro Pro Leu Glu Gly Glu Pro Gly Asp Pro Asp Leu
   2385 2390 2395 2400
   Ser Asp Gly Ser Trp Ser Thr Val Ser Ser Gly Ala Asp Thr Glu Asp
   2405 2410 2415
   Val Val Cys Cys Ser Met Ser Tyr Ser Trp Thr Gly Ala Leu Val Thr
   2420 2425 2430
   Pro Cys Ala Ala Glu Glu Gln Lys Leu Pro Ile Asn Ala Leu Ser Asn
   2435 2440 2445
   Ser Leu Leu Arg His His Asn Leu Val Tyr Ser Thr Thr Ser Arg Ser
   2450 2455 2460
   Ala Cys Gln Arg Lys Lys Lys Val Thr Phe Asp Arg Leu Gln Val Leu
   2465 2470 2475 2480
   Asp Ser His Tyr Gln Asp Val Leu Lys Glu Val Lys Ala Ala Ala Ser
   2485 2490 2495
   Lys Val Lys Ala Asn Leu Leu Ser Val Glu Glu Ala Cys Ser Leu Ala
   2500 2505 2510
   Pro Pro His Ser Ala Lys Ser Lys Phe Gly Tyr Gly Ala Lys Asp Val
   2515 2520 2525
   Arg Cys His Ala Arg Lys Ala Val Ala His Ile Asn Ser Val Trp Lys
   2530 2535 2540
   Asp Leu Leu Glu Asp Ser Val Thr Pro Ile Asp Thr Thr Ile Met Ala
   2545 2550 2555 2560
   Lys Asn Glu Val Phe Cys Val Gln Pro Glu Lys Gly Gly Arg Lys Pro
   2565 2570 2575
   Ala Arg Leu Ile Val Phe Pro Asp Leu Gly Val Arg Val Cys Glu Lys
   2580 2585 2590
   Met Ala Leu Tyr Asp Val Val Ser Lys Leu Pro Leu Ala Val Met Gly
   2595 2600 2605
   Ser Ser Tyr Gly Phe Gln Tyr Ser Pro Gly Gln Arg Val Glu Phe Leu
   2610 2615 2620
   Val Gln Ala Trp Lys Ser Lys Lys Thr Pro Met Gly Leu Ser Tyr Asp
   2625 2630 2635 2640
   Thr Arg Cys Phe Asp Ser Thr Val Thr Glu Ser Asp Ile Arg Thr Glu
   2645 2650 2655
   Glu Ala Ile Tyr Gln Cys Cys Asp Leu Asp Pro Gln Ala Arg Val Ala
   2660 2665 2670
   Ile Lys Ser Leu Thr Glu Arg Leu Tyr Val Gly Gly Pro Leu Thr Asn
   2675 2680 2685
   Ser Arg Gly Glu Asn Cys Gly Tyr Arg Arg Cys Arg Ala Ser Arg Val
   2690 2695 2700
   Leu Thr Thr Ser Cys Gly Asn Thr Leu Thr Arg Tyr Ile Lys Ala Arg
   2705 2710 2715 2720
   Ala Ala Cys Arg Ala Ala Gly Leu Gln Asp Cys Thr Met Leu Val Cys
   2725 2730 2735
   Gly Asp Asp Leu Val Val Ile Cys Glu Ser Ala Gly Val Gln Glu Asp
   2740 2745 2750
   Ala Ala Ser Leu Arg Ala Phe Thr Glu Ala Met Thr Arg Tyr Ser Ala
   2755 2760 2765
   Pro Pro Gly Asp Pro Pro Gln Pro Glu Tyr Asp Leu Glu Leu Ile Thr
   2770 2775 2780
   Ser Cys Ser Ser Asn Val Ser Val Ala His Asp Gly Ala Gly Lys Arg
   2785 2790 2795 2800
   Val Tyr Tyr Leu Thr Arg Asp Pro Thr Thr Pro Leu Ala Arg Ala Ala
   2805 2810 2815
   Trp Glu Thr Ala Arg His Thr Pro Val Asn Ser Trp Leu Gly Asn Ile
   2820 2825 2830
   Ile Met Phe Ala Pro Thr Leu Trp Ala Arg Met Ile Leu Met Thr His
   2835 2840 2845
   Phe Phe Ser Val Leu Ile Ala Arg Asp Gln Leu Glu Gln Ala Leu Asn
   2850 2855 2860
   Cys Glu Ile Tyr Gly Ala Cys Tyr Ser Ile Glu Pro Leu Asp Leu Pro
   2865 2870 2875 2880
   Pro Ile Ile Gln Arg Leu His Gly Leu Ser Ala Phe Ser Leu His Ser
   2885 2890 2895
   Tyr Ser Pro Gly Glu Ile Asn Arg Val Ala Ala Cys Leu Arg Lys Leu
   2900 2905 2910
   Gly Val Pro Pro Leu Arg Ala Trp Arg His Arg Ala Trp Ser Val Arg
   2915 2920 2925
   Ala Arg Leu Leu Ala Arg Gly Gly Lys Ala Ala Ile Cys Gly Lys Tyr
   2930 2935 2940
   Leu Phe Asn Trp Ala Val Arg Thr Lys Leu Lys Leu Thr Pro Ile Thr
   2945 2950 2955 2960
   Ala Ala Gly Arg Leu Asp Leu Ser Gly Trp Phe Thr Ala Gly Tyr Ser
   2965 2970 2975
   Gly Gly Asp Ile Tyr His Ser Val Ser His Ala Arg Pro Arg Trp Phe
   2980 2985 2990
   Trp Phe Cys Leu Leu Leu Leu Ala Ala Gly Val Gly Ile Tyr Leu Leu
   2995 3000 3005
   Pro Asn Arg
   3010
<210> 2
   <211> 182
   <212> PRT
   <213> Artificial Sequence
<220>
   <223> Hepatitis C virus core protein sequence
<400> 2
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Thr Trp Ala Gln Pro Gly
   65 70 75 80
   Tyr Pro Trp Pro Leu Tyr Gly Asn Glu Gly Cys Gly Trp Ala Gly Trp
   85 90 95
   Leu Leu Ser Pro Arg Gly Ser Arg Pro Ser Trp Gly Pro Thr Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
       130                 135                 140
   Gly Gly Ala Ala Arg Ala Leu Ala His Gly Val Arg Val Leu Glu Asp
   145                 150                 155                 160
   Gly Val Asn Tyr Ala Thr Gly Asn Leu Pro Gly Cys Ser Phe Ser Ile
                   165                 170                 175
   Phe Leu Leu Ala Leu Leu
               180
<210> 3
   <211> 197
   <212> PRT
   <213> Artificial Sequence
<220>
   <223> Hepatitis C virus E1 protein sequence
<400> 3
   Ser Cys Leu Thr Val Pro Ala Ser Ala Tyr Gln Val Arg Asn Ser Ser
   1               5                   10                  15
   Gly Leu Tyr His Val Thr Asn Asp Cys Pro Asn Ser Ser Val Val Tyr
               20                  25                  30
   Glu Ala Ala Asp Ala Ile Leu His Thr Pro Gly Cys Val Pro Cys Val
           35                  40                  45
   Arg Glu Gly Asn Ala Ser Arg Cys Trp Val Ala Val Thr Pro Thr Val
       50                  55                  60
   Ala Thr Arg Asp Gly Lys Leu Pro Thr Thr Gln Leu Arg Arg His Ile
   65                  70                  75                  80
   Asp Leu Leu Val Gly Ser Ala Thr Leu Cys Ser Ala Leu Tyr Val Gly
                   85                  90                  95
   Asp Leu Cys Gly Ser Val Phe Leu Val Gly Gln Leu Phe Thr Phe Ser
               100                 105                 110
   Pro Arg His His Trp Thr Thr Gln Asp Cys Asn Cys Ser Ile Tyr Pro
           115                 120                 125
   Gly His Ile Thr Gly His Arg Met Ala Trp Asn Met Met Met Asn Trp
       130                 135                 140
   Ser Pro Thr Ala Ala Leu Val Val Ala Gln Leu Leu Arg Ile Pro Gln
   145                 150                 155                 160
   Ala Ile Met Asp Met Ile Ala Gly Ala His Trp Gly Val Leu Ala Gly
                   165                 170                 175
   Ile Lys Tyr Phe Ser Met Val Gly Asn Trp Ala Lys Val Leu Val Val
               180                 185                 190
   Leu Leu Leu Phe Ala
           195
<210> 4
   <211> 350
   <212> PRT
   <213> Artificial Sequence
<220>
   <223> Hepatitis C virus E2 protein sequence
<400> 4
   Gly Val Asp Ala Glu Thr His Val Thr Gly Gly Asn Ala Gly Arg Thr
   1               5                   10                  15
   Thr Ala Gly Leu Val Gly Leu Leu Thr Pro Gly Ala Lys Gln Asn Ile
               20                  25                  30
   Gln Leu Ile Asn Thr Asn Gly Ser Trp His Ile Asn Ser Thr Ala Leu
           35                  40                  45
   Asn Cys Asn Glu Ser Leu Asn Thr Gly Trp Leu Ala Gly Leu Phe Tyr
       50                  55                  60
   Gln His Lys Phe Asn Ser Ser Gly Cys Pro Glu Arg Leu Ala Ser Cys
   65                  70                  75                  80
   Arg Arg Leu Thr Asp Phe Ala Gln Gly Trp Gly Pro Ile Ser Tyr Ala
                   85                  90                  95
   Asn Gly Ser Gly Leu Asp Glu Arg Pro Tyr Cys Trp His Tyr Pro Pro
               100                 105                 110
   Arg Pro Cys Gly Ile Val Pro Ala Lys Ser Val Cys Gly Pro Val Tyr
           115                 120                 125
   Cys Phe Thr Pro Ser Pro Val Val Val Gly Thr Thr Asp Arg Ser Gly
       130                 135                 140
   Ala Pro Thr Tyr Ser Trp Gly Ala Asn Asp Thr Asp Val Phe Val Leu
   145                 150                 155                 160
   Asn Asn Thr Arg Pro Pro Leu Gly Asn Trp Phe Gly Cys Thr Trp Met
                   165                 170                 175
   Asn Ser Thr Gly Phe Thr Lys Val Cys Gly Ala Pro Pro Cys Val Ile
               180                 185                 190
   Gly Gly Val Gly Asn Asn Thr Leu Leu Cys Pro Thr Asp Cys Phe Arg
           195                 200                 205
   Lys Tyr Pro Glu Ala Thr Tyr Ser Arg Cys Gly Ser Gly Pro Arg Ile
       210                 215                 220
   Thr Pro Arg Cys Met Val Asp Tyr Pro Tyr Arg Leu Trp His Tyr Pro
   225                 230                 235                 240
   Cys Thr Ile Asn Tyr Thr Ile Phe Lys Val Arg Met Tyr Val Gly Gly
                   245                 250                 255
   Val Glu His Arg Leu Glu Ala Ala Cys Asn Trp Thr Arg Gly Glu Arg
               260                 265                 270
   Cys Asp Leu Glu Asp Arg Asp Arg Ser Glu Leu Ser Pro Leu Leu Leu
           275                 280                 285
   Ser Thr Thr Gln Trp Gln Val Leu Pro Cys Ser Phe Thr Thr Leu Pro
       290                 295                 300
   Ala Leu Ser Thr Gly Leu Ile His Leu His Gln Asn Ile Val Asp Val
   305                 310                 315                 320
   Gln Tyr Leu Tyr Gly Val Gly Ser Ser Ile Ala Ser Trp Ala Ile Lys
                   325                 330                 335
   Trp Glu Tyr Val Val Leu Leu Phe Leu Leu Leu Ala Asp Ala
               340                 345                 350
<210> 5
   <211> 315
   <212> PRT
   <213> Artificial Sequence
<220>
   <223> Hepatitis C virus NS2 protein sequence
<400> 5
   Arg Val Cys Ser Cys Leu Trp Met Met Leu Leu Ile Ser Gln Ala Glu
   1               5                   10                  15
   Ala Ala Leu Glu Asn Leu Val Ile Leu Asn Ala Ala Ser Leu Ala Gly
               20                  25                  30
   Thr His Gly Leu Val Ser Phe Leu Val Phe Phe Cys Phe Ala Trp Tyr
           35                  40                  45
   Leu Lys Gly Arg Trp Val Pro Gly Ala Val Tyr Ala Leu Tyr Gly Met
       50                  55                  60
   Trp Pro Leu Leu Leu Leu Leu Leu Ala Leu Pro Gln Arg Ala Tyr Ala
   65                  70                  75                  80
   Leu Asp Thr Glu Val Ala Ala Ser Cys Gly Gly Val Val Leu Val Gly
                   85                  90                  95
   Leu Met Ala Leu Thr Leu Ser Pro Tyr Tyr Lys Arg Tyr Ile Ser Trp
               100                 105                 110
   Cys Met Trp Trp Leu Gln Tyr Phe Leu Thr Arg Val Glu Ala Gln Leu
           115                 120                 125
   His Val Trp Val Pro Pro Leu Asn Val Arg Gly Gly Arg Asp Ala Val
       130                 135                 140
   Ile Leu Leu Thr Cys Val Val His Pro Ala Leu Val Phe Asp Ile Thr
   145                 150                 155                 160
   Lys Leu Leu Leu Ala Ile Phe Gly Pro Leu Trp Ile Leu Gln Ala Ser
                   165                 170                 175
   Leu Leu Lys Val Pro Tyr Phe Val Arg Val Gln Gly Leu Leu Arg Ile
               180                 185                 190
   Cys Ala Leu Ala Arg Lys Ile Ala Gly Gly His Tyr Val Gln Met Ala
           195                 200                 205
   Ile Ile Lys Leu Gly Ala Leu Thr Gly Thr Cys Val Tyr Asn His Leu
       210                 215                 220
   Ala Pro Leu Arg Asp Trp Ala His Asn Gly Leu Arg Asp Leu Ala Val
   225                 230                 235                 240
   Ala Val Glu Pro Val Val Phe Ser Arg Met Glu Thr Lys Leu Ile Thr
                   245                 250                 255
   Trp Gly Ala Asp Thr Ala Ala Cys Gly Asp Ile Ile Asn Gly Leu Pro
               260                 265                 270
   Val Ser Ala Arg Arg Gly Gln Glu Ile Leu Leu Gly Pro Ala Asp Gly
           275                 280                 285
   Met Val Ser Lys Gly Trp Arg Leu Leu Ala Pro Ile Thr Ala Tyr Ala
       290                 295                 300
   Gln Gln Thr Arg Gly Leu Leu Gly Cys Ile Ile
   305                 310                 315
<210> 6
   <211> 613
   <212> PRT
   <213> Artificial Sequence
<220>
   <223> Hepatitis C virus NS3 protein sequence
<400> 6
   Thr Ser Leu Thr Gly Arg Asp Lys Asn Gln Val Glu Gly Glu Val Gln
   1               5                   10                  15
   Ile Val Ser Thr Ala Thr Gln Thr Phe Leu Ala Thr Cys Ile Asn Gly
                       20                  25                  30
   Val Cys Trp Thr Val Tyr His Gly Ala Gly Thr Arg Thr Ile Ala Ser
               35                  40                  45
   Pro Lys Gly Pro Val Ile Gln Thr Tyr Thr Asn Val Asp Gln Asp Leu
       50                  55                  60
   Val Gly Trp Pro Ala Pro Gln Gly Ser Arg Ser Leu Thr Pro Cys Thr
   65                  70                  75                  80
   Cys Gly Ser Ser Asp Leu Tyr Leu Val Thr Arg His Ala Asp Val Ile
                   85                  90                  95
   Pro Val Arg Arg Arg Gly Asp Ser Arg Gly Ser Leu Leu Ser Pro Arg
               100                 105                 110
   Pro Ile Ser Tyr Leu Lys Gly Ser Ser Gly Gly Pro Leu Leu Cys Pro
           115                 120                 125
   Thr Gly His Ala Val Gly Leu Phe Arg Ala Ala Val Cys Thr Arg Gly
       130                 135                 140
   Val Ala Lys Ala Val Asp Phe Ile Pro Val Glu Asn Leu Glu Thr Thr
   145                 150                 155                 160
   Met Arg Ser Pro Val Phe Thr Asp Asn Ser Ser Pro Pro Ala Val Pro
                   165                 170                 175
   Gln Ser Phe Gln Val Ala His Leu His Ala Pro Thr Gly Ser Gly Lys
               180                 185                 190
   Ser Thr Lys Val Pro Ala Ala Tyr Ala Ala Lys Gly Tyr Lys Val Leu
           195                 200                 205
   Val Leu Asn Pro Ser Val Ala Ala Thr Leu Gly Phe Gly Ala Tyr Met
       210                 215                 220
   Ser Lys Ala His Gly Val Asp Pro Asn Ile Arg Thr Gly Val Arg Thr
   225                 230                 235                 240
   Ile Thr Thr Gly Ser Pro Ile Thr Tyr Ser Thr Tyr Gly Lys Phe Leu
                   245                 250                 255
   Ala Asp Ala Gly Cys Ser Gly Gly Ala Tyr Asp Ile Ile Ile Cys Asp
               260                 265                 270
   Glu Cys His Ser Thr Asp Ala Thr Ser Ile Ser Gly Ile Gly Thr Val
           275                 280                 285
   Leu Asp Gln Ala Glu Thr Ala Gly Ala Arg Leu Val Val Leu Ala Thr
       290                 295                 300
   Ala Thr Pro Pro Gly Ser Val Thr Val Ser His Pro Asn Ile Glu Glu
   305                 310                 315                 320
   Val Ala Leu Ser Thr Thr Gly Glu Ile Pro Phe Tyr Gly Lys Ala Ile
                   325                 330                 335
   Pro Leu Glu Val Ile Lys Gly Gly Arg His Leu Ile Phe Cys His Ser
               340                 345                 350
   Lys Lys Lys Cys Asp Glu Leu Ala Ala Lys Leu Val Ala Leu Gly Ile
           355                 360                 365
   Asn Ala Val Ala Tyr Tyr Arg Gly Leu Asp Val Ser Val Ile Pro Thr
       370                 375                 380
   Ser Gly Asp Val Val Val Val Ser Thr Asp Ala Leu Met Thr Gly Phe
   385                 390                 395                 400
   Thr Gly Asp Phe Asp Ser Val Ile Asp Cys Asn Thr Cys Val Thr Gln
                   405                 410                 415
   Thr Val Asp Phe Ser Leu Asp Pro Thr Phe Thr Ile Glu Thr Thr Thr
               420                 425                 430
   Leu Pro Gln Asp Ala Val Ser Arg Thr Gln Arg Arg Gly Arg Thr Gly
           435                 440                 445
   Arg Gly Lys Pro Gly Ile Tyr Arg Phe Val Ala Pro Gly Glu Arg Pro
       450                 455                 460
   Ser Gly Met Phe Asp Ser Ser Val Leu Cys Glu Cys Tyr Asp Ala Gly
   465 470 475 480
   Cys Ala Trp Tyr Glu Leu Thr Pro Ala Glu Thr Thr Val Arg Leu Arg
   485 490 495
   Ala Tyr Met Asn Thr Pro Gly Leu Pro Val Cys Gln Asp His Leu Gly
   500 505 510
   Phe Trp Glu Gly Val Phe Thr Gly Leu Thr His Ile Asp Ala His Phe
   515 520 525
   Leu Ser Gln Thr Lys Gln Ser Gly Glu Asn Phe Pro Tyr Leu Val Ala
   530 535 540
   Tyr Gln Ala Thr Val Cys Ala Arg Ala Gln Ala Pro Pro Pro Ser Trp
   545 550 555 560
   Asp Gln Met Arg Lys Cys Leu Ile Arg Leu Lys Pro Thr Leu His Gly
   565 570 575
   Pro Thr Pro Leu Leu Tyr Arg Leu Gly Ala Val Gln Asn Glu Val Thr
   580 585 590
   Leu Thr His Pro Ile Thr Lys Tyr Ile Met Thr Cys Met Ser Ala Asp
   595 600 605
   Leu Glu Val Val Thr
   610
<210> 7
   <211> 54
   <212> PRT
   <213> Artificial Sequence
<220>
   <223> Hepatitis C virus NS4A protein sequence
<400> 7
   Ser Thr Trp Val Leu Val Gly Gly Val Leu Ala Ala Leu Ala Ala Tyr
   1 5 10 15
   Cys Leu Ser Thr Gly Cys Val Val Ile Val Gly Arg Ile Val Leu Ser
   20 25 30
   Gly Lys Pro Ala Ile Ile Pro Asp Arg Glu Val Leu Tyr Gln Glu Phe
   35 40 45
   Asp Glu Met Glu Glu Cys
   50
<210> 8
   <211> 260
   <212> PRT
   <213> Artificial Sequence
<220>
   <223> Hepatitis C virus NS4B protein sequence
<400> 8
   Ser Gln His Leu Pro Tyr Ile Glu Gln Gly Met Met Leu Ala Glu Gln
   1               5                   10                  15
   Phe Lys Gln Lys Ala Leu Gly Leu Leu Gln Thr Ala Ser Arg His Ala
               20                  25                  30
   Glu Val Ile Thr Pro Ala Val Gln Thr Asn Trp Gln Lys Leu Glu Val
           35                  40                  45
   Phe Trp Ala Lys His Met Trp Asn Phe Ile Ser Gly Ile Gln Tyr Leu
       50                  55                  60
   Ala Gly Leu Ser Thr Leu Pro Gly Asn Pro Ala Ile Ala Ser Leu Met
   65                  70                  75                  80
   Ala Phe Thr Ala Ala Val Thr Ser Pro Leu Thr Thr Gly Gln Thr Leu
                   85                  90                  95
   Leu Phe Asn Ile Leu Gly Gly Trp Val Ala Ala Gln Leu Ala Ala Pro
               100                 105                 110
   Gly Ala Ala Thr Ala Phe Val Gly Ala Gly Leu Ala Gly Ala Ala Leu
           115                 120                 125
   Asp Ser Val Gly Leu Gly Lys Val Leu Val Asp Ile Leu Ala Gly Tyr
       130                 135                 140
   Gly Ala Gly Val Ala Gly Ala Leu Val Ala Phe Lys Ile Met Ser Gly
   145                 150                 155                 160
   Glu Val Pro Ser Thr Glu Asp Leu Val Asn Leu Leu Pro Ala Ile Leu
                   165                 170                 175
   Ser Pro Gly Ala Leu Ala Val Gly Val Val Phe Ala Ser Ile Leu Arg
               180                 185                 190
   Arg Arg Val Gly Pro Gly Glu Gly Ala Val Gln Trp Met Asn Arg Leu
           195                 200                 205
   Ile Ala Phe Ala Ser Arg Gly Asn His Val Ser Pro Thr His Tyr Val
       210                 215                 220
   Pro Glu Ser Asp Ala Ala Ala Arg Val Thr Ala Ile Leu Ser Ser Leu
   225                 230                 235                 240
   Thr Val Thr Gln Leu Leu Arg Arg Leu His Gln Trp Ile Ser Ser Glu
                   245                 250                 255
   Cys Thr Thr Pro
               260
<210> 9
   <211> 1040
   <212> PRT
   <213> Artificial Sequence
<220>
   <223> Hepatitis C virus NSSA/B protein sequence
<400> 9
   Cys Ser Gly Ser Trp Leu Arg Asp Ile Trp Asp Trp Ile Cys Glu Val
    1               5                  10                  15
   Leu Ser Asp Phe Lys Thr Trp Leu Lys Ala Lys Leu Met Pro Gln Leu
               20                  25                  30
   Pro Gly Ile Pro Phe Val Ser Cys Gln Arg Gly Tyr Arg Gly Val Trp
           35                  40                  45
   Arg Gly Asp Gly Ile Met His Thr Arg Cys His Cys Gly Ala Glu Ile
       50                  55                  60
   Thr Gly His Val Lys Asn Gly Thr Met Arg Ile Val Gly Pro Arg Thr
   65                  70                  75                  80
   Cys Lys Asn Met Trp Ser Gly Thr Phe Phe Ile Asn Ala Tyr Thr Thr
                   85                  90                  95
   Gly Pro Cys Thr Pro Leu Pro Ala Pro Asn Tyr Lys Phe Ala Leu Trp
               100                 105                 110
   Arg Val Ser Ala Glu Glu Tyr Val Glu Ile Arg Arg Val Gly Asp Phe
   115 120 125
   His Tyr Val Ser Gly Met Thr Thr Asp Asn Leu Lys Cys Pro Cys Gln
   130 135 140
   Ile Pro Ser Pro Glu Phe Phe Thr Glu Leu Asp Gly Val Arg Leu His
   145 150 155 160
   Arg Phe Ala Pro Pro Cys Lys Pro Leu Leu Arg Glu Glu Val Ser Phe
   165 170 175
   Arg Val Gly Leu His Glu Tyr Pro Val Gly Ser Gln Leu Pro Cys Glu
   180 185 190
   Pro Glu Pro Asp Val Ala Val Leu Thr Ser Met Leu Thr Asp Pro Ser
   195 200 205
   His Ile Thr Ala Glu Ala Ala Gly Arg Arg Leu Ala Arg Gly Ser Pro
   210 215 220
   Pro Ser Met Ala Ser Ser Ser Ala Ser Gln Leu Ser Ala Pro Ser Leu
   225 230 235 240
   Lys Ala Thr Cys Thr Ala Asn His Asp Ser Pro Asp Ala Glu Leu Ile
   245 250 255
   Glu Ala Asn Leu Leu Trp Arg Gln Glu Met Gly Gly Asn Ile Thr Arg
   260 265 270
   Val Glu Ser Glu Asn Lys Val Val Ile Leu Asp Ser Phe Asp Pro Leu
   275 280 285
   Val Ala Glu Glu Asp Glu Arg Glu Val Ser Val Pro Ala Glu Ile Leu
   290 295 300
   Arg Lys Ser Arg Arg Phe Ala Pro Ala Leu Pro Val Trp Ala Arg Pro
   305 310 315 320
   Asp Tyr Asn Pro Leu Leu Val Glu Thr Trp Lys Lys Pro Asp Tyr Glu
   325 330 335
   Pro Pro Val Val His Gly Cys Pro Leu Pro Pro Pro Arg Ser Pro Pro
   340 345 350
   Val Pro Pro Pro Arg Lys Lys Arg Thr Val Val Leu Thr Glu Ser Thr
   355 360 365
   Leu Pro Thr Ala Leu Ala Glu Leu Ala Thr Lys Ser Phe Gly Ser Ser
   370 375 380
   Ser Thr Ser Gly Ile Thr Gly Asp Asn Thr Thr Thr Ser Ser Glu Pro
   385 390 395 400
   Ala Pro Ser Gly Cys Pro Pro Asp Ser Asp Val Glu Ser Tyr Ser Ser
   405 410 415
   Met Pro Pro Leu Glu Gly Glu Pro Gly Asp Pro Asp Leu Ser Asp Gly
   420 425 430
   Ser Trp Ser Thr Val Ser Ser Gly Ala Asp Thr Glu Asp Val Val Cys
   435 440 445
   Cys Ser Met Ser Tyr Ser Trp Thr Gly Ala Leu Val Thr Pro Cys Ala
   450 455 460
   Ala Glu Glu Gln Lys Leu Pro Ile Asn Ala Leu Ser Asn Ser Leu Leu
   465 470 475 480
   Arg His His Asn Leu Val Tyr Ser Thr Thr Ser Arg Ser Ala Cys Gln
   485 490 495
   Arg Lys Lys Lys Val Thr Phe Asp Arg Leu Gln Val Leu Asp Ser His
   500 505 510
   Tyr Gln Asp Val Leu Lys Glu Val Lys Ala Ala Ala Ser Lys Val Lys
   515 520 525
   Ala Asn Leu Leu Ser Val Glu Glu Ala Cys Ser Leu Ala Pro Pro His
   530 535 540
   Ser Ala Lys Ser Lys Phe Gly Tyr Gly Ala Lys Asp Val Arg Cys His
   545 550 555 560
   Ala Arg Lys Ala Val Ala His Ile Asn Ser Val Trp Lys Asp Leu Leu
   565 570 575
   Glu Asp Ser Val Thr Pro Ile Asp Thr Thr Ile Met Ala Lys Asn Glu
   580 585 590
   Val Phe Cys Val Gln Pro Glu Lys Gly Gly Arg Lys Pro Ala Arg Leu
   595 600 605
   Ile Val Phe Pro Asp Leu Gly Val Arg Val Cys Glu Lys Met Ala Leu
   610 615 620
   Tyr Asp Val Val Ser Lys Leu Pro Leu Ala Val Met Gly Ser Ser Tyr
   625 630 635 640
   Gly Phe Gln Tyr Ser Pro Gly Gln Arg Val Glu Phe Leu Val Gln Ala
   645 650 655
   Trp Lys Ser Lys Lys Thr Pro Met Gly Leu Ser Tyr Asp Thr Arg Cys
   660 665 670
   Phe Asp Ser Thr Val Thr Glu Ser Asp Ile Arg Thr Glu Glu Ala Ile
   675 680 685
   Tyr Gln Cys Cys Asp Leu Asp Pro Gln Ala Arg Val Ala Ile Lys Ser
   690 695 700
   Leu Thr Glu Arg Leu Tyr Val Gly Gly Pro Leu Thr Asn Ser Arg Gly
   705 710 715 720
   Glu Asn Cys Gly Tyr Arg Arg Cys Arg Ala Ser Arg Val Leu Thr Thr
   725 730 735
   Ser Cys Gly Asn Thr Leu Thr Arg Tyr Ile Lys Ala Arg Ala Ala Cys
   740 745 750
   Arg Ala Ala Gly Leu Gln Asp Cys Thr Met Leu Val Cys Gly Asp Asp
   755 760 765
   Leu Val Val Ile Cys Glu Ser Ala Gly Val Gln Glu Asp Ala Ala Ser
   770 775 780
   Leu Arg Ala Phe Thr Glu Ala Met Thr Arg Tyr Ser Ala Pro Pro Gly
   785 790 795 800
   Asp Pro Pro Gln Pro Glu Tyr Asp Leu Glu Leu Ile Thr Ser Cys Ser
   805 810 815
   Ser Asn Val Ser Val Ala His Asp Gly Ala Gly Lys Arg Val Tyr Tyr
   820 825 830
   Leu Thr Arg Asp Pro Thr Thr Pro Leu Ala Arg Ala Ala Trp Glu Thr
   835 840 845
   Ala Arg His Thr Pro Val Asn Ser Trp Leu Gly Asn Ile Ile Met Phe
   850 855 860
   Ala Pro Thr Leu Trp Ala Arg Met Ile Leu Met Thr His Phe Phe Ser
   865 870 875 880
   Val Leu Ile Ala Arg Asp Gln Leu Glu Gln Ala Leu Asn Cys Glu Ile
   885 890 895
   Tyr Gly Ala Cys Tyr Ser Ile Glu Pro Leu Asp Leu Pro Pro Ile Ile
   900 905 910
   Gln Arg Leu His Gly Leu Ser Ala Phe Ser Leu His Ser Tyr Ser Pro
   915 920 925
   Gly Glu Ile Asn Arg Val Ala Ala Cys Leu Arg Lys Leu Gly Val Pro
   930 935 940
   Pro Leu Arg Ala Trp Arg His Arg Ala Trp Ser Val Arg Ala Arg Leu
   945 950 955 960
   Leu Ala Arg Gly Gly Lys Ala Ala Ile Cys Gly Lys Tyr Leu Phe Asn
   965 970 975
   Trp Ala Val Arg Thr Lys Leu Lys Leu Thr Pro Ile Thr Ala Ala Gly
   980 985 990
   Arg Leu Asp Leu Ser Gly Trp Phe Thr Ala Gly Tyr Ser Gly Gly Asp
       995                     1000                    1005
   Ile Tyr His Ser Val Ser His Ala Arg Pro Arg Trp Phe Trp Phe Cys
       1010                1015                1020
   Leu Leu Leu Leu Ala Ala Gly Val Gly Ile Tyr Leu Leu Pro Asn Arg
   1025                1030                1035                1040
<210> 10
   <211> 226
   <212> PRT
   <213> Artificial Sequence
<220>
   <223> Hepatitis B virus S antigen (HBsAg) sequence
<400> 10
   Met Glu Asn Ile Thr Ser Gly Phe Leu Gly Pro Leu Leu Val Leu Gln
   1               5                   10                  15
   Ala Gly Phe Phe Leu Leu Thr Arg Ile Leu Thr Ile Pro Gln Ser Leu
               20                  25                  30
   Asp Ser Trp Trp Thr Ser Leu Asp Phe Leu Gly Gly Thr Thr Val Cys
           35                  40                  45
   Leu Gly Gln Asn Ser Gln Ser Pro Thr Ser Asn His Ser Pro Thr Ser
       50                  55                  60
   Cys Pro Pro Thr Cys Pro Gly Tyr Arg Trp Met Cys Leu Arg Arg Phe
   65                  70                  75                  80
   Ile Ile Phe Leu Phe Ile Leu Leu Leu Cys Leu Ile Phe Leu Leu Val
                   85                  90                  95
   Leu Leu Asp Tyr Gln Gly Met Leu Pro Val Cys Pro Leu Ile Pro Gly
               100                 105                 110
   Ser Ser Thr Thr Ser Thr Gly Pro Cys Arg Thr Cys Met Thr Thr Ala
           115                 120                 125
   Gln Gly Thr Ser Met Tyr Pro Ser Cys Cys Cys Thr Lys Pro Ser Asp
       130                 135                 140
   Gly Asn Cys Thr Cys Ile Pro Ile Pro Ser Ser Trp Ala Phe Gly Lys
   145                 150                 155                 160
   Phe Leu Trp Glu Trp Ala Ser Ala Arg Phe Ser Trp Leu Ser Leu Leu
                   165                 170                 175
   Val Pro Phe Val Gln Trp Phe Val Gly Leu Ser Pro Thr Val Trp Leu
               180                 185                 190
   Ser Val Ile Trp Met Met Trp Tyr Trp Gly Pro Ser Leu Tyr Ser Ile
           195                 200                 205
   Leu Ser Pro Phe Leu Pro Leu Leu Pro Ile Phe Phe Cys Leu Trp Val
       210                 215                 220
   Tyr Ile
   225
<210> 11
   <211> 212
   <212> PRT
   <213> Artificial Sequence
<220>
   <223> Hepatitis B virus C antigen and e antigen (HBcAg/HBeAg) sequence
<400> 11
   Met Gln Leu Phe His Leu Cys Leu Ile Ile Ser Cys Ser Cys Pro Thr
   1               5                   10                  15
   Val Gln Ala Ser Lys Leu Cys Leu Gly Trp Leu Trp Gly Met Asp Ile
               20                  25                  30
   Asp Pro Tyr Lys Glu Phe Gly Ala Thr Val Glu Leu Leu Ser Phe Leu
           35                  40                  45
   Pro Ser Asp Phe Phe Pro Ser Val Arg Asp Leu Leu Asp Thr Ala Ser
       50                  55                  60
   Ala Leu Tyr Arg Glu Ala Leu Glu Ser Pro Glu His Cys Ser Pro His
   65                  70                  75                  80
   His Thr Ala Leu Arg Gln Ala Ile Leu Cys Trp Gly Glu Leu Met Thr
                   85                  90                  95
   Leu Ala Thr Trp Val Gly Val Asn Leu Glu Asp Pro Ala Ser Arg Asp
               100                 105                 110
   Leu Val Val Ser Tyr Val Asn Thr Asn Met Gly Leu Lys Phe Arg Gln
           115                 120                 125
   Leu Leu Trp Phe His Ile Ser Cys Leu Thr Phe Gly Arg Glu Thr Val
       130                 135                 140
   Ile Glu Tyr Leu Val Ser Phe Gly Val Trp Ile Arg Thr Pro Pro Ala
   145                 150                 155                 160
   Tyr Arg Pro Pro Asn Ala Pro Ile Leu Ser Thr Leu Pro Glu Thr Thr
                   165                 170                 175
   Val Val Arg Arg Arg Gly Arg Ser Pro Arg Arg Arg Thr Pro Ser Pro
               180                 185                 190
   Arg Arg Arg Arg Ser Gln Ser Pro Arg Arg Arg Arg Ser Gln Ser Arg
           195                 200                 205
   Glu Ser Gln Cys
       210
<210> 12
   <211> 2227
   <212> PRT
   <213> Artificial Sequence
<220>
   <223> Hepatitis A virus sequence
<400> 12
   Met Asn Met Ser Lys Gln Gly Ile Phe Gln Thr Val Gly Ser Gly Leu
   1               5                   10                  15
   Asp His Ile Leu Ser Leu Ala Asp Ile Glu Glu Glu Gln Met Ile Gln
               20                  25                  30
   Ser Val Asp Arg Thr Ala Val Thr Gly Ala Ser Tyr Phe Thr Ser Val
           35                  40                  45
   Asp Gln Ser Ser Val His Thr Ala Glu Val Gly Ser His Gln Ile Glu
       50                  55                  60
   Pro Leu Lys Thr Ser Val Asp Lys Pro Gly Ser Lys Lys Thr Gln Gly
   65                  70                  75                  80
   Glu Lys Phe Phe Leu Ile His Ser Ala Asp Trp Leu Thr Thr His Ala
                   85                  90                  95
   Leu Phe His Glu Val Ala Lys Leu Asp Val Val Lys Leu Leu Tyr Asn
               100                 105                 110
   Glu Gln Phe Ala Val Gln Gly Leu Leu Arg Tyr His Thr Tyr Ala Arg
           115                 120                 125
   Phe Gly Ile Glu Ile Gln Val Gln Ile Asn Pro Thr Pro Phe Gln Gln
       130                 135                 140
   Gly Gly Leu Ile Cys Ala Met Val Pro Gly Asp Gln Ser Tyr Gly Ser
   145                 150                 155                 160
   Ile Ala Ser Leu Thr Val Tyr Pro His Gly Leu Leu Asn Cys Asn Ile
                   165                 170                 175
   Asn Asn Val Val Arg Ile Lys Val Pro Phe Ile Tyr Thr Arg Gly Ala
               180                 185                 190
   Tyr His Phe Lys Asp Pro Gln Tyr Pro Val Trp Glu Leu Thr Ile Arg
           195                 200                 205
   Val Trp Ser Glu Leu Asn Ile Gly Thr Gly Thr Ser Ala Tyr Thr Ser
       210                 215                 220
   Leu Asn Val Leu Ala Arg Phe Thr Asp Leu Glu Leu His Gly Leu Thr
   225                 230                 235                 240
   Pro Leu Ser Thr Gln Met Met Arg Asn Glu Phe Arg Val Ser Thr Thr
                   245                 250                 255
   Glu Asn Val Val Asn Leu Ser Asn Tyr Glu Asp Ala Arg Ala Lys Met
               260                 265                 270
   Ser Phe Ala Leu Asp Gln Glu Asp Trp Lys Ser Asp Pro Ser Gln Gly
           275                 280                 285
   Gly Gly Ile Lys Ile Thr His Phe Thr Thr Trp Thr Ser Ile Pro Thr
       290                 295                 300
   Leu Ala Ala Gln Phe Pro Phe Asn Ala Ser Asp Ser Val Gly Gln Gln
   305                 310                 315                 320
   Ile Lys Val Ile Pro Val Asp Pro Tyr Phe Phe Gln Met Thr Asn Thr
                   325                 330                 335
   Asn Pro Asp Gln Lys Cys Ile Thr Ala Leu Ala Ser Ile Cys Gln Met
               340                 345                 350
   Phe Cys Phe Trp Arg Gly Asp Leu Val Phe Asp Phe Gln Val Phe Pro
           355                 360                 365
   Thr Lys Tyr His Ser Gly Arg Leu Leu Phe Cys Phe Val Pro Gly Asn
       370                 375                 380
   Glu Leu Ile Asp Val Thr Gly Ile Thr Leu Lys Gln Ala Thr Thr Ala
   385                 390                 395                 400
   Pro Cys Ala Val Met Asp Ile Thr Gly Val Gln Ser Thr Leu Arg Phe
                   405                 410                 415
   Arg Val Pro Trp Ile Ser Asp Thr Pro Tyr Arg Val Asn Arg Tyr Thr
               420                 425                 430
   Lys Ser Ala His Gln Lys Gly Glu Tyr Thr Ala Ile Gly Lys Leu Ile
           435                 440                 445
   Val Tyr Cys Tyr Asn Arg Leu Thr Ser Pro Ser Asn Val Ala Ser His
       450                 455                 460
   Val Arg Val Asn Val Tyr Leu Ser Ala Ile Asn Leu Glu Cys Phe Ala
   465                 470                 475                 480
   Pro Leu Tyr His Ala Met Asp Val Thr Thr Gln Val Gly Asp Asp Ser
                   485                 490                 495
   Gly Gly Phe Ser Thr Thr Val Ser Thr Glu Gln Asn Val Pro Asp Pro
               500                 505                 510
   Gln Val Gly Ile Thr Thr Met Arg Asp Leu Lys Gly Lys Ala Asn Arg
           515                 520                 525
   Gly Lys Met Asp Val Ser Gly Val Gln Ala Pro Arg Gly Ser Tyr Gln
       530                 535                 540
   Gln Gln Leu Asn Asp Pro Val Leu Ala Lys Lys Val Pro Glu Thr Phe
   545 550 555 560
   Pro Glu Leu Lys Pro Gly Glu Ser Arg His Thr Ser Asp His Met Ser
   565 570 575
   Ile Tyr Lys Phe Met Gly Arg Ser His Phe Leu Cys Thr Phe Thr Phe
   580 585 590
   Asn Ser Asn Asn Lys Glu Tyr Thr Phe Pro Ile Thr Leu Ser Ser Thr
   595 600 605
   Ser Asn Pro Pro His Gly Leu Pro Ser Thr Leu Arg Trp Phe Phe Asn
   610 615 620
   Leu Phe Gln Leu Tyr Arg Gly Pro Leu Asp Leu Thr Ile Ile Ile Thr
   625 630 635 640
   Gly Ala Thr Asp Val Asp Gly Met Ala Trp Phe Thr Pro Val Gly Leu
   645 650 655
   Ala Val Asp Pro Trp Val Glu Lys Glu Ser Ala Leu Ser Ile Asp Tyr
   660 665 670
   Lys Thr Ala Leu Gly Ala Val Arg Phe Asn Thr Arg Arg Thr Gly Asn
   675 680 685
   Ile Gln Ile Arg Leu Pro Trp Tyr Ser Tyr Leu Tyr Ala Val Ser Gly
   690 695 700
   Ala Leu Asp Gly Leu Gly Asp Lys Thr Asp Ser Thr Phe Gly Leu Phe
   705 710 715 720
   Leu Phe Glu Ile Ala Asn Tyr Asn His Ser Asp Glu Tyr Leu Ser Phe
   725 730 735
   Ser Cys Tyr Leu Ser Val Thr Glu Gln Ser Glu Phe Tyr Phe Pro Arg
   740 745 750
   Ala Pro Leu Asn Ser Asn Ala Met Leu Ser Thr Glu Ser Met Met Ser
   755 760 765
   Arg Ile Ala Ala Gly Asp Leu Glu Ser Ser Val Asp Asp Pro Arg Ser
   770 775 780
   Glu Glu Asp Arg Arg Phe Glu Ser His Ile Glu Cys Arg Lys Pro Tyr
   785 790 795 800
   Lys Glu Leu Arg Leu Glu Val Gly Lys Gln Arg Leu Lys Tyr Ala Gln
   805 810 815
   Glu Glu Leu Ser Asn Glu Val Leu Pro Pro Pro Arg Lys Met Lys Gly
   820 825 830
   Leu Phe Ser Gln Ala Lys Ile Ser Leu Phe Tyr Thr Glu Glu His Glu
   835 840 845
   Ile Met Lys Phe Ser Trp Arg Gly Val Thr Ala Asp Thr Arg Ala Leu
   850 855 860
   Arg Arg Phe Gly Phe Ser Leu Ala Ala Gly Arg Ser Val Trp Thr Leu
   865 870 875 880
   Glu Met Asp Ala Gly Val Leu Thr Gly Arg Leu Ile Arg Leu Asn Asp
   885 890 895
   Glu Lys Trp Thr Glu Met Lys Asp Asp Lys Ile Val Ser Leu Ile Glu
   900 905 910
   Lys Phe Thr Ser Asn Lys Tyr Trp Ser Lys Val Asn Phe Pro His Gly
   915 920 925
   Met Leu Asp Leu Glu Glu Ile Ala Ala Asn Ser Lys Asp Phe Pro Asn
   930 935 940
   Met Ser Glu Thr Asp Leu Cys Phe Leu Leu His Trp Leu Asn Pro Lys
   945 950 955 960
   Lys Ile Asn Leu Ala Asp Arg Met Leu Gly Leu Ser Gly Val Gln Glu
   965 970 975
   Ile Lys Glu Gln Gly Val Gly Leu Ile Ala Glu Cys Arg Thr Phe Leu
   980 985 990
   Asp Ser Ile Ala Gly Thr Leu Lys Ser Met Met Phe Gly Phe His His
   995 1000 1005
   Ser Val Thr Val Glu Ile Ile Asn Thr Val Leu Cys Phe Val Lys Ser
   1010 1015 1020
   Gly Ile Leu Leu Tyr Val Ile Gln Gln Leu Asn Gln Asp Glu His Ser
   1025 1030 1035 1040
   His Ile Ile Gly Leu Leu Arg Val Met Asn Tyr Ala Asp Ile Gly Cys
   1045 1050 1055
   Ser Val Ile Ser Cys Gly Lys Val Phe Ser Lys Met Leu Glu Thr Val
   1060 1065 1070
   Phe Asn Trp Gln Met Asp Ser Arg Met Met Glu Leu Arg Thr Gln Ser
   1075 1080 1085
   Phe Ser Asn Trp Leu Arg Asp Ile Cys Ser Gly Ile Thr Ile Phe Lys
   1090 1095 1100
   Ser Phe Lys Asp Ala Ile Tyr Trp Leu Tyr Thr Lys Leu Lys Asp Phe
   1105 1110 1115 1120
   Tyr Glu Val Asn Tyr Gly Lys Lys Lys Asp Ile Leu Asn Ile Leu Lys
   1125 1130 1135
   Asp Asn Gln Gln Lys Ile Glu Lys Ala Ile Glu Glu Ala Asp Asn Phe
   1140 1145 1150
   Cys Ile Leu Gln Ile Gln Asp Val Glu Lys Phe Asp Gln Tyr Gln Lys
   1155 1160 1165
   Gly Val Asp Leu Ile Gln Lys Leu Arg Thr Val His Ser Met Ala Gln
   1170 1175 1180
   Val Asp Pro Asn Leu Gly Val His Leu Ser Pro Leu Arg Asp Cys Ile
   1185 1190 1195 1200
   Ala Arg Val His Gln Lys Leu Lys Asn Leu Gly Ser Ile Asn Gln Ala
   1205 1210 1215
   Met Val Thr Arg Cys Glu Pro Val Val Cys Tyr Leu Tyr Gly Lys Arg
   1220 1225 1230
   Gly Gly Gly Lys Ser Leu Thr Ser Ile Ala Leu Ala Thr Lys Ile Cys
   1235 1240 1245
   Lys His Tyr Gly Val Glu Pro Glu Lys Asn Ile Tyr Thr Lys Pro Val
   1250 1255 1260
   Ala Ser Asp Tyr Trp Asp Gly Tyr Ser Gly Gln Leu Val Cys Ile Ile
   1265 1270 1275 1280
   Asp Asp Ile Gly Gln Asn Thr Thr Asp Glu Asp Trp Ser Asp Phe Cys
   1285 1290 1295
   Gln Leu Val Ser Gly Cys Pro Met Arg Leu Asn Met Ala Ser Leu Glu
   1300 1305 1310
   Glu Lys Gly Arg His Phe Ser Ser Pro Phe Ile Ile Ala Thr Ser Asn
   1315 1320 1325
   Trp Ser Asn Pro Ser Pro Lys Thr Val Tyr Val Lys Glu Ala Ile Asp
   1330 1335 1340
   Arg Arg Leu His Phe Lys Val Glu Val Lys Pro Ala Ser Phe Phe Lys
   1345 1350 1355 1360
   Asn Pro His Asn Asp Met Leu Asn Val Asn Leu Ala Lys Thr Asn Asp
   1365 1370 1375
   Ala Ile Lys Asp Met Ser Cys Val Asp Leu Ile Met Asp Gly His Asn
   1380 1385 1390
   Ile Ser Leu Met Asp Leu Leu Ser Ser Leu Val Met Thr Val Glu Ile
   1395 1400 1405
   Arg Lys Gln Asn Met Ser Glu Phe Met Glu Leu Trp Ser Gln Gly Ile
   1410 1415 1420
   Ser Asp Asp Asp Asn Asp Ser Ala Val Ala Glu Phe Phe Gln Ser Phe
   1425 1430 1435 1440
   Pro Ser Gly Glu Pro Ser Asn Trp Lys Leu Ser Ser Phe Phe Gln Ser
   1445 1450 1455
   Val Thr Asn His Lys Trp Val Ala Val Gly Ala Ala Val Gly Ile Leu
   1460 1465 1470
   Gly Val Leu Val Gly Gly Trp Phe Val Tyr Lys His Phe Ser Arg Lys
   1475 1480 1485
   Glu Glu Glu Pro Ile Pro Ala Glu Gly Val Tyr His Gly Val Thr Lys
   1490 1495 1500
   Pro Lys Gln Val Ile Lys Leu Asp Ala Asp Pro Val Glu Ser Gln Ser
   1505 1510 1515 1520
   Thr Leu Glu Ile Ala Gly Leu Val Arg Lys Asn Leu Val Gln Phe Gly
   1525 1530 1535
   Val Gly Glu Lys Asn Gly Cys Val Arg Trp Val Met Asn Ala Leu Gly
   1540 1545 1550
   Val Lys Asp Asp Trp Leu Leu Val Pro Ser His Ala Tyr Lys Phe Glu
   1555 1560 1565
   Lys Asp Tyr Glu Met Met Glu Phe Tyr Phe Asn Arg Gly Gly Thr Tyr
   1570 1575 1580
   Tyr Ser Ile Ser Ala Gly Asn Val Val Ile Gln Ser Leu Asp Val Gly
   1585 1590 1595 1600
   Phe Gln Asp Val Val Leu Met Lys Val Pro Thr Ile Pro Lys Phe Arg
   1605 1610 1615
   Asp Ile Thr Gln His Phe Ile Lys Lys Gly Asp Val Pro Arg Ala Leu
   1620 1625 1630
   Asn Arg Leu Ala Thr Leu Val Thr Thr Val Asn Gly Thr Pro Met Leu
   1635 1640 1645
   Ile Ser Glu Gly Pro Leu Lys Met Glu Glu Lys Ala Thr Tyr Val His
   1650 1655 1660
   Lys Lys Asn Asp Gly Thr Thr Val Asp Leu Thr Val Asp Gln Ala Trp
   1665 1670 1675 1680
   Arg Gly Lys Gly Glu Gly Leu Pro Gly Met Cys Gly Gly Ala Leu Val
   1685 1690 1695
   Ser Ser Asn Gln Ser Ile Gln Asn Ala Ile Leu Gly Ile His Val Ala
   1700 1705 1710
   Gly Gly Asn Ser Ile Leu Val Ala Lys Leu Val Thr Gln Glu Met Phe
   1715 1720 1725
   Gln Asn Ile Asp Lys Lys Ile Glu Ser Gln Arg Ile Met Lys Val Glu
   1730 1735 1740
   Phe Thr Gln Cys Ser Met Asn Val Val Ser Lys Thr Leu Phe Arg Lys
   1745 1750 1755 1760
   Ser Pro Ile His His His Ile Asp Lys Thr Met Ile Asn Phe Pro Ala
   1765 1770 1775
   Ala Met Pro Phe Ser Lys Ala Glu Ile Asp Pro Met Ala Met Met Leu
   1780 1785 1790
   Ser Lys Tyr Ser Leu Pro Ile Val Glu Glu Pro Glu Asp Tyr Lys Glu
   1795 1800 1805
   Ala Ser Val Phe Tyr Gln Asn Lys Ile Val Gly Lys Thr Gln Leu Val
   1810 1815 1820
   Asp Asp Phe Leu Asp Leu Asp Met Ala Ile Thr Gly Ala Pro Gly Ile
   1825 1830 1835 1840
   Asp Ala Ile Asn Met Asp Ser Ser Pro Gly Phe Pro Tyr Val Gln Glu
   1845 1850 1855
   Lys Leu Thr Lys Arg Asp Leu Ile Trp Leu Asp Glu Asn Gly Leu Leu
                   1860                1865                1870
   Leu Gly Val His Pro Arg Leu Ala Gln Arg Ile Leu Phe Asn Thr Val
           1875                1880                1885
   Met Met Glu Asn Cys Ser Asp Leu Asp Val Val Phe Thr Thr Cys Pro
       1890                1895                1900
   Lys Asp Glu Leu Arg Pro Leu Glu Lys Val Leu Glu Ser Lys Thr Arg
   1905                1910                1915                1920
   Ala Ile Asp Ala Cys Pro Leu Asp Tyr Thr Ile Leu Cys Arg Met Tyr
                   1925                1930                1935
   Trp Gly Pro Ala Ile Ser Tyr Phe His Leu Asn Pro Gly Phe His Thr
               1940                1945                1950
   Gly Val Ala Ile Gly Ile Asp Pro Asp Arg Gln Trp Asp Glu Leu Phe
           1955                1960                1965
   Lys Thr Met Ile Arg Phe Gly Asp Val Gly Leu Asp Leu Asp Phe Ser
       1970                1975                1980
   Ala Phe Asp Ala Ser Leu Ser Pro Phe Met Ile Arg Glu Ala Gly Arg
   1985                1990                1995                2000
   Ile Met Ser Glu Leu Ser Gly Thr Pro Ser His Phe Gly Thr Ala Leu
                   2005                2010                2015
   Ile Asn Thr Ile Ile Tyr Ser Lys His Leu Leu Tyr Asn Cys Cys Tyr
               2020                2025                2030
   His Val Cys Gly Ser Met Pro Ser Gly Ser Pro Cys Thr Ala Leu Leu
           2035                2040                2045
   Asn Ser Ile Ile Asn Asn Ile Asn Leu Tyr Tyr Val Phe Ser Lys Ile
       2050                2055                2060
   Phe Gly Lys Ser Pro Val Phe Phe Cys Gln Ala Leu Arg Ile Leu Cys
   2065                2070                2075                2080
   Tyr Gly Asp Asp Val Leu Ile Val Phe Ser Arg Asp Val Gln Ile Asp
                   2085                2090                2095
   Asn Leu Asp Leu Ile Gly Gln Lys Ile Val Asp Glu Phe Lys Lys Leu
               2100                2105                2110
   Gly Met Thr Ala Thr Ser Ala Asp Lys Asn Val Pro Gln Leu Lys Pro
           2115                2120                2125
   Val Ser Glu Leu Thr Phe Leu Lys Arg Ser Phe Asn Leu Val Glu Asp
       2130                2135                2140
   Arg Ile Arg Pro Ala Ile Ser Glu Lys Thr Ile Trp Ser Leu Met Ala
   2145                2150                2155                2160
   Trp Gln Arg Ser Asn Ala Glu Phe Glu Gln Asn Leu Glu Asn Ala Gln
                   2165                2170                2175
   Trp Phe Ala Phe Met His Gly Tyr Glu Phe Tyr Gln Lys Phe Tyr Tyr
               2180                2185                2190
   Phe Val Gln Ser Cys Leu Glu Lys Glu Met Ile Glu Tyr Arg Leu Lys
           2195                2200                2205
   Ser Tyr Asp Trp Trp Arg Met Arg Phe Tyr Asp Gln Cys Phe Ile Cys
       2210                2215                2220
   Asp Leu Ser
   2225
<210> 13
   <211> 9416
   <212> DNA
   <213> Artificial Sequence
<220>
   <223> Hepatitis C virus sequence
<400> 13
   gccagccccc tgatgggggc gacactccac catgaatcac tcccctgtga ggaactactg 60
   tcttcacgca gaaagcgtct agccatggcg ttagtatgag tgtcgtgcag cctccaggac 120
   cccccctccc gggagagcca tagtggtctg cggaaccggt gagtacaccg gaattgccag 180
   gacgaccggg tcctttcttg gataaacccg ctcaatgcct ggagatttgg gcgtgccccc 240
   gcaagactgc tagccgagta gtgttgggtc gcgaaaggcc ttgtggtact gcctgatagg 300
   gtgcttgcga gtgccccggg aggtctcgta gaccgtgcac catgagcacg aatcctaaac 360
   ctcaaagaaa aaccaaacgt aacaccaacc gtcgcccaca ggacgtcaag ttcccgggtg 420
   gcggtcagat cgttggtgga gtttacttgt tgccgcgcag gggccctaga ttgggtgtgc 480
   gcgcgacgag gaagacttcc gagcggtcgc aacctcgagg tagacgtcag cctatcccca 540
   aggcacgtcg gcccgagggc aggacctggg ctcagcccgg gtacccttgg cccctctatg 600
   gcaatgaggg ttgcgggtgg gcgggatggc tcctgtctcc ccgtggctct cggcctagct 660
   ggggccccac agacccccgg cgtaggtcgc gcaatttggg taaggtcatc gataccctta 720
   cgtgcggctt cgccgacctc atggggtaca taccgctcgt cggcgcccct cttggaggcg 780
   ctgccagggc cctggcgcat ggcgtccggg ttctggaaga cggcgtgaac tatgcaacag 840
   ggaaccttcc tggttgctct ttctctatct tccttctggc cctgctctct tgcctgactg 900
   tgcccgcttc agcctaccaa gtgcgcaatt cctcggggct ttaccatgtc accaatgatt 960
   gccctaactc gagtgttgtg tacgaggcgg ccgatgccat cctgcacact ccggggtgtg 1020
   tcccttgcgt tcgcgagggt aacgcctcga ggtgttgggt ggcggtgacc cccacggtgg 1080
   ccaccaggga cggcaaactc cccacaacgc agcttcgacg tcatatcgat ctgcttgtcg 1140
   ggagcgccac cctctgctcg gccctctacg tgggggacct gtgcgggtct gtctttcttg 1200
   ttggtcaact gtttaccttc tctcccaggc accactggac gacgcaagac tgcaattgtt 1260
   ctatctatcc cggccatata acgggtcatc gcatggcatg gaatatgatg atgaactggt 1320
   cccctacggc agcgttggtg gtagctcagc tgctccgaat cccacaagcc atcatggaca 1380
   tgatcgctgg cgcccactgg ggagtcctgg cgggcataaa gtatttctcc atggtgggga 1440
   actgggcgaa ggtcctggta gtgctgctgc tatttgccgg cgtcgacgcg gaaacccacg 1500
   tcaccggggg aaatgccggc cgcaccacgg ctgggcttgt tggtctcctt acaccaggcg 1560
   ccaagcagaa catccaactg atcaacacca acggcagttg gcacatcaat agcacggcct 1620
   tgaactgcaa tgaaagcctt aacaccggct ggttagcagg gctcttctat cagcacaaat 1680
   tcaactcttc aggctgtcct gagaggttgg ccagctgccg acgccttacc gattttgccc 1740
   agggctgggg tcctatcagt tatgccaacg gaagcggcct cgacgaacgc ccctactgct 1800
   ggcactaccc tccaagacct tgtggcattg tgcccgcaaa gagcgtgtgt ggcccggtat 1860
   attgcttcac tcccagcccc gtggtggtgg gaacgaccga caggtcgggc gcgcctacct 1920
   acagctgggg tgcaaatgat acggatgtct tcgtccttaa caacaccagg ccaccgctgg 1980
   gcaattggtt cggttgtacc tggatgaact caactggatt caccaaagtg tgcggagcgc 2040
   ccccttgtgt catcggaggg gtgggcaaca acaccttgct ctgccccact gattgcttcc 2100
   gcaaatatcc ggaagccaca tactctcggt gcggctccgg tcccaggatt acacccaggt 2160
   gcatggtcga ctacccgtat aggctttggc actatccttg taccatcaat tacaccatat 2220
   tcaaagtcag gatgtacgtg ggaggggtcg agcacaggct ggaagcggcc tgcaactgga 2280
   cgcggggcga acgctgtgat ctggaagaca gggacaggtc cgagctcagc ccgttgctgc 2340
   tgtccaccac acagtggcag gtccttccgt gttctttcac gaccctgcca gccttgtcca 2400
   ccggcctcat ccacctccac cagaacattg tggacgtgca gtacttgtac ggggtagggt 2460
   caagcatcgc gtcctgggcc attaagtggg agtacgtcgt tctcctgttc cttctgcttg 2520
   cagacgcgcg cgtctgttcc tgcttgtgga tgatgttact catatcccaa gcggaggcgg 2580
   ctttggagaa cctcgtaata ctcaatgcag catccctggc cgggacgcat ggtcttgtgt 2640
   ccttcctcgt gttcttctgc tttgcgtggt atctgaaggg taggtgggtg cccggagcgg 2700
   tctacgccct ctacgggatg tggcctctcc tcctgctcct gctggcgttg cctcagcggg 2760
   catacgcact ggacacggag gtggccgcgt cgtgtggcgg cgttgttctt gtcgggttaa 2820
   tggcgctgac tctgtcgcca tattacaagc gctatatcag ctggtgcatg tggtggcttc 2880
   agtattttct gaccagagta gaagcgcaac tgcacgtgtg ggttcccccc ctcaacgtcc 2940
   ggggggggcg cgatgccgtc atcttactca cgtgtgtagt acacccggcc ctggtatttg 3000
   acatcaccaa actactcctg gccatcttcg gacccctttg gattcttcaa gccagtttgc 3060
   ttaaagtccc ctacttcgtg cgcgttcaag gccttctccg gatctgcgcg ctagcgcgga 3120
   agatagccgg aggtcattac gtgcaaatgg ccatcatcaa gttaggggcg cttactggca 3180
   cctgtgtgta taaccatctc gctcctcttc gagactgggc gcacaacggc ctgcgagatc 3240
   tggccgtggc tgtggaacca gtcgtcttct cccgaatgga gaccaagctc atcacgtggg 3300
   gggcagatac cgccgcgtgc ggtgacatca tcaacggctt gcccgtctct gcccgtaggg 3360
   gccaggagat actgcttggg ccagccgacg gaatggtctc caaggggtgg aggttgctgg 3420
   cgcccatcac ggcgtacgcc cagcagacga gaggcctcct agggtgtata atcaccagcc 3480
   tgactggccg ggacaaaaac caagtggagg gtgaggtcca gatcgtgtca actgctaccc 3540
   agaccttcct ggcaacgtgc atcaatgggg tatgctggac tgtctaccac ggggccggaa 3600
   cgaggaccat cgcatcaccc aagggtcctg tcatccagac gtataccaat gtggatcaag 3660
   acctcgtggg ctggcccgct cctcaaggtt cccgctcatt gacaccctgc acctgcggct 3720
   cctcggacct ttacctggtc acgaggcacg ccgatgtcat tcccgtgcgc cggcgaggtg 3780
   atagcagggg tagcctgctt tcgccccggc ccatttccta cttgaaaggc tcctcggggg 3840
   gtccgctgtt gtgccccacg ggacacgccg tgggcctatt cagggccgcg gtgtgcaccc 3900
   gtggagtggc taaggcggtg gactttatcc ctgtggagaa cctagagaca accatgagat 3960
   ccccggtgtt cacggacaac tcctctccac cagcagtgcc ccagagcttc caggtggccc 4020
   acctgcatgc tcccaccggc agcggtaaga gcaccaaggt cccggctgcg tacgcagcca 4080
   agggctacaa ggtgttggtg ctcaacccct ctgttgctgc aacactgggc tttggtgctt 4140
   acatgtccaa ggcccatggg gttgatccta atatcaggac cggggtgaga acaattacca 4200
   ctggcagccc catcacgtac tccacctacg gcaagttcct tgccgacgcc gggtgctcag 4260
   gaggtgctta tgacataata atttgtgacg agtgccactc cacggatgcc acatccatct 4320
   cgggcatcgg cactgtcctt gaccaagcag agactgcggg ggcgagactg gttgtgctcg 4380
   ccactgctac ccctccgggc tccgtcactg tgtcccatcc taacatcgag gaggttgctc 4440
   tgtccaccac cggagagatc cccttttacg gcaaggctat ccccctcgag gtgatcaagg 4500
   ggggaagaca tctcatcttc tgccactcaa agaagaagtg cgacgagctc gccgcgaagc 4560
   tggtcgcatt gggcatcaat gccgtggcct actaccgcgg tcttgacgtg tctgtcatcc 4620
   cgaccagcgg cgatgttgtc gtcgtgtcga ccgatgctct catgactggc tttaccggcg 4680
   acttcgactc tgtgatagac tgcaacacgt gtgtcactca gacagtcgat tttagccttg 4740
   accctacctt taccattgag acaaccacgc tcccccagga tgctgtctcc aggactcaac 4800
   gccggggcag gactggcagg gggaagccag gcatctatag atttgtggca ccgggggagc 4860
   gcccctccgg catgttcgac tcgtccgtcc tctgtgagtg ctatgacgcg ggctgtgctt 4920
   ggtatgagct cacgcccgcc gagactacag ttaggctacg agcgtacatg aacaccccgg 4980
   ggcttcccgt gtgccaggac catcttggat tttgggaggg cgtctttacg ggcctcactc 5040
   atatagatgc ccactttcta tcccagacaa agcagagtgg ggagaacttt ccttacctgg 5100
   tagcgtacca agccaccgtg tgcgctaggg ctcaagcccc tcccccatcg tgggaccaga 5160
   tgcggaagtg tttgatccgc cttaaaccca ccctccatgg gccaacaccc ctgctataca 5220
   gactgggcgc tgttcagaat gaagtcaccc tgacgcaccc aatcaccaaa tacatcatga 5280
   catgcatgtc ggccgacctg gaggtcgtca cgagcacctg ggtgctcgtt ggcggcgtcc 5340
   tggctgctct ggccgcgtat tgcctgtcaa caggctgcgt ggtcatagtg ggcaggatcg 5400
   tcttgtccgg gaagccggca attatacctg acagggaggt tctctaccag gagttcgatg 5460
   agatggaaga gtgctctcag cacttaccgt acatcgagca agggatgatg ctcgctgagc 5520
   agttcaagca gaaggccctc ggcctcctgc agaccgcgtc ccgccatgca gaggttatca 5580
   cccctgctgt ccagaccaac tggcagaaac tcgaggtctt ttgggcgaag cacatgtgga 5640
   atttcatcag tgggatacaa tacttggcgg gcctgtcaac gctgcctggt aaccccgcca 5700
   ttgcttcatt gatggctttt acagctgccg tcaccagccc actaaccact ggccaaaccc 5760
   tcctcttcaa catattgggg gggtgggtgg ctgcccagct cgccgccccc ggtgccgcta 5820
   ccgcctttgt gggcgctggc ttagctggcg ccgcactcga cagcgttgga ctggggaagg 5880
   tcctcgtgga cattcttgca ggctatggcg cgggcgtggc gggagctctt gtggcattca 5940
   agatcatgag cggtgaggtc ccctccacgg aggacctggt caatctgctg cccgccatcc 6000
   tctcacctgg agcccttgca gtcggtgtgg tctttgcatc aatactgcgc cggcgtgttg 6060
   gcccgggcga gggggcagtg caatggatga accggctaat agccttcgcc tcccggggga 6120
   accatgtttc ccccacacac tacgtgccgg agagcgatgc agccgcccgc gtcactgcca 6180
   tactcagcag cctcactgta acccagctcc tgaggcgact gcatcagtgg ataagctcgg 6240
   agtgtaccac tccatgctcc ggttcctggc taagggacat ctgggactgg atatgcgagg 6300
   tgctgagcga ctttaagacc tggctgaaag ccaagctcat gccacaactg cctgggattc 6360
   cctttgtgtc ctgccagcgc gggtataggg gggtctggcg aggagacggc attatgcaca 6420
   ctcgctgcca ctgtggagct gagatcactg gacatgtcaa aaacgggacg atgaggatcg 6480
   tcggtcctag gacctgcaag aacatgtgga gtgggacgtt cttcattaat gcctacacca 6540
   cgggcccctg tactcccctt cctgcgccga actataagtt cgcgctgtgg agggtgtctg 6600
   cagaggaata cgtggagata aggcgggtgg gggacttcca ctacgtatcg ggcatgacta 6660
   ctgacaatct caaatgcccg tgccagatcc catcgcccga atttttcaca gaattggacg 6720
   gggtgcgcct acataggttt gcgccccctt gcaagccctt gctgcgggag gaggtatcat 6780
   tcagagtagg actccacgag tacccggtgg ggtcgcaatt accttgcgag cccgaaccgg 6840
   acgtagccgt gttgacgtcc atgctcactg atccctccca tataacagca gaggcggccg 6900
   ggagaaggtt ggcgagaggg tcaccccctt ctatggccag ctcctcggct agccagctgt 6960
   ccgctccatc tctcaaggca acttgcaccg ccaaccatga ctcccctgac gccgagctca 7020
   tagaggctaa cctcctgtgg aggcaggaga tgggcggcaa catcaccagg gttgagtcag 7080
   agaacaaagt ggtgattctg gactccttcg atccgcttgt ggcagaggag gatgagcggg 7140
   aggtctccgt acccgcagaa attctgcgga agtctcggag attcgcccca gccctgcccg 7200
   tctgggcgcg gccggactac aaccccctgc tagtagagac gtggaaaaag cctgactacg 7260
   aaccacctgt ggtccatggc tgcccgctac cacctccacg gtcccctcct gtgcctccgc 7320
   ctcggaaaaa gcgtacggtg gtcctcaccg aatcaaccct acctactgcc ttggccgagc 7380
   ttgccaccaa aagttttggc agctcctcaa cttccggcat tacgggcgac aatacgacaa 7440
   catcctctga gcccgcccct tctggctgcc cccccgactc cgacgttgag tcctattctt 7500
   ccatgccccc cctggagggg gagcctgggg atccggatct cagcgacggg tcatggtcga 7560
   cggtcagtag tggggccgac acggaagatg tcgtgtgctg ctcaatgtct tattcctgga 7620
   caggcgcact cgtcaccccg tgcgctgcgg aggaacaaaa actgcccatc aacgcactga 7680
   gcaactcgtt gctacgccat cacaatctgg tgtattccac cacttcacgc agtgcttgcc 7740
   aaaggaagaa gaaagtcaca tttgacagac tgcaagttct ggacagccat taccaggacg 7800
   tgctcaagga ggtcaaagca gcggcgtcaa aagtgaaggc taacttgcta tccgtagagg 7860
   aagcttgcag cctggcgccc ccacattcag ccaaatccaa gtttggctat ggggcaaaag 7920
   acgtccgttg ccatgccaga aaggccgtag cccacatcaa ctccgtgtgg aaagaccttc 7980
   tggaagacag tgtaacacca atagacacta ccatcatggc caagaacgag gttttctgcg 8040
   ttcagcctga gaaggggggt cgtaagccag ctcgtctcat cgtgttcccc gacctgggcg 8100
   tgcgcgtgtg cgagaagatg gccctgtacg acgtggttag caagctcccc ttggccgtga 8160
   tgggaagctc ctacggattc caatactcac caggacagcg ggttgaattc ctcgtgcaag 8220
   cgtggaagtc caagaagacc ccgatggggc tctcgtatga tacccgctgt tttgactcca 8280
   cagtcactga gagcgacatc cgtacggagg aggcaattta ccaatgttgt gacctggacc 8340
   cccaagcccg cgtggccatc aagtccctca ctgagaggct ttatgttggg ggccctctta 8400
   ctaattcaag gggggaaaac tgcggctacc gcaggtgccg cgcgagcaga gtactgacaa 8460
   ctagctgtgg taacaccctc actcgctaca tcaaggcccg ggcagcctgt cgagccgcag 8520
   ggctccagga ctgcaccatg ctcgtgtgtg gcgacgactt agtcgttatc tgtgaaagtg 8580
   cgggggtcca ggaggacgcg gcgagcctga gagccttcac ggaggctatg accaggtact 8640
   ccgccccccc cggggacccc ccacaaccag aatacgactt ggagcttata acatcatgct 8700
   cctccaacgt gtcagtcgcc cacgacggcg ctggaaagag ggtctactac cttacccgtg 8760
   accctacaac ccccctcgcg agagccgcgt gggagacagc aagacacact ccagtcaatt 8820
   cctggctagg caacataatc atgtttgccc ccacactgtg ggcgaggatg atactgatga 8880
   cccacttctt tagcgtcctc atagccaggg atcagcttga acaggctctc aactgcgaga 8940
   tctacggagc ctgctactcc atagaaccac tggatctacc tccaatcatt caaagactcc 9000
   atggcctcag cgcattttca ctccacagtt actctccagg tgaaattaat agggtggccg 9060
   catgcctcag aaaacttggg gtcccgccct tgcgagcttg gagacaccgg gcctggagcg 9120
   tccgcgctag gcttctggcc agaggaggca aggctgccat atgtggcaag tacctcttca 9180
   actgggcagt aagaacaaag ctcaaactca ctccgataac ggccgctggc cggctggact 9240
   tgtccggctg gttcacggct ggctacagcg ggggagacat ttatcacagc gtgtctcatg 9300
   cccggccccg ctggttctgg ttttgcctac tcctgcttgc tgcaggggta ggcatctacc 9360
   tcctccccaa ccgatgaaga ttgggctaac cactccaggc caataggcca ttccct 9416
<210> 14
   <211> 3182
   <212> DNA
   <213> Artificial Sequence
<220>
   <223> Hepatitis B virus sequence
<400> 14
   aattccacaa ccttccacca aactctgcaa gatcccagag tgagaggcct gtatttccct 60
   gctggtggct ccagttcagg aacagtaaac cctgttctga ctactgcctc tcccttatcg 120
   tcaatcttct cgaggattgg ggaccctgcg ctgaacatgg agaacatcac atcaggattc 180
   ctaggacccc ttctcgtgtt acaggcgggg tttttcttgt tgacaagaat cctcacaata 240
   ccgcagagtc tagactcgtg gtggacttct ctcaattttc tagggggaac taccgtgtgt 300
   cttggccaaa attcgcagtc cccaacctcc aatcactcac caacctcttg tcctccaact 360
   tgtcctggtt atcgctggat gtgtctgcgg cgttttatca tcttcctctt catcctgctg 420
   ctatgcctca tcttcttgtt ggttcttctg gactatcaag gtatgttgcc cgtttgtcct 480
   ctaattccag gatcctcaac aaccagcacg ggaccatgcc ggacctgcat gactactgct 540
   caaggaacct ctatgtatcc ctcctgttgc tgtaccaaac cttcggacgg aaattgcacc 600
   tgtattccca tcccatcatc ctgggctttc ggaaaattcc tatgggagtg ggcctcagcc 660
   cgtttctcct ggctcagttt actagtgcca tttgttcagt ggttcgtagg gctttccccc 720
   actgtttggc tttcagttat atggatgatg tggtattggg ggccaagtct gtacagcatc 780
   ttgagtccct ttttaccgct gttaccaatt ttcttttgtc tttgggtata catttaaacc 840
   ctaacaaaac aaagagatgg ggttactctc taaattttat gggttatgtc attggatgtt 900
   atgggtcctt gccacaagaa cacatcatac aaaaaatcaa agaatgtttt agaaaacttc 960
   ctattaacag gcctattgat tggaaagtat gtcaacgaat tgtgggtctt ttgggttttg 1020
   ctgccccttt tacacaatgt ggttatcctg cgttgatgcc tttgtatgca tgtattcaat 1080
   ctaagcaggc tttcactttc tcgccaactt acaaggcctt tctgtgtaaa caatacctga 1140
   acctttaccc cgttgcccgg caacggccag gtctgtgcca agtgtttgct gacgcaaccc 1200
   ccactggctg gggcttggtc atgggccatc agcgcatgcg tggaaccttt tcggctcctc 1260
   tgccgatcca tactgcggaa ctcctagccg cttgttttgc tcgcagcagg tctggagcaa 1320
   acattatcgg gactgataac tctgttgtcc tatcccgcaa atatacatcg tttccatggc 1380
   tgctaggctg tgctgccaac tggatcctgc gcgggacgtc ctttgtttac gtcccgtcgg 1440
   cgctgaatcc tgcggacgac ccttctcggg gtcgcttggg actctctcgt ccccttctcc 1500
   gtctgccgtt ccgaccgacc acggggcgca cctctcttta cgcggactcc ccgtctgtgc 1560
   cttctcatct gccggaccgt gtgcacttcg cttcacctct gcacgtcgca tggagaccac 1620
   cgtgaacgcc caccaaatat tgcccaaggt cttacataag aggactcttg gactctcagc 1680
   aatgtcaacg accgaccttg aggcatactt caaagactgt ttgtttaaag actgggagga 1740
   gttgggggag gagattaggt taaaggtctt tgtactagga ggctgtaggc ataaattggt 1800
   ctgcgcacca gcaccatgca actttttcac ctctgcctaa tcatctcttg ttcatgtcct 1860
   actgttcaag cctccaagct gtgccttggg tggctttggg gcatggacat cgacccttat 1920
   aaagaatttg gagctactgt ggagttactc tcgtttttgc cttctgactt ctttccttca 1980
   gtacgagatc ttctagatac cgcctcagct ctgtatcggg aagccttaga gtctcctgag 2040
   cattgttcac ctcaccatac tgcactcagg caagcaattc tttgctgggg ggaactaatg 2100
   actctagcta cctgggtggg tgttaatttg gaagatccag cgtctagaga cctagtagtc 2160
   agttatgtca acactaatat gggcctaaag ttcaggcaac tcttgtggtt tcacatttct 2220
   tgtctcactt ttggaagaga aacagttata gagtatttgg tgtctttcgg agtgtggatt 2280
   cgcactcctc cagcttatag accaccaaat gcccctatcc tatcaacact tccggagact 2340
   actgttgtta gacgacgagg caggtcccct agaagaagaa ctccctcgcc tcgcagacga 2400
   aggtctcaat cgccgcgtcg cagaagatct caatctcggg aatctcaatg ttagtattcc 2460
   ttggactcat aaggtgggga actttactgg gctttattct tctactgtac ctgtctttaa 2520
   tcctcattgg aaaacaccat cttttcctaa tatacattta caccaagaca ttatcaaaaa 2580
   atgtgaacag tttgtaggcc cactcacagt taatgagaaa agaagattgc aattgattat 2640
   gcctgccagg ttttatccaa aggttaccaa atatttacca ttggataagg gtattaaacc 2700
   ttattatcca gaacatctag ttaatcatta cttccaaact agacactatt tacacactct 2760
   atggaaggcg ggtatattat ataagagaga aacaacacat agcgcctcat tttgtgggtc 2820
   accatattct tgggaacaag atctacagca tggggcagaa tctttccacc agcaatcctc 2880
   tgggattctt tcccgaccac cagttggatc cagccttcag agcaaacacc gcaaatccag 2940
   attgggactt caatcccaac aaggacacct ggccagacgc caacaaggta ggagctggag 3000
   cattcgggct gggtttcacc ccaccgcacg gaggcctttt ggggtggagc cctcaggctc 3060
   agggcatact acaaactttg ccagcaaatc cgcctcctgc ctccaccaat cgccagtcag 3120
   gaaggcagcc taccccgctg tctccacctt tgagaaacac tcatcctcag gccatgcagt 3180
   gg                                                                3182
<210> 15
   <211> 7478
   <212> RNA
   <213> Artificial Sequence
<220>
   <223> Hepatitis A virus sequence
<400> 15
<210> 16
   <211> 2061
   <212> DNA
   <213> Artificial Sequence
<220>
   <223> Hepatitis C virus NS3/4A coding region
<400> 16
   atggcgccta tcacggccta tgcccagcag acaaggggcc ttttgggatg cataatcacc 60
   agcttgaccg gccgggacaa aaaccaggtg gagggtgagg ttcagatcgt gtcaactgct 120
   gcccagactt tcttggcaac ctgcattaac ggggtgtgtt ggactgtcta ccatggagcc 180
   ggaacaagga ccattgcgtc acctaagggt cctgttatcc agatgtacac caatgtggac 240
   caagacctcg taggctggcc cgctccccaa ggtgcccgct cattaacacc atgcacttgc 300
   ggctcctcgg acctttacct ggtcacgagg cacgccgatg tcattcctgt gcgccgacgg 360
   ggtgatggca ggggcagcct gctttcgccc cggcctatct cttacttgaa aggctcctcg 420
   ggaggccctc tgctgtgccc cgcaggacat gccgtaggca tattcagagc cgcggtatgc 480
   acccgtggag tggctaaggc ggtggacttc atccccgtag agagcttaga gacaaccatg 540
   aggtccccgg tgttctcaga caactcctcc ccaccagcag tgccccagag ctaccaagtg 600
   gcccacctgc atgctcccac cggcagcggt aagagcacca aggtcccggc cgcatacgca 660
   gctcagggct acaaggtgct ggtgctcaac ccctccgttg ctgcaacaat gggctttggt 720
   gcttacatgt ccaaggccca tgggattgat cctaacatca ggactggggt gaggacaatt 780
   actactggca gcccgatcac gtattccacc tacggcaagt tccttgccga cggcgggtgt 840
   tcagggggtg cttatgacat aataatttgt gacgagtgcc actccacgga tgcaacatcc 900
   atcttgggca ttggcactgt ccttgaccaa gcagagaccg cgggggcgag actgactgtg 960
   ctcgccaccg ctacccctcc gggctccgtc actgtgcccc atcctaacat cgaggaggtt 1020
   gctctgtcca ctaccggaga gatccccttt tatggcaagg ctattcccct tgaagcaatt 1080
   aaggggggga gacatctcat cttctgccac tcaaagaaga agtgcgacga gctcgccgca 1140
   aaactggtcg cgttgggcgt caatgccgtg gcttactacc gcggccttga tgtgtccgtc 1200
   atcccgacca gtggtgacgt tgtcgtcgtg gcaactgacg ccctcatgac cggctttacc 1260
   ggcgacttcg attcggtgat agactgcaac acgtgtgtca cccagacagt cgacttcagc 1320
   cttgacccta ccttcaccat tgagacaatc acgcttcccc aggatgctgt ctcccgtact 1380
   caacgtcggg gtaggactgg cagagggaag ccaggcatct acagatttgt ggcaccgggg 1440
   gagcgtcctt ctggcatgtt tgactcgtct gtcctctgcg agtgctatga cgcgggttgt 1500
   gcttggtatg agcttacgcc cgccgagacc acagttaggc tacgagcata catgaacacc 1560
   ccgggacttc ccgtgtgcca agaccatctt gaattttggg agggcgtctt tacgggtctc 1620
   acccacatag acgcccactt cctatcccag acaaagcaga gtggggaaaa ccttccctat 1680
   ctggtagcgt accaagccac cgtgtgcgct agagctcaag cccctccccc gtcgtgggac 1740
   cagatgtgga agtgcttgat ccgtctcaag cccaccctcc atgggccaac acctctgcta 1800
   tatagactgg gcgctgtcca gaatgaagtc accctgacgc acccagtcac caagtatatc 1860
   atgacatgta tgtcggctga cctggaggtc gtcacgagta cctgggtgct cgttggcggc 1920
   gttctggctg ctttggccgc gtattgccta tccacaggct gcgtggtcat agtaggtagg 1980
   attgtcttgt ccggaaagcc ggcaatcata cccgacaggg aagtcctcta ccgggagttc 2040
   gatgaaatgg aagagtgctg a                                          2061
<210> 17
   <211> 686
   <212> PRT
   <273> Artificial Sequence
<220>
   <223> Hepatitis C virus NS3/4A peptide
<400> 17
   Met Ala Pro Ile Thr Ala Tyr Ala Gln Gln Thr Arg Gly Leu Leu Gly
   1               5                   10                  15
   Cys Ile Ile Thr Ser Leu Thr Gly Arg Asp Lys Asn Gln Val Glu Gly
               20                  25                  30
   Glu Val Gln Ile Val Ser Thr Ala Ala Gln Thr Phe Leu Ala Thr Cys
           35                  40                  45
   Ile Asn Gly Val Cys Trp Thr Val Tyr His Gly Ala Gly Thr Arg Thr
       50                  55                  60
   Ile Ala Ser Pro Lys Gly Pro Val Ile Gln Met Tyr Thr Asn Val Asp
   65                  70                  75                  80
   Gln Asp Leu Val Gly Trp Pro Ala Pro Gln Gly Ala Arg Ser Leu Thr
                   85                  90                  95
   Pro Cys Thr Cys Gly Ser Ser Asp Leu Tyr Leu Val Thr Arg His Ala
               100                 105                 110
   Asp Val Ile Pro Val Arg Arg Arg Gly Asp Gly Arg Gly Ser Leu Leu
           115                 120                 125
   Ser Pro Arg Pro Ile Ser Tyr Leu Lys Gly Ser Ser Gly Gly Pro Leu
       130                 135                 140
   Leu Cys Pro Ala Gly His Ala Val Gly Ile Phe Arg Ala Ala Val Cys
   145                 150                 155                 160
   Thr Arg Gly Val Ala Lys Ala Val Asp Phe Ile Pro Val Glu Ser Leu
                   165                 170                 175
   Glu Thr Thr Met Arg Ser Pro Val Phe Ser Asp Asn Ser Ser Pro Pro
               180                 185                 190
   Ala Val Pro Gln Ser Tyr Gln Val Ala His Leu His Ala Pro Thr Gly
           195                 200                 205
   Ser Gly Lys Ser Thr Lys Val Pro Ala Ala Tyr Ala Ala Gln Gly Tyr
       210                 215                 220
   Lys Val Leu Val Leu Asn Pro Ser Val Ala Ala Thr Met Gly Phe Gly
   225                 230                 235                 240
   Ala Tyr Met Ser Lys Ala His Gly Ile Asp Pro Asn Ile Arg Thr Gly
                   245                 250                 255
   Val Arg Thr Ile Thr Thr Gly Ser Pro Ile Thr Tyr Ser Thr Tyr Gly
               260                 265                 270
   Lys Phe Leu Ala Asp Gly Gly Cys Ser Gly Gly Ala Tyr Asp Ile Ile
           275                 280                 285
   Ile Cys Asp Glu Cys His Ser Thr Asp Ala Thr Ser Ile Leu Gly Ile
       290                 295                 300
   Gly Thr Val Leu Asp Gln Ala Glu Thr Ala Gly Ala Arg Leu Thr Val
   305                 310                 315                 320
   Leu Ala Thr Ala Thr Pro Pro Gly Ser Val Thr Val Pro His Pro Asn
                   325                 330                 335
   Ile Glu Glu Val Ala Leu Ser Thr Thr Gly Glu Ile Pro Phe Tyr Gly
               340                 345                 350
   Lys Ala Ile Pro Leu Glu Ala Ile Lys Gly Gly Arg His Leu Ile Phe
           355                 360                 365
   Cys His Ser Lys Lys Lys Cys Asp Glu Leu Ala Ala Lys Leu Val Ala
       370                 375                 380
   Leu Gly Val Asn Ala Val Ala Tyr Tyr Arg Gly Leu Asp Val Ser Val
   385                 390                 395                 400
   Ile Pro Thr Ser Gly Asp Val Val Val Val Ala Thr Asp Ala Leu Met
                   405                 410                 415
   Thr Gly Phe Thr Gly Asp Phe Asp Ser Val Ile Asp Cys Asn Thr Cys
               420                 425                 430
   Val Thr Gln Thr Val Asp Phe Ser Leu Asp Pro Thr Phe Thr Ile Glu
           435                 440                 445
   Thr Ile Thr Leu Pro Gln Asp Ala Val Ser Arg Thr Gln Arg Arg Gly
       450                 455                 460
   Arg Thr Gly Arg Gly Lys Pro Gly Ile Tyr Arg Phe Val Ala Pro Gly
   465                 470                 475                 480
   Glu Arg Pro Ser Gly Met Phe Asp Ser Ser Val Leu Cys Glu Cys Tyr
                   485                 490                 495
   Asp Ala Gly Cys Ala Trp Tyr Glu Leu Thr Pro Ala Glu Thr Thr Val
               500                 505                 510
   Arg Leu Arg Ala Tyr Met Asn Thr Pro Gly Leu Pro Val Cys Gln Asp
           515                 520                 525
   His Leu Glu Phe Trp Glu Gly Val Phe Thr Gly Leu Thr His Ile Asp
       530                 535                 540
   Ala His Phe Leu Ser Gln Thr Lys Gln Ser Gly Glu Asn Leu Pro Tyr
   545                 550                 555                 560
   Leu Val Ala Tyr Gln Ala Thr Val Cys Ala Arg Ala Gln Ala Pro Pro
                   565                 570                 575
   Pro Ser Trp Asp Gln Met Trp Lys Cys Leu Ile Arg Leu Lys Pro Thr
               580                 585                 590
   Leu His Gly Pro Thr Pro Leu Leu Tyr Arg Leu Gly Ala Val Gln Asn
           595                 600                 605
   Glu Val Thr Leu Thr His Pro Val Thr Lys Tyr Ile Met Thr Cys Met
            610                     615                     620
   Ser Ala Asp Leu Glu Val Val Thr Ser Thr Trp Val Leu Val Gly Gly
   625                     630                     635                     640
   Val Leu Ala Ala Leu Ala Ala Tyr Cys Leu Ser Thr Gly Cys Val Val
                   645                     650                     655
   Ile Val Gly Arg Ile Val Leu Ser Gly Lys Pro Ala Ile Ile Pro Asp
               660                     665                     670
   Arg Glu Val Leu Tyr Arg Glu Phe Asp Glu Met Glu Glu Cys
           675                     680                     685
<210> 18
   <211> 30
   <212> DNA
   <213> Artificial Sequence
<220>
   <223> cloning oligonucleotide
<400> 18
   ccgtctagat cagcactctt ccatttcatc 30
<210> 19
   <211> 30
   <212> DNA
   <213> Artificial Sequence
<220>
   <223> cloning oligonucleotide
<400> 19
   cctgaattca tggcgcctat cacggcctat 30
<210> 20
   <211> 24
   <212> DNA
   <213> Artificial Sequence
<220>
   <223> cloning oligonucleotide
<400> 20
   ccacgcggcc gcgacgacct acag 24
<210> 21
   <211> 33
   <212> DNA
   <213> Artificial Sequence
<220>
   <223> cloning oligonucleotide
<400> 21
   ctggaggtcg tcacgcctac ctgggtgctc gtt 33
<210> 22
   <211> 33
   <212> DNA
   <213> Artificial Sequence
<220>
   <223> cloning oligonucleotide
<400> 22
   accgagcacc caggtaggcg tgacgacctc cag 33
<210> 23
   <211> 33
   <212> DNA
   <213> Artificial Sequence
<220>
   <223> cloning oligonucleotide
<400> 23
   ctggaggtcg tccgcggtac ctgggtgctc gtt 33
<210> 24
   <211> 33
   <212> DNA
   <213> Artificial Sequence
<220>
   <223> cloning oligonucleotide
<400> 24
   accgagcacc caggtaccgc ggacgacctc cag 33
<210> 25
   <211> 25
   <212> PRT
   <213> Artificial Sequence
<220>
   <223> Hepatitis C virus NS3/4A peptide
<400> 25
   Thr Lys Tyr Met Thr Cys Met Ser Ala Asp Leu Glu Val Val Thr Ser
   1               5                   10                  15
   Thr Trp Val Leu Val Gly Gly Val Leu
               20                  25
<210> 26
   <211> 25
   <212> PRT
   <213> Artificial Sequence
<220>
   <223> Mutant Hepatitis C virus NS3/4A peptide
<400> 26
   Thr Lys Tyr Met Thr Cys Met Ser Ala Asp Leu Glu Val Val Thr Gly
   1               5                   10                  15
   Thr Trp Val Leu Val Gly Gly Val Leu
               20                  25
<210> 27
   <211> 25
   <212> PRT
   <213> Artificial Sequence
<220>
   <223> Mutant-Hepatitis C virus NS3/4A peptide
<400> 27
   Thr Lys Tyr Met Thr Cys Met Ser Ala Asp Leu Glu Val Val Arg Gly
   1               5                   10                  15
   Thr Trp Val Leu Val Gly Gly Val Leu
               20                  25
<210> 28
   <211> 12
   <212> PRT
   <213> Artificial Sequence
<220>
   <223> Hepatitis C virus NS3/4A peptide
<400> 28
   Ser Ala Asp Leu Glu Val Val Thr Ser Thr Trp Val
   1               5                   10
<210> 29
   <221> 632
   <212> PRT
   <213> Hepatitis C virus NS3 peptideArtificial Sequence
<220>
   <223> Hepatitis C virus NS3 peptide
<400> 29
<210> 30
   <211> 54
   <212> PRT
   <213> Artificial Sequence
<220>
   <223> Hepatitis C virus NS4A peptide
<400> 30
   Ser Thr Trp Val Leu Val Gly Gly Val Leu Ala Ala Leu Ala Ala Tyr
   1               5                   10                  15
   Cys Leu Ser Thr Gly Cys Val Val Ile Val Gly Arg Ile Val Leu Ser
               20                  25                  30
   Gly Lys Pro Ala Ile Ile Pro Asp Arg Glu Val Leu Tyr Arg Glu Phe
           35                  40                  45
   Asp Glu Met Glu Glu Cys
       50
<210> 31
   <211> 686
   <212> PRT
   <213> Artificial Sequence
<220>
   <223> Mutant Hepatitis C virus NS3/4A
<400> 31
   Met Ala Pro Ile Thr Ala Tyr Ala Gln Gln Thr Arg Gly Leu Leu Gly
   1 5 10 15
   Cys Ile Ile Thr Ser Leu Thr Gly Arg Asp Lys Asn Gln Val Glu Gly
   20 25 30
   Glu Val Gln Ile Val Ser Thr Ala Ala Gln Thr Phe Leu Ala Thr Cys
   35 40 45
   Ile Asn Gly Val Cys Trp Thr Val Tyr His Gly Ala Gly Thr Arg Thr
   50 55 60
   Ile Ala Ser Pro Lys Gly Pro Val Ile Gln Met Tyr Thr Asn Val Asp
   65 70 75 80
   Gln Asp Leu Val Gly Trp Pro Ala Pro Gln Gly Ala Arg Ser Leu Thr
   85 90 95
   Pro Cys Thr Cys Gly Ser Ser Asp Leu Tyr Leu Val Thr Arg His Ala
   100 105 110
   Asp Val Ile Pro Val Arg Arg Arg Gly Asp Gly Arg Gly Ser Leu Leu
   115 120 125
   Ser Pro Arg Pro Ile Ser Tyr Leu Lys Gly Ser Ser Gly Gly Pro Leu
   130 135 140
   Leu Cys Pro Ala Gly His Ala Val Gly Ile Phe Arg Ala Ala Val Cys
   145 150 155 160
   Thr Arg Gly Val Ala Lys Ala Val Asp Phe Ile Pro Val Glu Ser Leu
   165 170 175
   Glu Thr Thr Met Arg Ser Pro Val Phe Ser Asp Asn Ser Ser Pro Pro
   180 185 190
   Ala Val Pro Gln Ser Tyr Gln Val Ala His Leu His Ala Pro Thr Gly
   195 200 205
   Ser Gly Lys Ser Thr Lys Val Pro Ala Ala Tyr Ala Ala Gln Gly Tyr
   210 215 220
   Lys Val Leu Val Leu Asn Pro Ser Val Ala Ala Thr Met Gly Phe Gly
   225 230 235 240
   Ala Tyr Met Ser Lys Ala His Gly Ile Asp Pro Asn Ile Arg Thr Gly
   245 250 255
   Val Arg Thr Ile Thr Thr Gly Ser Pro Ile Thr Tyr Ser Thr Tyr Gly
   260 265 270
   Lys Phe Leu Ala Asp Gly Gly Cys Ser Gly Gly Ala Tyr Asp Ile Ile
   275 280 285
   Ile Cys Asp Glu Cys His Ser Thr Asp Ala Thr Ser Ile Leu Gly Ile
   290 295 300
   Gly Thr Val Leu Asp Gln Ala Glu Thr Ala Gly Ala Arg Leu Thr Val
   305 310 315 320
   Leu Ala Thr Ala Thr Pro Pro Gly Ser Val Thr Val Pro His Pro Asn
   325 330 335
   Ile Glu Glu Val Ala Leu Ser Thr Thr Gly Glu Ile Pro Phe Tyr Gly
   340 345 350
   Lys Ala Ile Pro Leu Glu Ala Ile Lys Gly Gly Arg His Leu Ile Phe
   355 360 365
   Cys His Ser Lys Lys Lys Cys Asp Glu Leu Ala Ala Lys Leu Val Ala
   370 375 380
   Leu Gly Val Asn Ala Val Ala Tyr Tyr Arg Gly Leu Asp Val Ser Val
   385 390 395 400
   Ile Pro Thr Ser Gly Asp Val Val Val Val Ala Thr Asp Ala Leu Met
   405 410 415
   Thr Gly Phe Thr Gly Asp Phe Asp Ser Val Ile Asp Cys Asn Thr Cys
   420 425 430
   Val Thr Gln Thr Val Asp Phe Ser Leu Asp Pro Thr Phe Thr Ile Glu
   435 440 445
   Thr Ile Thr Leu Pro Gln Asp Ala Val Ser Arg Thr Gln Arg Arg Gly
   450 455 460
   Arg Thr Gly Arg Gly Lys Pro Gly Ile Tyr Arg Phe Val Ala Pro Gly
   465 470 475 480
   Glu Arg Pro Ser Gly Met Phe Asp Ser Ser Val Leu Cys Glu Cys Tyr
   485 490 495
   Asp Ala Gly Cys Ala Trp Tyr Glu Leu Thr Pro Ala Glu Thr Thr Val
   500 505 510
   Arg Leu Arg Ala Tyr Met Asn Thr Pro Gly Leu Pro Val Cys Gln Asp
               515                 520                 525
   His Leu Glu Phe Trp Glu Gly Val Phe Thr Gly Leu Thr His Ile Asp
           530                 535                 540
   Ala His Phe Leu Ser Gln Thr Lys Gln Ser Gly Glu Asn Leu Pro Tyr
   545                 550                 555                 560
   Leu Val Ala Tyr Gln Ala Thr Val Cys Ala Arg Ala Gln Ala Pro Pro
                   565                 570                 575
   Pro Ser Trp Asp Gln Met Trp Lys Cys Leu Ile Arg Leu Lys Pro Thr
               580                 585                 590
   Leu His Gly Pro Thr Pro Leu Leu Tyr Arg Leu Gly Ala Val Gln Asn
           595                 600                 605
   Glu Val Thr Leu Thr His Pro Val Thr Lys Tyr Ile Met Thr Cys Met
       610                 615                 620
   Ser Ala Asp Leu Glu Val Val Thr Gly Thr Trp Val Leu Val Gly Gly
   625                 630                 635                 640
   Val Leu Ala Ala Leu Ala Ala Tyr Cys Leu Ser Thr Gly Cys Val Val
                   645                 650                 655
   Ile Val Gly Arg Ile Val Leu Ser Gly Lys Pro Ala Ile Ile Pro Asp
               660                 665                 670
   Arg Glu Val Leu Tyr Arg Glu Phe Asp Glu Met Glu Glu Cys
           675                 680                 685
<210> 32
   <211> 686
   <212> PRT
   <213> Artificial Sequence
<220>
   <223> Mutant Hepatitis C virus NS3/4A
<400> 32
   Met Ala Pro Ile Thr Ala Tyr Ala Gln Gln Thr Arg Gly Leu Leu Gly
   1 5 10 15
   Cys Ile Ile Thr Ser Leu Thr Gly Arg Asp Lys Asn Gln Val Glu Gly
   20 25 30
   Glu Val Gln Ile Val Ser Thr Ala Ala Gln Thr Phe Leu Ala Thr Cys
   35 40 45
   Ile Asn Gly Val Cys Trp Thr Val Tyr His Gly Ala Gly Thr Arg Thr
   50 55 60
   Ile Ala Ser Pro Lys Gly Pro Val Ile Gln Met Tyr Thr Asn Val Asp
   65 70 75 80
   Gln Asp Leu Val Gly Trp Pro Ala Pro Gln Gly Ala Arg Ser Leu Thr
   85 90 95
   Pro Cys Thr Cys Gly Ser Ser Asp Leu Tyr Leu Val Thr Arg His Ala
   100 105 110
   Asp Val Ile Pro Val Arg Arg Arg Gly Asp Gly Arg Gly Ser Leu Leu
   115 120 125
   Ser Pro Arg Pro Ile Ser Tyr Leu Lys Gly Ser Ser Gly Gly Pro Leu
   130 135 140
   Leu Cys Pro Ala Gly His Ala Val Gly Ile Phe Arg Ala Ala Val Cys
   145 150 155 160
   Thr Arg Gly Val Ala Lys Ala Val Asp Phe Ile Pro Val Glu Ser Leu
   165 170 175
   Glu Thr Thr Met Arg Ser Pro Val Phe Ser Asp Asn Ser Ser Pro Pro
                   180                 185                 190
   Ala Val Pro Gln Ser Tyr Gln Val Ala His Leu His Ala Pro Thr Gly
               195                 200                 205
   Ser Gly Lys Ser Thr Lys Val Pro Ala Ala Tyr Ala Ala Gln Gly Tyr
           210                 215                 220
   Lys Val Leu Val Leu Asn Pro Ser Val Ala Ala Thr Met Gly Phe Gly
   225                 230                 235                 240
   Ala Tyr Met Ser Lys Ala His Gly Ile Asp Pro Asn Ile Arg Thr Gly
                       245                 250                 255
   Val Arg Thr Ile Thr Thr Gly Ser Pro Ile Thr Tyr Ser Thr Tyr Gly
                   260                 265                 270
   Lys Phe Leu Ala Asp Gly Gly Cys Ser Gly Gly Ala Tyr Asp Ile Ile
               275                 280                 285
   Ile Cys Asp Glu Cys His Ser Thr Asp Ala Thr Ser Ile Leu Gly Ile
           290                 295                 300
   Gly Thr Val Leu Asp Gln Ala Glu Thr Ala Gly Ala Arg Leu Thr Val
   305                 310                 315                 320
   Leu Ala Thr Ala Thr Pro Pro Gly Ser Val Thr Val Pro His Pro Asn
                       325                 330                 335
   Ile Glu Glu Val Ala Leu Ser Thr Thr Gly Glu Ile Pro Phe Tyr Gly
                   340                 345                 350
   Lys Ala Ile Pro Leu Glu Ala Ile Lys Gly Gly Arg His Leu Ile Phe
               355                 360                 365
   Cys His Ser Lys Lys Lys Cys Asp Glu Leu Ala Ala Lys Leu Val Ala
           370                 375                 380
   Leu Gly Val Asn Ala Val Ala Tyr Tyr Arg Gly Leu Asp Val Ser Val
   385                 390                 395                 400
   Ile Pro Thr Ser Gly Asp Val Val Val Val Ala Thr Asp Ala Leu Met
                       405                 410                 415
   Thr Gly Phe Thr Gly Asp Phe Asp Ser Val Ile Asp Cys Asn Thr Cys
                   420                 425                 430
   Val Thr Gln Thr Val Asp Phe Ser Leu Asp Pro Thr Phe Thr Ile Glu
               435                 440                 445
   Thr Ile Thr Leu Pro Gln Asp Ala Val Ser Arg Thr Gln Arg Arg Gly
           450                 455                 460
   Arg Thr Gly Arg Gly Lys Pro Gly Ile Tyr Arg Phe Val Ala Pro Gly
   465                 470                 475                 480
   Glu Arg Pro Ser Gly Met Phe Asp Ser Ser Val Leu Cys Glu Cys Tyr
                       485                 490                 495
   Asp Ala Gly Cys Ala Trp Tyr Glu Leu Thr Pro Ala Glu Thr Thr Val
                   500                 505                 510
   Arg Leu Arg Ala Tyr Met Asn Thr Pro Gly Leu Pro Val Cys Gln Asp
               515                 520                 525
   His Leu Glu Phe Trp Glu Gly Val Phe Thr Gly Leu Thr His Ile Asp
           530                 535                 540
   Ala His Phe Leu Ser Gln Thr Lys Gln Ser Gly Glu Asn Leu Pro Tyr
   545                 550                 555                 560
   Leu Val Ala Tyr Gln Ala Thr Val Cys Ala Arg Ala Gln Ala Pro Pro
                       565                 570                 575
   Pro Ser Trp Asp Gln Met Trp Lys Cys Leu Ile Arg Leu Lys Pro Thr
                   580                 585                 590
   Leu His Gly Pro Thr Pro Leu Leu Tyr Arg Leu Gly Ala Val Gln Asn
               595                 600                 605
   Glu Val Thr Leu Thr His Pro Val Thr Lys Tyr Ile Met Thr Cys Met
           610                 615                 620
   Ser Ala Asp Leu Glu Val Val Arg Gly Thr Trp Val Leu Val Gly Gly
   625 630 635 640
   Val Leu Ala Ala Leu Ala Ala Tyr Cys Leu Ser Thr Gly Cys Val Val
   645 650 655
   Ile Val Gly Arg Ile Val Leu Ser Gly Lys Pro Ala Ile Ile Pro Asp
   660 665 670
   Arg Glu Val Leu Tyr Arg Glu Phe Asp Glu Met Glu Glu Cys
   675 680 685
<210> 33
   <211> 25
   <212> PRT
   <213> Artificial Sequence
<220>
   <223> Mutant Hepatitis C virus NS3/4A peptide
<400> 33
   Thr Lys Tyr Met Thr Cys Met Ser Ala Asp Leu Glu Val Val Thr Pro
   1 5 10 15
   Thr Trp Val Leu Val Gly Gly Val Leu
   20 25
<210> 34
   <211> 25
   <212> PRT
   <213> Artificial Sequence
<220>
   <223> Mutant Hepatitis C virus NS3/4A peptide
<400> 34
   Thr Lys Tyr Met Thr Cys Met Ser Ala Asp Leu Glu Val Val Arg Pro
   1 5 10 15
   Thr Trp Val Leu Val Gly Gly Val Leu
   20 25
<210> 35
   <211> 25
   PRT
   <213> Artificial Sequence
<220>
   <223> Mutant Hepatitis C virus NS3/4A peptide
<400> 35
   Thr Lys Tyr Met Thr Cys Met Ser Ala Asp Leu Glu Val Val Arg Pro
   1               5                   10                  15
   Ala Trp Val Leu Val Gly Gly Val Leu
               20                  25
<210> 36
   <211> 25
   <212> PRT
   <213> Artificial Sequence
<220>
   <223> Mutant Hepatitis C virus NS3/4A peptide
<400> 36
   Thr Lys Tyr Met Thr Cys Met Ser Ala Asp Leu Glu Val Val Cys Ser
   1               5                   10                  15
   Thr Trp Val Leu Val Gly Gly Val Leu
               20                  25
<210> 37
   <211> 25
   <212> PRT
   <213> Artificial Sequence
<220>
   <223> Mutant Hepatitis C virus NS3/4A peptide
<400> 37
   Thr Lys Tyr Met Thr Cys Met Ser Ala Asp Leu Glu Val Cys Cys Ser
   1               5                   10                  15
   Thr Trp Val Leu Val Gly Gly Val Leu
               20                  25
<210> 38
   <211> 25
   <212> PRT
   <213> Artificial Sequence
<220>
   <223> Mutant Hepatitis C virus NS3/4A peptide
<400> 38
   Thr Lys Tyr Met Thr Cys Met Ser Ala Asp Leu Glu Val Ser Ser Ser
   1               5                   10                  15
   Thr Trp Val Leu Val Gly Gly Val Leu
               20                  25
<210> 39
   <211> 25
   <212> PRT
   <213> Artificial Sequence
<220>
   <223> Mutant Hepatitis C virus Ns3/4A peptide
<400> 39
   Thr Lys Tyr Met Thr Cys Met Ser Ala Asp Ser Ser Ser Ser Cys Ser
   1 5 10 15
   Thr Trp Val Leu Val Gly Gly Val Leu
   20 25
<210> 40
   <211> 25
   <212> PRT
   <213> Artificial Sequence
<220>
   <223> Mutant Hepatitis C virus NS3/4A peptide
<400> 40
   Thr Lys Tyr Met Thr Cys Met Ser Ala Asp Val Val Val Val Thr Ser
   1 5 10 15
   Thr Trp Val Leu Val Gly Gly Val Leu
   20 25
<210> 41
   <211> 16
   <212> PRT
   <213> Artificial Sequence
<220>
   <223> Hepatitis C virus NS5 peptide
<400> 41
   Ala Ser Glu Asp Val Val Cys Cys Ser Met Ser Tyr Thr Trp Thr Gly
   1 5 10 15
<210> 42
   <211> 18
   <212> PRT
   <213> Artificial Sequence
<220>
   <223> Mutant Hepatitis C virus NSSA/B peptide
<400> 42
   Ser Ser Glu Asp Val Val Cys Cys Ser Met Trp Val Leu Val Gly Gly
   1 5 10 15
   Val Leu
<210> 43
   <211> 686
   <212> PRT
   <213> Artificial Sequence
<220>
   <223> Mutant Hepatitis C virus NS3/4A
<400> 43
   Met Ala Pro Ile Thr Ala Tyr Ala Gln Gln Thr Arg Gly Leu Leu Gly
   1 5 10 15
   Cys Ile Ile Thr Ser Leu Thr Gly Arg Asp Lys Asn Gln Val Glu Gly
   20 25 30
   Glu Val Gln Ile Val Ser Thr Ala Ala Gln Thr Phe Leu Ala Thr Cys
   35 40 45
   Ile Asn Gly Val Cys Trp Thr Val Tyr His Gly Ala Gly Thr Arg Thr
   50 55 60
   Ile Ala Ser Pro Lys Gly Pro Val Ile Gln Met Tyr Thr Asn Val Asp
   65 70 75 80
   Gln Asp Leu Val Gly Trp Pro Ala Pro Gln Gly Ala Arg Ser Leu Thr
   85 90 95
   Pro Cys Thr Cys Gly Ser Ser Asp Leu Tyr Leu Val Thr Arg His Ala
   100 105 110
   Asp Val Ile Pro Val Arg Arg Arg Gly Asp Gly Arg Gly Ser Leu Leu
   115 120 125
   Ser Pro Arg Pro Ile Ser Tyr Leu Lys Gly Ser Ser Gly Gly Pro Leu
   130 135 140
   Leu Cys Pro Ala Gly His Ala Val Gly Ile Phe Arg Ala Ala Val Cys
   145 150 155 160
   Thr Arg Gly Val Ala Lys Ala Val Asp Phe Ile Pro Val Glu Ser Leu
   165 170 175
   Glu Thr Thr Met Arg Ser Pro Val Phe Ser Asp Asn Ser Ser Pro Pro
   180 185 190
   Ala Val Pro Gln Ser Tyr Gln Val Ala His Leu His Ala Pro Thr Gly
   195 200 205
   Ser Gly Lys Ser Thr Lys Val Pro Ala Ala Tyr Ala Ala Gln Gly Tyr
   210 215 220
   Lys Val Leu Val Leu Asn Pro Ser Val Ala Ala Thr Met Gly Phe Gly
   225 230 235 240
   Ala Tyr Met Ser Lys Ala His Gly Ile Asp Pro Asn Ile Arg Thr Gly
   245 250 255
   Val Arg Thr Ile Thr Thr Gly Ser Pro Ile Thr Tyr Ser Thr Tyr Gly
   260 265 270
   Lys Phe Leu Ala Asp Gly Gly Cys Ser Gly Gly Ala Tyr Asp Ile Ile
   275 280 285
   Ile Cys Asp Glu Cys His Ser Thr Asp Ala Thr Ser Ile Leu Gly Ile
   290 295 300
   Gly Thr Val Leu Asp Gln Ala Glu Thr Ala Gly Ala Arg Leu Thr Val
   305 310 315 320
   Leu Ala Thr Ala Thr Pro Pro Gly Ser Val Thr Val Pro His Pro Asn
   325 330 335
   Ile Glu Glu Val Ala Leu Ser Thr Thr Gly Glu Ile Pro Phe Tyr Gly
   340 345 350
   Lys Ala Ile Pro Leu Glu Ala Ile Lys Gly Gly Arg His Leu Ile Phe
   355 360 365
   Cys His Ser Lys Lys Lys Cys Asp Glu Leu Ala Ala Lys Leu Val Ala
   370 375 380
   Leu Gly Val Asn Ala Val Ala Tyr Tyr Arg Gly Leu Asp Val Ser Val
   385 390 395 400
   Ile Pro Thr Ser Gly Asp Val Val Val Val Ala Thr Asp Ala Leu Met
   405 410 415
   Thr Gly Phe Thr Gly Asp Phe Asp Ser Val Ile Asp Cys Asn Thr Cys
           420                 425                 430
   Val Thr Gln Thr Val Asp Phe Ser Leu Asp Pro Thr Phe Thr Ile Glu
       435                 440                 445
   Thr Ile Thr Leu Pro Gln Asp Ala Val Ser Arg Thr Gln Arg Arg Gly
       450                 455                 460
   Arg Thr Gly Arg Gly Lys Pro Gly Ile Tyr Arg Phe Val Ala Pro Gly
   465                 470                 475                 480
   Glu Arg Pro Ser Gly Met Phe Asp Ser Ser Val Leu Cys Glu Cys Tyr
                   485                 490                 495
   Asp Ala Gly Cys Ala Trp Tyr Glu Leu Thr Pro Ala Glu Thr Thr Val
               500                 505                 510
   Arg Leu Arg Ala Tyr Met Asn Thr Pro Gly Leu Pro Val Cys Gln Asp
           515                 520                 525
   His Leu Glu Phe Trp Glu Gly Val Phe Thr Gly Leu Thr His Ile Asp
       530                 535                 540
   Ala His Phe Leu Ser Gln Thr Lys Gln Ser Gly Glu Asn Leu Pro Tyr
   545                 550                 555                 560
   Leu Val Ala Tyr Gln Ala Thr Val Cys Ala Arg Ala Gln Ala Pro Pro
                   565                 570                 575
   Pro Ser Trp Asp Gln Met Trp Lys Cys Leu Ile Arg Leu Lys Pro Thr
               580                 585                 590
   Leu His Gly Pro Thr Pro Leu Leu Tyr Arg Leu Gly Ala Val Gln Asn
           595                 600                 605
   Glu Val Thr Leu Thr His Pro Val Thr Lys Tyr Ile Met Thr Cys Met
       610                 615                 620
   Ser Ala Asp Leu Glu Val Val Thr Pro Thr Trp Val Leu Val Gly Gly
   625                 630                 635                 640
   Val Leu Ala Ala Leu Ala Ala Tyr Cys Leu Ser Thr Gly Cys Val Val
                   645                 650                 655
   Ile Val Gly Arg Ile Val Leu Ser Gly Lys Pro Ala Ile Ile Pro Asp
               660                 665                 670
   Arg Glu Val Leu Tyr Arg Glu Phe Asp Glu Met Glu Glu Cys
           675                 680                 685
<210> 44
   <211> 686
   <212> PRT
   <213> Artificial Sequence
<220>
   <223> Mutant Hepatitis C virus NS3/4A
<400> 44
   Met Ala Pro Ile Thr Ala Tyr Ala Gln Gln Thr Arg Gly Leu Leu Gly
   1               5                   10                  15
   Cys Ile Ile Thr Ser Leu Thr Gly Arg Asp Lys Asn Gln Val Glu Gly
               20                  25                  30
   Glu Val Gln Ile Val Ser Thr Ala Ala Gln Thr Phe Leu Ala Thr Cys
           35                  40                  45
   Ile Asn Gly Val Cys Trp Thr Val Tyr His Gly Ala Gly Thr Arg Thr
       50                  55                  60
   Ile Ala Ser Pro Lys Gly Pro Val Ile Gln Met Tyr Thr Asn Val Asp
   65                  70                  75                  80
   Gln Asp Leu Val Gly Trp Pro Ala Pro Gln Gly Ala Arg Ser Leu Thr
                   85                  90                  95
   Pro Cys Thr Cys Gly Ser Ser Asp Leu Tyr Leu Val Thr Arg His Ala
               100                 105                 110
   Asp Val Ile Pro Val Arg Arg Arg Gly Asp Gly Arg Gly Ser Leu Leu
           115                 120                 125
   Ser Pro Arg Pro Ile Ser Tyr Leu Lys Gly Ser Ser Gly Gly Pro Leu
       130                 135                 140
   Leu Cys Pro Ala Gly His Ala Val Gly Ile Phe Arg Ala Ala Val Cys
   145                 150                 155                 160
   Thr Arg Gly Val Ala Lys Ala Val Asp Phe Ile Pro Val Glu Ser Leu
                   165                 170                 175
   Glu Thr Thr Met Arg Ser Pro Val Phe Ser Asp Asn Ser Ser Pro Pro
               180                 185                 190
   Ala Val Pro Gln Ser Tyr Gln Val Ala His Leu His Ala Pro Thr Gly
           195                 200                 205
   Ser Gly Lys Ser Thr Lys Val Pro Ala Ala Tyr Ala Ala Gln Gly Tyr
       210                 215                 220
   Lys Val Leu Val Leu Asn Pro Ser Val Ala Ala Thr Met Gly Phe Gly
   225                 230                 235                 240
   Ala Tyr Met Ser Lys Ala His Gly Ile Asp Pro Asn Ile Arg Thr Gly
                   245                 250                 255
   Val Arg Thr Ile Thr Thr Gly Ser Pro Ile Thr Tyr Ser Thr Tyr Gly
               260                 265                 270
   Lys Phe Leu Ala Asp Gly Gly Cys Ser Gly Gly Ala Tyr Asp Ile Ile
           275                 280                 285
   Ile Cys Asp Glu Cys His Ser Thr Asp Ala Thr Ser Ile Leu Gly Ile
       290                 295                 300
   Gly Thr Val Leu Asp Gln Ala Glu Thr Ala Gly Ala Arg Leu Thr Val
   305                 310                 315                 320
   Leu Ala Thr Ala Thr Pro Pro Gly Ser Val Thr Val Pro His Pro Asn
                   325                 330                 335
   Ile Glu Glu Val Ala Leu Ser Thr Thr Gly Glu Ile Pro Phe Tyr Gly
               340                 345                 350
   Lys Ala Ile Pro Leu Glu Ala Ile Lys Gly Gly Arg His Leu Ile Phe
           355                 360                 365
   Cys His Ser Lys Lys Lys Cys Asp Glu Leu Ala Ala Lys Leu Val Ala
       370                 375                 380
   Leu Gly Val Asn Ala Val Ala Tyr Tyr Arg Gly Leu Asp Val Ser Val
   385                 390                 395                 400
   Ile Pro Thr Ser Gly Asp Val Val Val Val Ala Thr Asp Ala Leu Met
                   405                 410                 415
   Thr Gly Phe Thr Gly Asp Phe Asp Ser Val Ile Asp Cys Asn Thr Cys
               420                 425                 430
   Val Thr Gln Thr Val Asp Phe Ser Leu Asp Pro Thr Phe Thr Ile Glu
           435                 440                 445
   Thr Ile Thr Leu Pro Gln Asp Ala Val Ser Arg Thr Gln Arg Arg Gly
       450                 455                 460
   Arg Thr Gly Arg Gly Lys Pro Gly Ile Tyr Arg Phe Val Ala Pro Gly
   465                 470                 475                 480
   Glu Arg Pro Ser Gly Met Phe Asp Ser Ser Val Leu Cys Glu Cys Tyr
                   485                 490                 495
   Asp Ala Gly Cys Ala Trp Tyr Glu Leu Thr Pro Ala Glu Thr Thr Val
               500                 505                 510
   Arg Leu Arg Ala Tyr Met Asn Thr Pro Gly Leu Pro Val Cys Gln Asp
           515                 520                 525
   His Leu Glu Phe Trp Glu Gly Val Phe Thr Gly Leu Thr His Ile Asp
   530 535 540
   Ala His Phe Leu Ser Gln Thr Lys Gln Ser Gly Glu Asn Leu Pro Tyr
   545 550 555 560
   Leu Val Ala Tyr Gln Ala Thr Val Cys Ala Arg Ala Gln Ala Pro Pro
   565 570 575
   Pro Ser Trp Asp Gln Met Trp Lys Cys Leu Ile Arg Leu Lys Pro Thr
   580 585 590
   Leu His Gly Pro Thr Pro Leu Leu Tyr Arg Leu Gly Ala Val Gln Asn
   595 600 605
   Glu Val Thr Leu Thr His Pro Val Thr Lys Tyr Ile Met Thr Cys Met
   610 615 620
   Ser Ala Asp Leu Glu Val Val Arg Pro Thr Trp Val Leu Val Gly Gly
   625 630 635 640
   Val Leu Ala Ala Leu Ala Ala Tyr Cys Leu Ser Thr Gly Cys Val Val
   645 650 655
   Ile Val Gly Arg Ile Val Leu Ser Gly Lys Pro Ala Ile Ile Pro Asp
   660 665 670
   Arg Glu Val Leu Tyr Arg Glu Phe Asp Glu Met Glu Glu Cys
   675 680 685
<210> 45
   <211> 686
   <212> PRT
   <213> Artificial Sequence
<220>
   <223> Mutant Hepatitis C virus NS3/4A
<400> 45
   Met Ala Pro Ile Thr Ala Tyr Ala Gln Gln Thr Arg Gly Leu Leu Gly
   1 5 10 15
   Cys Ile Ile Thr Ser Leu Thr Gly Arg Asp Lys Asn Gln Val Glu Gly
   20 25 30
   Glu Val Gln Ile Val Ser Thr Ala Ala Gln Thr Phe Leu Ala Thr Cys
   35 40 45
   Ile Asn Gly Val Cys Trp Thr Val Tyr His Gly Ala Gly Thr Arg Thr
   50 55 60
   Ile Ala Ser Pro Lys Gly Pro Val Ile Gln Met Tyr Thr Asn Val Asp
   65 70 75 80
   Gln Asp Leu Val Gly Trp Pro Ala Pro Gln Gly Ala Arg Ser Leu Thr
   85 90 95
   Pro Cys Thr Cys Gly Ser Ser Asp Leu Tyr Leu Val Thr Arg His Ala
   100 105 110
   Asp Val Ile Pro Val Arg Arg Arg Gly Asp Gly Arg Gly Ser Leu Leu
   115 120 125
   Ser Pro Arg Pro Ile Ser Tyr Leu Lys Gly Ser Ser Gly Gly Pro Leu
   130 135 140
   Leu Cys Pro Ala Gly His Ala Val Gly Ile Phe Arg Ala Ala Val Cys
   145 150 155 160
   Thr Arg Gly Val Ala Lys Ala Val Asp Phe Ile Pro Val Glu Ser Leu
   165 170 175
   Glu Thr Thr Met Arg Ser Pro Val Phe Ser Asp Asn Ser Ser Pro Pro
   180 185 190
   Ala Val Pro Gln Ser Tyr Gln Val Ala His Leu His Ala Pro Thr Gly
           195                 200                 205
   Ser Gly Lys Ser Thr Lys Val Pro Ala Ala Tyr Ala Ala Gln Gly Tyr
       210                 215                 220
   Lys Val Leu Val Leu Asn Pro Ser Val Ala Ala Thr Met Gly Phe Gly
   225                 230                 235                 240
   Ala Tyr Met Ser Lys Ala His Gly Ile Asp Pro Asn Ile Arg Thr Gly
                   245                 250                 255
   Val Arg Thr Ile Thr Thr Gly Ser Pro Ile Thr Tyr Ser Thr Tyr Gly
               260                 265                 270
   Lys Phe Leu Ala Asp Gly Gly Cys Ser Gly Gly Ala Tyr Asp Ile Ile
           275                 280                 285
   Ile Cys Asp Glu Cys His Ser Thr Asp Ala Thr Ser Ile Leu Gly Ile
       290                 295                 300
   Gly Thr Val Leu Asp Gln Ala Glu Thr Ala Gly Ala Arg Leu Thr Val
   305                 310                 315                 320
   Leu Ala Thr Ala Thr Pro Pro Gly Ser Val Thr Val Pro His Pro Asn
                   325                 330                 335
   Ile Glu Glu Val Ala Leu Ser Thr Thr Gly Glu Ile Pro Phe Tyr Gly
               340                 345                 350
   Lys Ala Ile Pro Leu Glu Ala Ile Lys Gly Gly Arg His Leu Ile Phe
           355                 360                 365
   Cys His Ser Lys Lys Lys Cys Asp Glu Leu Ala Ala Lys Leu Val Ala
       370                 375                 380
   Leu Gly Val Asn Ala Val Ala Tyr Tyr Arg Gly Leu Asp Val Ser Val
   385                 390                 395                 400
   Ile Pro Thr Ser Gly Asp Val Val Val Val Ala Thr Asp Ala Leu Met
                   405                 410                 415
   Thr Gly Phe Thr Gly Asp Phe Asp Ser Val Ile Asp Cys Asn Thr Cys
               420                 425                 430
   Val Thr Gln Thr Val Asp Phe Ser Leu Asp Pro Thr Phe Thr Ile Glu
           435                 440                 445
   Thr Ile Thr Leu Pro Gln Asp Ala Val Ser Arg Thr Gln Arg Arg Gly
       450                 455                 460
   Arg Thr Gly Arg Gly Lys Pro Gly Ile Tyr Arg Phe Val Ala Pro Gly
   465                 470                 475                 480
   Glu Arg Pro Ser Gly Met Phe Asp Ser Ser Val Leu Cys Glu Cys Tyr
                   485                 490                 495
   Asp Ala Gly Cys Ala Trp Tyr Glu Leu Thr Pro Ala Glu Thr Thr Val
               500                 505                 510
   Arg Leu Arg Ala Tyr Met Asn Thr Pro Gly Leu Pro Val Cys Gln Asp
           515                 520                 525
   His Leu Glu Phe Trp Glu Gly Val Phe Thr Gly Leu Thr His Ile Asp
       530                 535                 540
   Ala His Phe Leu Ser Gln Thr Lys Gln Ser Gly Glu Asn Leu Pro Tyr
   545                 550                 555                 560
   Leu Val Ala Tyr Gln Ala Thr Val Cys Ala Arg Ala Gln Ala Pro Pro
                   565                 570                 575
   Pro Ser Trp Asp Gln Met Trp Lys Cys Leu Ile Arg Leu Lys Pro Thr
               580                 585                 590
   Leu His Gly Pro Thr Pro Leu Leu Tyr Arg Leu Gly Ala Val Gln Asn
           595                 600                 605
   Glu Val Thr Leu Thr His Pro Val Thr Lys Tyr Ile Met Thr Cys Met
       610                 615                 620
   Ser Ala Asp Leu Glu Val Val Arg Pro Ala Trp Val Leu Val Gly Gly
   625 630 635 640
   Val Leu Ala Ala Leu Ala Ala Tyr Cys Leu Ser Thr Gly Cys Val Val
   645 650 655
   Ile Val Gly Arg Ile Val Leu Ser Gly Lys Pro Ala Ile Ile Pro Asp
   660 665 670
   Arg Glu Val Leu Tyr Arg Glu Phe Asp Glu Met Glu Glu Cys
   675 680 685
<210> 46
   <211> 686
   <212> PRT
   <213> Artificial Sequence
<220>
   <223> Mutant Hepatitis C virus NS3/4A
<400> 46
   Met Ala Pro Ile Thr Ala Tyr Ala Gln Gln Thr Arg Gly Leu Leu Gly
   1 5 10 15
   Cys Ile Ile Thr Ser Leu Thr Gly Arg Asp Lys Asn Gln Val Glu Gly
   20 25 30
   Glu Val Gln Ile Val Ser Thr Ala Ala Gln Thr Phe Leu Ala Thr Cys
   35 40 45
   Ile Asn Gly Val Cys Trp Thr Val Tyr His Gly Ala Gly Thr Arg Thr
   50 55 60
   Ile Ala Ser Pro Lys Gly Pro Val Ile Gln Met Tyr Thr Asn Val Asp
   65 70 75 80
   Gln Asp Leu Val Gly Trp Pro Ala Pro Gln Gly Ala Arg Ser Leu Thr
   85 90 95
   Pro Cys Thr Cys Gly Ser Ser Asp Leu Tyr Leu Val Thr Arg His Ala
   100 105 110
   Asp Val Ile Pro Val Arg Arg Arg Gly Asp Gly Arg Gly Ser Leu Leu
   115 120 125
   Ser Pro Arg Pro Ile Ser Tyr Leu Lys Gly Ser Ser Gly Gly Pro Leu
   130 135 140
   Leu Cys Pro Ala Gly His Ala Val Gly Ile Phe Arg Ala Ala Val Cys
   145 150 155 160
   Thr Arg Gly Val Ala Lys Ala Val Asp Phe Ile Pro Val Glu Ser Leu
   165 170 175
   Glu Thr Thr Met Arg Ser Pro Val Phe Ser Asp Asn Ser Ser Pro Pro
   180 185 190
   Ala Val Pro Gln Ser Tyr Gln Val Ala His Leu His Ala Pro Thr Gly
   195 200 205
   Ser Gly Lys Ser Thr Lys Val Pro Ala Ala Tyr Ala Ala Gln Gly Tyr
   210 215 220
   Lys Val Leu Val Leu Asn Pro Ser Val Ala Ala Thr Met Gly Phe Gly
   225 230 235 240
   Ala Tyr Met Ser Lys Ala His Gly Ile Asp Pro Asn Ile Arg Thr Gly
   245 250 255
   Val Arg Thr Ile Thr Thr Gly Ser Pro Ile Thr Tyr Ser Thr Tyr Gly
   260 265 270
   Lys Phe Leu Ala Asp Gly Gly Cys Ser Gly Gly Ala Tyr Asp Ile Ile
   275 280 285
   Ile Cys Asp Glu Cys His Ser Thr Asp Ala Thr Ser Ile Leu Gly Ile
           290                 295                 300
   Gly Thr Val Leu Asp Gln Ala Glu Thr Ala Gly Ala Arg Leu Thr Val
   305                 310                 315                 320
   Leu Ala Thr Ala Thr Pro Pro Gly Ser Val Thr Val Pro His Pro Asn
                   325                 330                 335
   Ile Glu Glu Val Ala Leu Ser Thr Thr Gly Glu Ile Pro Phe Tyr Gly
               340                 345                 350
   Lys Ala Ile Pro Leu Glu Ala Ile Lys Gly Gly Arg His Leu Ile Phe
           355                 360                 365
   Cys His Ser Lys Lys Lys Cys Asp Glu Leu Ala Ala Lys Leu Val Ala
       370                 375                 380
   Leu Gly Val Asn Ala Val Ala Tyr Tyr Arg Gly Leu Asp Val Ser Val
   385                 390                 395                 400
   Ile Pro Thr Ser Gly Asp Val Val Val Val Ala Thr Asp Ala Leu Met
                   405                 410                 415
   Thr Gly Phe Thr Gly Asp Phe Asp Ser Val Ile Asp Cys Asn Thr Cys
               420                 425                 430
   Val Thr Gln Thr Val Asp Phe Ser Leu Asp Pro Thr Phe Thr Ile Glu
           435                 440                 445
   Thr Ile Thr Leu Pro Gln Asp Ala Val Ser Arg Thr Gln Arg Arg Gly
       450                 455                 460
   Arg Thr Gly Arg Gly Lys Pro Gly Ile Tyr Arg Phe Val Ala Pro Gly
   465                 470                 475                 480
   Glu Arg Pro Ser Gly Met Phe Asp Ser Ser Val Leu Cys Glu Cys Tyr
                   485                 490                 495
   Asp Ala Gly Cys Ala Trp Tyr Glu Leu Thr Pro Ala Glu Thr Thr Val
               500                 505                 510
   Arg Leu Arg Ala Tyr Met Asn Thr Pro Gly Leu Pro Val Cys Gln Asp
           515                 520                 525
   His Leu Glu Phe Trp Glu Gly Val Phe Thr Gly Leu Thr His Ile Asp
       530                 535                 540
   Ala His Phe Leu Ser Gln Thr Lys Gln Ser Gly Glu Asn Leu Pro Tyr
   545                 550                 555                 560
   Leu Val Ala Tyr Gln Ala Thr Val Cys Ala Arg Ala Gln Ala Pro Pro
                   565                 570                 575
   Pro Ser Trp Asp Gln Met Trp Lys Cys Leu Ile Arg Leu Lys Pro Thr
               580                 585                 590
   Leu His Gly Pro Thr Pro Leu Leu Tyr Arg Leu Gly Ala Val Gln Asn
           595                 600                 605
   Glu Val Thr Leu Thr His Pro Val Thr Lys Tyr Ile Met Thr Cys Met
       610                 615                 620
   Ser Ala Asp Leu Glu Val Val Cys Ser Thr Trp Val Leu Val Gly Gly
   625                 630                 635                 640
   Val Leu Ala Ala Leu Ala Ala Tyr Cys Leu Ser Thr Gly Cys Val Val
                   645                 650                 655
   Ile Val Gly Arg Ile Val Leu Ser Gly Lys Pro Ala Ile Ile Pro Asp
               660                 665                 670
   Arg Glu Val Leu Tyr Arg Glu Phe Asp Glu Met Glu Glu Cys
           675                 680                 685
<210> 47
   <211> 686
   <212> PRT
   <213> Artificial Sequence
<220>
   <223> Mutant Hepatitis C₁-virus NS3/4A
<400> 47
   Met Ala Pro Ile Thr Ala Tyr Ala Gln Gln Thr Arg Gly Leu Leu Gly
   1               5                   10                  15
   Cys Ile Ile Thr Ser Leu Thr Gly Arg Asp Lys Asn Gln Val Glu Gly
               20                  25                  30
   Glu Val Gln Ile Val Ser Thr Ala Ala Gln Thr Phe Leu Ala Thr Cys
           35                  40                  45
   Ile Asn Gly Val Cys Trp Thr Val Tyr His Gly Ala Gly Thr Arg Thr
       50                  55                  60
   Ile Ala Ser Pro Lys Gly Pro Val Ile Gln Met Tyr Thr Asn Val Asp
   65                  70                  75                  80
   Gln Asp Leu Val Gly Trp Pro Ala Pro Gln Gly Ala Arg Ser Leu Thr
                   85                  90                  95
   Pro Cys Thr Cys Gly Ser Ser Asp Leu Tyr Leu Val Thr Arg His Ala
               100                 105                 110
   Asp Val Ile Pro Val Arg Arg Arg Gly Asp Gly Arg Gly Ser Leu Leu
           115                 120                 125
   Ser Pro Arg Pro Ile Ser Tyr Leu Lys Gly Ser Ser Gly Gly Pro Leu
       130                 135                 140
   Leu Cys Pro Ala Gly His Ala Val Gly Ile Phe Arg Ala Ala Val Cys
   145                 150                 155                 160
   Thr Arg Gly Val Ala Lys Ala Val Asp Phe Ile Pro Val Glu Ser Leu
                   165                 170                 175
   Glu Thr Thr Met Arg Ser Pro Val Phe Ser Asp Asn Ser Ser Pro Pro
               180                 185                 190
   Ala Val Pro Gln Ser Tyr Gln Val Ala His Leu His Ala Pro Thr Gly
           195                 200                 205
   Ser Gly Lys Ser Thr Lys Val Pro Ala Ala Tyr Ala Ala Gln Gly Tyr
       210                 215                 220
   Lys Val Leu Val Leu Asn Pro Ser Val Ala Ala Thr Met Gly Phe Gly
   225                 230                 235                 240
   Ala Tyr Met Ser Lys Ala His Gly Ile Asp Pro Asn Ile Arg Thr Gly
                   245                 250                 255
   Val Arg Thr Ile Thr Thr Gly Ser Pro Ile Thr Tyr Ser Thr Tyr Gly
               260                 265                 270
   Lys Phe Leu Ala Asp Gly Gly Cys Ser Gly Gly Ala Tyr Asp Ile Ile
           275                 280                 285
   Ile Cys Asp Glu Cys His Ser Thr Asp Ala Thr Ser Ile Leu Gly Ile
       290                 295                 300
   Gly Thr Val Leu Asp Gln Ala Glu Thr Ala Gly Ala Arg Leu Thr Val
   305                 310                 315                 320
   Leu Ala Thr Ala Thr Pro Pro Gly Ser Val Thr Val Pro His Pro Asn
                   325                 330                 335
   Ile Glu Glu Val Ala Leu Ser Thr Thr Gly Glu Ile Pro Phe Tyr Gly
               340                 345                 350
   Lys Ala Ile Pro Leu Glu Ala Ile Lys Gly Gly Arg His Leu Ile Phe
           355                 360                 365
   Cys His Ser Lys Lys Lys Cys Asp Glu Leu Ala Ala Lys Leu Val Ala
       370                 375                 380
   Leu Gly Val Asn Ala Val Ala Tyr Tyr Arg Gly Leu Asp Val Ser Val
   385                 390                 395                 400
   Ile Pro Thr Ser Gly Asp Val Val Val Val Ala Thr Asp Ala Leu Met
   405 410 415
   Thr Gly Phe Thr Gly Asp Phe Asp Ser Val Ile Asp Cys Asn Thr Cys
   420 425 430
   Val Thr Gln Thr Val Asp Phe Ser Leu Asp Pro Thr Phe Thr Ile Glu
   435 440 445
   Thr Ile Thr Leu Pro Gln Asp Ala Val Ser Arg Thr Gln Arg Arg Gly
   450 455 460
   Arg Thr Gly Arg Gly Lys Pro Gly Ile Tyr Arg Phe Val Ala Pro Gly
   465 470 475 480
   Glu Arg Pro Ser Gly Met Phe Asp Ser Ser Val Leu Cys Glu Cys Tyr
   485 490 495
   Asp Ala Gly Cys Ala Trp Tyr Glu Leu Thr Pro Ala Glu Thr Thr Val
   500 505 510
   Arg Leu Arg Ala Tyr Met Asn Thr Pro Gly Leu Pro Val Cys Gln Asp
   515 520 525
   His Leu Glu Phe Trp Glu Gly Val Phe Thr Gly Leu Thr His Ile Asp
   530 535 540
   Ala His Phe Leu Ser Gln Thr Lys Gln Ser Gly Glu Asn Leu Pro Tyr
   545 550 555 560
   Leu Val Ala Tyr Gln Ala Thr Val Cys Ala Arg Ala Gln Ala Pro Pro
   565 570 575
   Pro Ser Trp Asp Gln Met Trp Lys Cys Leu Ile Arg Leu Lys Pro Thr
   580 585 590
   Leu His Gly Pro Thr Pro Leu Leu Tyr Arg Leu Gly Ala Val Gln Asn
   595 600 605
   Glu Val Thr Leu Thr His Pro Val Thr Lys Tyr Ile Met Thr Cys Met
   610 615 620
   Ser Ala Asp Leu Glu Val Cys Cys Ser Thr Trp Val Leu Val Gly Gly
   625 630 635 640
   Val Leu Ala Ala Leu Ala Ala Tyr Cys Leu Ser Thr Gly Cys Val Val
   645 650 655
   Ile Val Gly Arg Ile Val Leu Ser Gly Lys Pro Ala Ile Ile Pro Asp
   660 665 670
   Arg Glu Val Leu Tyr Arg Glu Phe Asp Glu Met Glu Glu Cys
   675 680 685
<210> 48
   <211> 686
   <212> PRT
   <213> Artificial Sequence
<220>
   <223> Mutant Hepatitis C virus NS3/4A
<400> 48
   Met Ala Pro Ile Thr Ala Tyr Ala Gln Gln Thr Arg Gly Leu Leu Gly
   1 5 10 15
   Cys Ile Ile Thr Ser Leu Thr Gly Arg Asp Lys Asn Gln Val Glu Gly
   20 25 30
   Glu Val Gln Ile Val Ser Thr Ala Ala Gln Thr Phe Leu Ala Thr Cys
   35 40 45
   Ile Asn Gly Val Cys Trp Thr Val Tyr His Gly Ala Gly Thr Arg Thr
   50 55 60
   Ile Ala Ser Pro Lys Gly Pro Val Ile Gln Met Tyr Thr Asn Val Asp
   65 70 75 80
   Gln Asp Leu Val Gly Trp Pro Ala Pro Gln Gly Ala Arg Ser Leu Thr
   85 90 95
   Pro Cys Thr Cys Gly Ser Ser Asp Leu Tyr Leu Val Thr Arg His Ala
   100 105 110
   Asp Val Ile Pro Val Arg Arg Arg Gly Asp Gly Arg Gly Ser Leu Leu
   115 120 125
   Ser Pro Arg Pro Ile Ser Tyr Leu Lys Gly Ser Ser Gly Gly Pro Leu
   130 135 140
   Leu Cys Pro Ala Gly His Ala Val Gly Ile Phe Arg Ala Ala Val Cys
   145 150 155 160
   Thr Arg Gly Val Ala Lys Ala Val Asp Phe Ile Pro Val Glu Ser Leu
   165 170 175
   Glu Thr Thr Met Arg Ser Pro Val Phe Ser Asp Asn Ser Ser Pro Pro
   180 185 190
   Ala Val Pro Gln Ser Tyr Gln Val Ala His Leu His Ala Pro Thr Gly
   195 200 205
   Ser Gly Lys Ser Thr Lys Val Pro Ala Ala Tyr Ala Ala Gln Gly Tyr
   210 215 220
   Lys Val Leu Val Leu Asn Pro Ser Val Ala Ala Thr Met Gly Phe Gly
   225 230 235 240
   Ala Tyr Met Ser Lys Ala His Gly Ile Asp Pro Asn Ile Arg Thr Gly
   245 250 255
   Val Arg Thr Ile Thr Thr Gly Ser Pro Ile Thr Tyr Ser Thr Tyr Gly
   260 265 270
   Lys Phe Leu Ala Asp Gly Gly Cys Ser Gly Gly Ala Tyr Asp Ile Ile
   275 280 285
   Ile Cys Asp Glu Cys His Ser Thr Asp Ala Thr Ser Ile Leu Gly Ile
   290 295 300
   Gly Thr Val Leu Asp Gln Ala Glu Thr Ala Gly Ala Arg Leu Thr Val
   305 310 315 320
   Leu Ala Thr Ala Thr Pro Pro Gly Ser Val Thr Val Pro His Pro Asn
   325 330 335
   Ile Glu Glu Val Ala Leu Ser Thr Thr Gly Glu Ile Pro Phe Tyr Gly
   340 345 350
   Lys Ala Ile Pro Leu Glu Ala Ile Lys Gly Gly Arg His Leu Ile Phe
   355 360 365
   Cys His Ser Lys Lys Lys Cys Asp Glu Leu Ala Ala Lys Leu Val Ala
   370 375 380
   Leu Gly Val Asn Ala Val Ala Tyr Tyr Arg Gly Leu Asp Val Ser Val
   385 390 395 400
   Ile Pro Thr Ser Gly Asp Val Val Val Val Ala Thr Asp Ala Leu Met
   405 410 415
   Thr Gly Phe Thr Gly Asp Phe Asp Ser Val Ile Asp Cys Asn Thr Cys
   420 425 430
   Val Thr Gln Thr Val Asp Phe Ser Leu Asp Pro Thr Phe Thr Ile Glu
   435 440 445
   Thr Ile Thr Leu Pro Gln Asp Ala Val Ser Arg Thr Gln Arg Arg Gly
   450 455 460
   Arg Thr Gly Arg Gly Lys Pro Gly Ile Tyr Arg Phe Val Ala Pro Gly
   465 470 475 480
   Glu Arg Pro Ser Gly Met Phe Asp Ser Ser Val Leu Cys Glu Cys Tyr
   485 490 495
   Asp Ala Gly Cys Ala Trp Tyr Glu Leu Thr Pro Ala Glu Thr Thr Val
                   500                 505                 510
   Arg Leu Arg Ala Tyr Met Asn Thr Pro Gly Leu Pro Val Cys Gln Asp
               515                 520                 525
   His Leu Glu Phe Trp Glu Gly Val Phe Thr Gly Leu Thr His Ile Asp
           530                 535                 540
   Ala His Phe Leu Ser Gln Thr Lys Gln Ser Gly Glu Asn Leu Pro Tyr
   545                 550                 555                 560
   Leu Val Ala Tyr Gln Ala Thr Val Cys Ala Arg Ala Gln Ala Pro Pro
                   565                 570                 575
   Pro Ser Trp Asp Gln Met Trp Lys Cys Leu Ile Arg Leu Lys Pro Thr
               580                 585                 590
   Leu His Gly Pro Thr Pro Leu Leu Tyr Arg Leu Gly Ala Val Gln Asn
           595                 600                 605
   Glu Val Thr Leu Thr His Pro Val Thr Lys Tyr Ile Met Thr Cys Met
       610                 615                 620
   Ser Ala Asp Leu Glu Val Ser Ser Ser Thr Trp Val Leu Val Gly Gly
   625                 630                 635                 640
   Val Leu Ala Ala Leu Ala Ala Tyr Cys Leu Ser Thr Gly Cys Val Val
                   645                 650                 655
   Ile Val Gly Arg Ile Val Leu Ser Gly Lys Pro Ala Ile Ile Pro Asp
               660                 665                 670
   Arg Glu Val Leu Tyr Arg Glu Phe Asp Glu Met Glu Glu Cys
           675                 680                 685
<210> 49
   <211> 686
   <212> PRT
   <213> Artificial Sequence
<220>
   <223> Mutant Hepatitis C virus NS3/4A
<400> 49
   Met Ala Pro Ile Thr Ala Tyr Ala Gln Gln Thr Arg Gly Leu Leu Gly
   1               5                   10                  15
   Cys Ile Ile Thr Ser Leu Thr Gly Arg Asp Lys Asn Gln Val Glu Gly
               20                  25                  30
   Glu Val Gln Ile Val Ser Thr Ala Ala Gln Thr Phe Leu Ala Thr Cys
           35                  40                  45
   Ile Asn Gly Val Cys Trp Thr Val Tyr His Gly Ala Gly Thr Arg Thr
       50                  55                  60
   Ile Ala Ser Pro Lys Gly Pro Val Ile Gln Met Tyr Thr Asn Val Asp
   65                  70                  75                  80
   Gln Asp Leu Val Gly Trp Pro Ala Pro Gln Gly Ala Arg Ser Leu Thr
                   85                  90                  95
   Pro Cys Thr Cys Gly Ser Ser Asp Leu Tyr Leu Val Thr Arg His Ala
               100                 105                 110
   Asp Val Ile Pro Val Arg Arg Arg Gly Asp Gly Arg Gly Ser Leu Leu
           115                 120                 125
   Ser Pro Arg Pro Ile Ser Tyr Leu Lys Gly Ser Ser Gly Gly Pro Leu
       130                 135                 140
   Leu Cys Pro Ala Gly His Ala Val Gly Ile Phe Arg Ala Ala Val Cys
   145                 150                 155                 160
   Thr Arg Gly Val Ala Lys Ala Val Asp Phe Ile Pro Val Glu Ser Leu
   165 170 175
   Glu Thr Thr Met Arg Ser Pro Val Phe Ser Asp Asn Ser Ser Pro Pro
   180 185 190
   Ala Val Pro Gln Ser Tyr Gln Val Ala His Leu His Ala Pro Thr Gly
   195 200 205
   Ser Gly Lys Ser Thr Lys Val Pro Ala Ala Tyr Ala Ala Gln Gly Tyr
   210 215 220
   Lys Val Leu Val Leu Asn Pro Ser Val Ala Ala Thr Met Gly Phe Gly
   225 230 235 240
   Ala Tyr Met Ser Lys Ala His Gly Ile Asp Pro Asn Ile Arg Thr Gly
   245 250 255
   Val Arg Thr Ile Thr Thr Gly Ser Pro Ile Thr Tyr Ser Thr Tyr Gly
   260 265 270
   Lys Phe Leu Ala Asp Gly Gly Cys Ser Gly Gly Ala Tyr Asp Ile Ile
   275 280 285
   Ile Cys Asp Glu Cys His Ser Thr Asp Ala Thr Ser Ile Leu Gly Ile
   290 295 300
   Gly Thr Val Leu Asp Gln Ala Glu Thr Ala Gly Ala Arg Leu Thr Val
   305 310 315 320
   Leu Ala Thr Ala Thr Pro Pro Gly Ser Val Thr Val Pro His Pro Asn
   325 330 335
   Ile Glu Glu Val Ala Leu Ser Thr Thr Gly Glu Ile Pro Phe Tyr Gly
   340 345 350
   Lys Ala Ile Pro Leu Glu Ala Ile Lys Gly Gly Arg His Leu Ile Phe
   355 360 365
   Cys His Ser Lys Lys Lys Cys Asp Glu Leu Ala Ala Lys Leu Val Ala
   370 375 380
   Leu Gly Val Asn Ala Val Ala Tyr Tyr Arg Gly Leu Asp Val Ser Val
   385 390 395 400
   Ile Pro Thr Ser Gly Asp Val Val Val Val Ala Thr Asp Ala Leu Met
   405 410 415
   Thr Gly Phe Thr Gly Asp Phe Asp Ser Val Ile Asp Cys Asn Thr Cys
   420 425 430
   Val Thr Gln Thr Val Asp Phe Ser Leu Asp Pro Thr Phe Thr Ile Glu
   435 440 445
   Thr Ile Thr Leu Pro Gln Asp Ala Val Ser Arg Thr Gln Arg Arg Gly
   450 455 460
   Arg Thr Gly Arg Gly Lys Pro Gly Ile Tyr Arg Phe Val Ala Pro Gly
   465 470 475 480
   Glu Arg Pro Ser Gly Met Phe Asp Ser Ser Val Leu Cys Glu Cys Tyr
   485 490 495
   Asp Ala Gly Cys Ala Trp Tyr Glu Leu Thr Pro Ala Glu Thr Thr Val
   500 505 510
   Arg Leu Arg Ala Tyr Met Asn Thr Pro Gly Leu Pro Val Cys Gln Asp
   515 520 525
   His Leu Glu Phe Trp Glu Gly Val Phe Thr Gly Leu Thr His Ile Asp
   530 535 540
   Ala His Phe Leu Ser Gln Thr Lys Gln Ser Gly Glu Asn Leu Pro Tyr
   545 550 555 560
   Leu Val Ala Tyr Gln Ala Thr Val Cys Ala Arg Ala Gln Ala Pro Pro
   565 570 575
   Pro Ser Trp Asp Gln Met Trp Lys Cys Leu Ile Arg Leu Lys Pro Thr
   580 585 590
   Leu His Gly Pro Thr Pro Leu Leu Tyr Arg Leu Gly Ala Val Gln Asn
   595 600 605
   Glu Val Thr Leu Thr His Pro Val Thr Lys Tyr Ile Met Thr Cys Met
       610                 615                 620
   Ser Ala Asp Ser Ser Ser Ser Cys Ser Thr Trp Val Leu Val Gly Gly
   625                 630                 635                 640
   Val Leu Ala Ala Leu Ala Ala Tyr Cys Leu Ser Thr Gly Cys Val Val
                   645                 650                 655
   Ile Val Gly Arg Ile Val Leu Ser Gly Lys Pro Ala Ile Ile Pro Asp
               660                 665                 670
   Arg Glu Val Leu Tyr Arg Glu Phe Asp Glu Met Glu Glu Cys
           675                 680                 685

## Description

### FIELD OF THE INVENTION

The present invention relates to compositions for enhancing the effect of vaccines in animals, such as domestic, sport, or pet species, and humans. Disclosure is made of the use of ribavirin as an adjuvant and compositions having ribavirin and an antigen.

### BACKGROUND OF THE INVENTION

The use of vaccines to prevent disease in humans, farm livestock, sports animals, and household pets is a common practice. Frequently, however, the antigen used in a vaccine is not sufficiently immunogenic to raise the antibody titre to levels that are sufficient to provide protection against subsequent challenge or to maintain the potential for mounting these levels over extended time periods. Further, many vaccines are altogether deficient in inducing cell-mediated immunity, which is a primary immune defense against bacterial and viral infection. A considerable amount of research is currently focussed on the development of more potent vaccines and ways to enhance the immunogenicity of antigen-containing preparations. (*See e.g.*, U.S. Pat. Nos. 6,056,961; 6,060,068; 6,063,380; and Li et al., Science 288:2219-2222 (2000)).

Notorious among such "weak" vaccines are hepatitis B vaccines. For example, recombinant vaccines against hepatitis B virus such as Genhevacb (Pasteur Merieux Serums et Vaccines, 58, Avenue Leclerc 69007 Lyon, France), Engerixb (Smith, Kline and Symbol French), and Recombivaxhb (Merck, Sharp, and Dhome) are effective only after at least three injections at 0,30, and 60 or 180 days, followed by an obligatory booster after one year. (Chedid et al., U.S. Patent No. 6,063,380). Additionally, many subjects receiving these vaccines respond poorly, if at all. Because many regions of the world are endemic for HBV infection, the poorly immunogenic character of existing HBV vaccines has become an extremely serious problem.

To obtain a stronger, humoral and/or cellular response, it is common to administer a vaccine in a material that enhances the immune response of the patient to the antigen present in the vaccine. The most commonly used adjuvants for vaccine protocols are oil preparations and alum. (Chedid et al., U.S. Patent No. 6,063,380). A greater repertoire of safe and effective adjuvants is needed.

Nucleoside analogs have been widely used in anti-viral therapies due to their capacity to reduce viral replication. (Hosoya et al., J. Inf. Dis., 168:641-646 (1993)). ribavirin (1-β-D-ribofuranosyl-1,2,4-triazole-3-carboxamide) is a synthetic guanosine analog that has been used to inhibit RNA and DNA virus replication. (Huffman et al.; Antimicrob. Agents. Chemother., 3:235 (1973); Sidwell et al., Science, 177:705 (1972)). ribavirin has been shown to be a competitive inhibitor of inositol mono-phosphate (IMP) dehydrogenase (IMPDH), which converts IMP to IMX (which is then converted to GMP). De Clercq, Anti viral Agents: characteristic activity spectrum depending on the molecular target with which they interact, Academic press, Inc., New York N.Y., pp. 1-55 (1993). Intracellular pools of GTP become depleted as a result of long term ribavirin treatment.

In addition to antiviral activity, investigators have observed that some guanosine analogs have an effect on the immune system. (U.S. Patent Nos. 6,063,772 and 4,950,647). ribavirin has been shown to inhibit functional humoral immune responses (Peavy et al., J. Immunol., 126:861-864 (1981); Powers et al., Antimicrob. Agents. Chemother., 22:108-114 (1982)) and IgE-mediated modulation of mast cell secretion. (Marquardt et al., J. Pharmacol. Exp. Therapeutics, 240:145-149 (1987)). Some investigators report that a daily oral therapy of ribavirin has an immune modulating effect on humans and mice. (Hultgren et al., J. Gen. Virol., 79:2381-2391 (1998) and Cramp et al., Gastron. Enterol., 118:346-355 (2000)). The current understanding of the effects of ribavirin on the immune system is in its infancy.

### SUMMARY OF THE INVENTION

The invention is the following:
1. A purified or isolated nucleic acid consisting of the sequence of SEQ. ID. NO.: 16 or the complement thereof;
2. A purified or isolated nucleic acid encoding the peptide sequence of SEQ. ID. NO.: 17;
3. A purified or isolated nucleic acid comprising SEQ ID. NO.: 16;
4. A purified or isolated nucleic acid comprising a nucleic acid encoding the peptide sequence of SEQ. ID. No.: 17;
5. A purified or isolated nucleic acid comprising at least 100,200, 500, or 1000 consecutive nucleotides of the purified or isolated nucleic acid of anyone of 1-4;
6. A vector comprising the purified or isolated nucleic acid of any one of 1-5;
7. An isolated cell comprising the purified or isolated nucleic acid of any one of 1-5;
8. An immunogenic composition comprising the purified or isolated nucleic acid of any one of 1-5;
9. A composition comprising the purified or isolated nucleic acid of any one of 1-5;
10. An isolated cell comprising the vector of 6;
11. An immunogenic composition comprising the vector of 6;
12. A composition comprising the vector of 6;
13. The immunogenic composition of 8 or 11 further comprising an adjuvant.

### BRIEF DESCRIPTION OF THE DRAWINGS

FIGURE 1 is a graph showing the humoral response to 10 and 100µg recombinant Hepatitis C virus (HCV) non structural 3 protein (NS3), as determined by mean end point titres, when a single dose of 1mg of ribavirin was co-administered.
FIGURE 2 is a graph showing the humoral response to 20µg recombinant Hepatitis C virus (HCV) non-structural 3 protein (NS3), as determined by mean end point titres, when a single dose of 0.1, 1.0, or 10mg of ribavirin was co-administered.
FIGURE 3 is a graph showing the effects of a single dose of 1mg ribavirin on NS3-specific lymph node proliferative responses, as determined by *in vitro* recall responses.
FIGURE 4 is a graph showing the antibody titer in H-2^{d} mice against NS3 as a function of time after the first immunization. Diamonds denote antibody titer in mice immunized with NS3/4A-pVAX and squares denote antibody titer in mice immunized with NS3-pVAX.
FIGURE 5A is a graph showing the percentage of specific CTL-mediated lysis of SP2/0 target cells as a function of the effector to target ratio. Phosphate Buffered Saline (PBS) was used as a control immunogen.
FIGURE 5B Is a graph showing the percentage specific CTL-mediated lysis of SP2/0 target cells as a function of the effector to target ratio. Plasmid NS3/4A-pVAX was used as the immunogen.

### DETAILED DESCRIPTION OF THE INVENTION AND OTHER ASPECTS OF THE DISCLOSURE

It has been discovered that compositions comprising ribavirin and an antigen (e.g., a molecule containing an epitope of a pathogen such as a virus, bacteria, mold, yeast, or parasite) enhance and/or facilitate an animal's immune response to the antigen. That is, it was discovered that ribavirin is an effective "adjuvant," which for the purposes of this disclosure, refers to a material that has the ability to enhance or facilitate an immune response to a particular antigen. The adjuvant activity of ribavirin was manifested by a significant increase in immune-mediated protection against the antigen, an increase in the titer of antibody raised to the antigen, and an increase in proliferative T cell responses.

Several compositions (e.g., vaccines) that comprise ribavirin and an antigen or epitope are described herein. Vaccine formulations containing ribavirin, for example, can vary according to the amount of ribavirin, the form of ribavirin, and the type of antigen. The antigen can be a peptide or a nucleic acid (e.g., a RNA encoding a peptide antigen or a construct that expresses a peptide antigen when introduced into a subject): Compositions may comprise ribavirin and a hepatitis viral antigen (e.g., HAV antigen, HBV antigen, HCV antigen, a nucleic acid encoding these molecules, or any combination thereof). At least one HCV antigen or an epitope present on SEQ. ID. NO.: 1 or a nucleic acid encoding said HCV antigen are desired for mixing with ribavirin to make compositions. Compositions comprising ribavirin and a peptide comprising SEQ. ID. NO.: 1, or a fragment thereof having at least 2500, 2000, 1600, 1200, 800, 400, 200, 100, 50, 10, or 3 consecutive amino acids of SEQ. ID. NO.: 1 are disclosed. Compositions comprising ribavirin and a nucleic acid encoding SEQ. ID. NO.: 13 or a fragment thereof having at least 9, 12, 15, 20, 30,50,75,100,200, 500 consecutive nucleotides of SEQ. ID. NO.: 13. are also disclosed.

Compositions (e.g., a vaccine) may comprises ribavirin and a specific fragment of SEQ. ID. NO.: 1, wherein said fragment corresponds to a particular domain of HCV. Fragments of HCV corresponding to amino acids 1-182, 183-379, 380-729, 730-1044, 1045-1657, 1658-1711, 1712-1971, or 1972-3011 of SEQ. ID. NO.: 1, and compositions comprising ribavirin and a nucleic acid encoding one or more of these fragments are disclosed.

A novel HCV sequence has been discovered. A novel nucleic acid and protein corresponding to the NS3/4A domain of HCV was cloned from a patient infected with HCV (SEQ. ID. NO.: 16). A Genebank search revealed that the cloned sequence had the greatest homology to HCV sequences but was only 93% homologous to the closest HCV relative (accession no AJ 278830). This sequence is disclosed in J. Viral Hepat., 7(6), 2000, 459-465. This novel peptide (SEQ. ID. NO.: 17) and fragments thereof at least 3, 4, 6, 8, 10,12, 15 or 20 amino acids in length, nucleic acids encoding these molecules, vectors having said nucleic acids, and cells having said vectors, nucleic acids, or peptides are disclosed. Aspects described herein comprise ribavirin and this novel NS3/4A peptide or a fragment thereof at least 3, 4, 6, 8, 10, 12, 15 or 20 amino acids in length (e.g., SEQ. ID. NO.: 25) or a nucleic acid encoding one or more of these molecules. The present invention is the subject matter of the claims.

Mutants of the novel NS3/4A peptide were also created. It was discovered that truncated mutants (e.g., SEQ. ID. NO.: 29) and mutants, which lack a proteolytic cleavage site, are highly immunogenic. Novel peptides SEQ. ID. NOs.: 29- 32 and 43-49 and fragments thereof at least 3, 4, 6, 8, 10, 12, 15 or 20 amino acids in length (e.g., SEQ. ID. NOs.: 26, 27, and 33-42), nucleic acids encoding these molecules, vectors having said nucleic acids, and cells having said vectors, nucleic acids, or peptides are disclosed. Some of the compositions described herein comprise ribavirin and at least one of the mutant HCV peptides described above or a fragment thereof at least 3, 4, 6, 8, 10, 12, 15 or 20 amino acids in length. Vaccines comprising ribavirin and a nucleic acid (e.g., DNA) encoding one or more of the peptides described above are disclosed.

Methods of making and using the compositions above are disclosed. For example, the compositions described above can be made by providing ribavirin, providing an antigen (e.g., a peptide comprising an HCV antigen or a nucleic acid encoding said peptide), and mixing said ribavirin and said antigen so as to formulate a composition that can be used to enhance or facilitate an immune response in a subject to said antigen. Methods entail mixing a preferred antigen or epitope (e.g., a peptide comprising SEQ. ID. NO.: 1, 6, 7, or 17 or specific fragments thereof, such as amino acids 1-182, 183-379, 380-729, 730-1044, 1045-1657, 1658-1711, 1712-1971, 1972-3011 of SEQ. ID. NO.: 1 and nucleic acids encoding these molecules) with ribavirin. Other antigens or epitopes can also be mixed with ribavirin including, but not limited to, fragments of SEQ. ID. NO.: 1 that have at least 2500, 2000, 1600,1200, 800, 400, 200, 100,50,10, or 3 consecutive amino acids and nucleic acids encoding these fragments. Particularly preferred methods concern the making of vaccine compositions comprising the newly discovered NS3/4A fragment or an NS3/4A mutant (e.g., a truncated mutant or a mutant lacking a proteolytic cleavage site), or a fragment thereof of at least four amino acids in length or a nucleic acid encoding one or more of these molecules.

Methods of enhancing or facilitating the immune response of an animal, including humans, to an antigen are disclosed. Such methods can be practiced, for example, by identifying an animal in need of a potent immune response to an antigen/epitope and providing said animal a composition comprising the antigen/epitope and an amount of ribavirin that is effective to enhance or facilitate an immune response to the antigen/epitope. The ribavirin and the antigen may be administered separately, instead of in a single mixture. Preferably, in this instance, the ribavirin is administered a short time before or a short time after administering the antigen. Methods may involve providing the animal in need with ribavirin and a hepatitis antigen (e.g., HAV antigen, HBV antigen, HCV antigen, a nucleic acid encoding these molecules, or any combination thereof). Some of these methods involve HBV antigens, such as a peptide comprising SEQ. ID. NO.: 1, or a fragment thereof having at least 2500, 2000, 1600, 1200, 800, 400, 200, 100, 50, 10, or 3 consecutive amino acids of SEQ. ID.

NO.: 1. Additional methods involve compositions comprising ribavirin and a nucleic acid encoding SEQ. ID. NO.: 13 or a nucleic acid encoding one or more of the fragments discussed above.

Methods may concern the use of a composition (e.g., a vaccine) that comprises ribavirin and a peptide comprising SEQ. ID. NO.: 1 or a specific fragment thereof, which corresponds to an HCV domain including, but not limited to, a peptide comprising amino acids 1-182, 183-379, 380-729, 730-1044, 1045-1657, 1658-1711, 1712-1971, or 1977-3011 of SEQ. ID. NO.: 1. Methods may concern the use of a vaccine composition comprising the NS3/4A fragment of SEQ. ID. NO.: 17 or the mutant NS3/4A (e.g., SEQ. ID. NOs:. 29- 32 and 43-49), which lack a proteolytic cleavage site, or a fragment thereof of at least 3, 4, 6, 8, 10, 12, 15 or 20 amino acids in length (e.g., SEQ. ID. NOs.: 26, 27, and 33-42). Compositions comprising ribavirin and a nucleic acid encoding these fragments can also be used with the methods described herein.

Methods of treating and preventing HCV infection are disclosed. By one approach, ribavirin and an HCV antigen or epitope are used to prepare a medicament for the treatment and/or prevention of HCV infection. By another approach, an individual in need of a medicament that prevents and/or treats HCV infection is identified and said individual is provided a medicament comprising ribavirin and an HCV antigen or epitope, preferably an epitope present on SEQ. ID. NO.: 1, more preferably a fragment of SEQ. ID. NO.: 1 having at least 2500, 2000, 1600, 1200, 800, 400, 200, 100, 50, 10, or 3 consecutive amino acids or most preferably a fragment of SEQ. ID. NO.: 1 such as 1-182, 183-379, 380-729, 730-1044, 1045-1657, 1658-1711, 1712-1971, or 1972-3011 or a nucleic acid encoding SEQ. ID. NO.: 1 or said fragments above. Methods may concern the use of a vaccine composition comprising ribavirin and the NS3/4A fragment of SEQ. ID. NO.: 17 or the mutant NS3/4A, which lacks a proteolytic cleavage site (e.g., SEQ. ID. NOs.: 29- 32 and 43-49) or a fragment thereof of at least 3, 4, 6, 8, 10,12, 15 or 20 amino acids in length (e.g., SEQ. ID. NOs.: 25-27, and 33-42) or a nucleic acid encoding one or more of these molecules. The section below discusses the use of ribavirin as an adjuvant in greater detail.

### Ribavirin

Compositions described herein can be manufactured in accordance with conventional methods of galenic pharmacy to produce medicinal agents for administration to animals, e.g., mammals including humans. Ribavirin can be obtained from commercial suppliers (e.g., Sigma and ICN). Ribavirin and/or the antigen can be formulated into the vaccine with and without modification. For example, the ribavirin and/or antigen can be modified or derivatized to make a more stable molecule and/or a more potent adjuvant. By one approach, the stability of ribavirin and/or an antigen can be enhanced by coupling the molecules to a support such as a hydrophilic polymer (e.g., polyethylene glycol).

Many more ribavirin derivatives can be generated using conventional techniques in rational drug design and combinatorial chemistry. For example, Molecular Simulations Inc. (MSI), as well as many other suppliers, provide software that allows one of skill to build a combinatorial library of organic molecules. The C2 Analog Builder program, for example, can be integrated with MSrs suite of Cerius2 molecular diversity software to develop a library of ribavirin derivatives that can be used with the embodiments described herein. (See e.g., http://msi.com/life/products/cerius2/index.html).

By one approach, the chemical structure of ribavirin is recorded on a computer readable medium and is accessed by one or more modeling software application programs. The C2.Analog Builder program in conjunction with C2Diversity program allows the user to generate a very large virtual library based on the diversity of R-groups for each substituent positions, for example. Compounds having the same structure as the modeled ribavirin derivatives created in the virtual library are then made using conventional chemistry or can be obtained from a commercial, source.

The newly manufactured ribavirin derivatives are then screened in assays, which determine the extent of adjuvant activity of the molecule and/or the extent of its ability to modulate of an immune response. Some assays may involve virtual drug screening software, such as C2.Ludi. C2.Ludi is a software program that allows a user to explore databases of molecules (e.g., ribavirin derivatives) for their ability to interact with the active site of a protein of interest (e.g., RAC2 or another GTP binding protein). Based upon predicted interactions discovered with the virtual drug screening software, the ribavirin derivatives can be prioritized for further characterization in conventional assays that determine adjuvant activity and/or the extent of a molecule to modulate an immune response. In so far as the following sections marked "examples" pertain to the subject matter of the claims, in particular the subject matter of independent claims 1-5, such are illustrative of the invention. All other aspects referred to are provided for information purposes only.

*Example 1* describes several assays that were used to evaluate the adjuvant activity of ribavirin.

### EXAMPLE 1

This following assays can be used with any ribavirin derivative or combinations of ribavirin derivatives to determine the extent of adjuvant activity of the particular composition. In a first set of experiments, groups of three to five Balb/c mice (BK Universal, Uppsala; Sweden) were immunized *i.p* or *s.c.* (e.g., at the base of the tail) with 10µg or 100µg of recombinant hepatitis C virus non-structural 3 (rNS3) protein at weeks zero and four. The rNS3 was dissolved in phosphate buffered saline (PBS) alone or PBS containing 1mg ribavirin (obtained from ICN, Costa Mesa, CA). Mice were injected with a total volume of 100µl per injection.

At two, four, and six weeks following first *i*.*p*. immunization, all mice were bled by retro-orbital sampling. Serum samples were collected and analyzed for the presence of antibodies to rNS3. To determine the antibody titer, an enzyme immunoassay (EIA) was performed. (See e.g., Hultgren et al., J Gen Virol. 79:2381-91 (1998) and Hultgren et al., Clin. Diagn. Lab. Immunol. 4:630-632 (1997)). The antibody levels were recorded as the highest serum dilution giving an optical density at 405nm more than twice that of non-immunized mice.

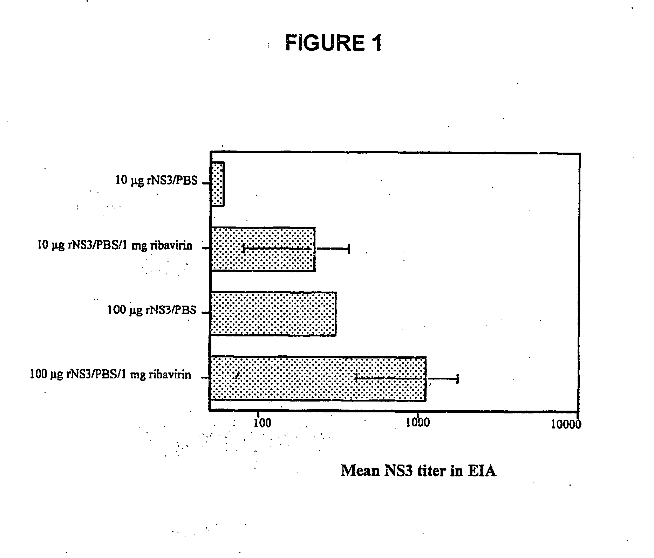

Mice that received 10µg or, 100µg rNS3 mixed with 1mg ribavirin in PBS displayed consistently higher levels of NS3 antibodies. The antibody titer that was detected by EIA at two weeks post-immunization is shown in **FIGURE 1****.** The vaccine formulations having 1mg of ribavirin and either 10µg or 100µg of rNS3 induced a significantly greater antibody titer than the vaccine formulations composed of only rNS3.

In a second set of experiments, groups of eight Balb/c mice were at weeks zero and four immunized intraperitoneally with 10 or 50 µg of rNS3 in 100 µL phosphate buffered saline containing either 0 mg, 1 mg, 3 mg, or 10 mg ribavirin (Sigma). At four, six and eight weeks the mice were bled and serum was separated and frozen. After completion of the study, sera were tested for the levels of antibodies to recombinant NS3, as described above. Mean antibody levels to rNS3 were compared between the groups using Student's t-test (parametric analysis) or Mann-Whitney (non-parametric analysis) and the software package StatView 4.5 (Abacus Concepts, Berkely, CA). The adjuvant effect of ribavirin when added in three doses to 10 µg of rNS3 are provided in **TABLE 1.** The adjuvant effect of ribavirin when added in three doses to 50 µg of rNS3 are provided in **TABLE 2.** Parametrical comparison of the mean rNS3 antibody titres in mice receiving different 10µg or 50 µg of rNS3 and different doses of ribavirin are provided in **TABLES 3** and 4, respectively. Non-parametrical comparison of mean NS3 antibody titres in mice receiving different 10µg or 50 µg of rNS3 and different doses of ribavirin are provided in **TABLES 5** and **6,** respectively. The values given represent end point titres to recombinant rNS3.

**TABLE 1**

| Amount ribavirin (mg/dose) | Amount immunogen (µg/dose) | Mouse ID | Antibody titre to rNS3 at indicated week | | |
|---|---|---|---|---|---|
| | | | Week 4 | Week 6 | Week 8 |
| None | 10 | 5:1 | 300 | 1500 | 1500 |
| None | 10 | 5:2 | <60 | 7500 | 1500 |
| None | 10 | 5:3 | <60 | 1500 | 300 |
| None | 10 | 5:4 | 60 | 1500 | 1500 |
| None | 10 | 5:5 | <60 | 1500 | nt |
| None | 10 | 5:6 | 60 | 1500 | 1500 |
| None | 10 | 5:7 | <60 | 7500 | 7500 |
| None | 10 | 5:8 | 300 | 37500 | 7500 |
| Group mean titre (mean±SD) | | | 180 ±139 | 7500 ±12421 | 3042 ±3076 |
| 1 | 10 | 6:1 | 300 | 37500 | 37500 |
| 1 | 10 | 6:2 | <60 | 1500 | 1500 |
| 1 | 10 | 6:3 | 300 | 37500 | 187500 |
| 1 | 10 | 6:4 | 300 | 37500 | 7500 |
| 1 | 10 | 6:5 | 60 | nt | nt |
| 1 | 10 | 6:6 | <60 | 37500 | 7500 |
| 1 | 10 | 6:7 | <60 | 37500 | 7500 |
| 1 | 10 | 6:8 | 300 | 7500 | 7500 |
| Group mean titre (mean±SD) | | | 252 ±107 | 28071 ±16195 | 36642 ±67565 |
| 3 | 10 | 7:1 | 60 | 37500 | 7500 |
| 3 | 10 | 7:2 | 60 | 37500 | 37500 |
| 3 | 10 | 7:3 | 300 | 7500 | 7500 |
| 3 | 10 | 7:4 | 300 | 37500 | 7500 |
| 3 | 10 | 7:5 | 300 | 37500 | 37500 |
| 3 | 10 | 7:6 | 300 | 37500 | 37500 |
| 3 | 10 | 7:7 | 60 | 7500 | 7500 |
| 3 | 10 | 7:8 | 60 | 37500 | 37500 |
| Group mean titre (mean±SD) | | | 180± 128 | 30000± 13887 | 22500± 34637 |
| 10 | 10 | 8:1 | 300 | 37500 | 37500 |
| 10 | 10 | 8:2 | 300 | 37500 | 37500 |
| 10 | 10 | 8:3 | <60 | 300 | 300 |
| 10 | 10 | 8:4 | 60 | 7500 | 7500 |
| 10 | 10 | 8:5 | <60 | 300 | 300 |
| 10 | 10 | 8:6 | <60 | 37500 | 37500 |
| 10 | 10 | 8:7 | <60 | 7500 | 7500 |
| 10 | 10 | 8:8 | <60 | nt | nt |
| Group mean titre (mean±SD) | | | 220± 139 | 18300± 18199 | 18300± 18199 |

**TABLE 2**

| Amount ribavirin (mg/dose) | Amount immunogen (µg/dose) | Mouse ID | Antibody titre to rNS3 at indicated week | | |
|---|---|---|---|---|---|
| | | | Week 4 | Week 6 | Week 8 |
| None | 50 | 1:1 | 60 | 7500 | 7500 |
| None | 50 | 1:2 | 60 | 7500 | 7500 |
| None | 50 | 1:3 | 60 | 7500 | 7500 |
| None | 50 | 1:4 | <60 | 1500 | 300 |
| None | 50 | 1:5 | 300 | 37500 | 37500 |
| None | 50 | 1:6 | 60 | 7500 | 7500 |
| None | 50 | 1:7 | 60 | 37500 | 7500 |
| None | 50 | 1:8 | . | . | . |
| Group mean titre (mean±SD) | | | 100 ±98 | 15214 ±15380 | 10757 ±12094 |
| 1 | 50 | 2:1 | 60 | 7500 | 7500 |
| 1 | 50 | 2:2 | 300 | 37500 | 7500 |
| 1 | 50 | 2:3 | 60 | 187500 | 7500 |
| 1 | 50 | 2:4 | 60 | 37500 | 187500 |
| 1 | 50 | 2:5 | 60 | 37500 | 7500 |
| 1 | 50 | 2:6 | 60 | 37500 | 37500 |
| 1 | 50 | 2:7 | 300 | 37500 | 7500 |
| 1 | 50 | 2:8 | 300 | 37500 | 37500 |
| Group mean titre (mean±SD) | | | 150 ±124 | 52500 ±55549 | 37500 ±62105 |
| 3 | 50 | 3:1 | 60 | 37500 | 7500 |
| 3 | 50 | 3:2 | 300 | 37500 | 37500 |
| 3 | 50 | 3:3 | 300 . | 37500 | 7500 |
| 3 | 50 | 3:4 | 60 | 37500 | 7500 |
| 3 | 50 | 3:5 | 300 | 37500 | 7500 |
| 3 | 50 | 3:6 | 60 | 37500 | 7500 |
| 3 | 50 | 3:7 | - | 7500 | 37500 |
| 3 | 50 | 3:8 | 1500 | 7500 | 37500 |
| Group mean titre (mean±SD) | | | 387 ±513 | 30000' ±13887 | 18750 ±15526 |
| 10 | 50 | 4:1 | 300 | 7500 | 7500 |
| 10 | 50 | 4:2 | 300 | 37500 | 37500 |
| 10 | 50 | 4:3 | 60 | 7500 | 7500 |
| 10 | 50 | 4:4 | 60 | 7500 | 7500 |
| 10 | 50 | 4:5 | 60 | 1500 | 1500 |
| 10 | 50 | 4:6 | 60 | 7500 | 37500 |
| 10 | 50 | 4:7 | - | 7500 | 7500 |
| 10 | 50 | 8:8 | 60 | 37500 | 7500 |
| Group mean titre (mean±SD) | | | 140 ±124 | 10929 ±11928 | 15214 ±15380 |

**TABLE 3**

| Group | Week | Mean±SD | Group | Mean±SD | analysis | p-value |
|---|---|---|---|---|---|---|
| 10µgNS3/no ribavirin | 4 | 180 ±139 | 10µg NS3/ 1 mg ribavirin | 252 ±107 | Students t-test | 0.4071 |
| | 6 | 7500 ±12421 | | 28071 ±16195 | Students t-test | 0.0156 |
| | 8 | 3042 ±3076 | | 36642 ±67565 | Students t-test | 0.2133 |
| 10µg NS3/no ribavirin | 4 | 180 ±139 | 10 µg NS3/ 3 mg ribavirin | 180± 128 | Students t-test | 1.000 |
| | 6 | 7500 ±12421 | | 30000± 13887 | Students t-test | 0.0042 |
| | 8 | 3042 ±3076 | | 22500± 34637 | Students t-test | 0.0077 |
| 10µg NS3/no ribavirin | 4 | 180 ±139 | 10µgNS3/ 10mg ribavirin | 220± 139 | Students t-test | 0.7210 |
| | 6 | 7500 ±12421 | | 18300± 18199 | Students t-test | 0.1974 |
| | 8 | 3042 ±3070 | | 18300± 18199 | Students t-test | 0.0493 |

**TABLE4**

| Group | Week | Mean±SD | Group | Mean±SD | analysis | p-value |
|---|---|---|---|---|---|---|
| 50µgNS3/no ribavirin | 4 | 100 ±98 | 50µgNS3/ 1 mg ribavirin | 150 ±124 | Students t-test | 0.4326 |
| | 6 | 15214 ±15380 | | 52500 ±55549 | Students t-test | 0.1106 |
| | 8 | 10757 ±12094 | | 37500 ±62105 | Students t-test | 0.2847 |
| 50µg NS3/no ribavirin | 4 | 100 ±98 | 50 µgNS3/ 3 mg ribavirin | 387 ±513 | Students t-test | 0.2355 |
| | 6 | 15214 ±15380 | | 30000 ±13887 | Students t-test | 0.0721 |
| | 8 | 10757 ±12094 | | 18750 ±15526 | Students t-test | 0.2915 |
| 50µg NS3/no ribavirin | 4 | 100 ±98 | 50 µg NS3/ 10mg ribavirin | 140 ±124 | Students t-test | 0.5490 |
| | 6 | 15214 ±15380 | | 10929 ±11928 | Students t-test | 0.5710 |
| | 8 | 10757 ±12094 | | 15214 ±15380 | Students t-test | 0.5579 |

| | | | | | | |
|---|---|---|---|---|---|---|
| Significance levels: NS = not significant; * = p<0.05; ** = p<0.01; *** = p<0.001 | | | | | | |

**TABLE 5**

| Group | Week | Mean±SD | Group | Mean±SD | analysis | p-value |
|---|---|---|---|---|---|---|
| 10µg NS3/no ribavirin | 4 | 180 ±139 | 10µg NS3/ 1 mg ribavirin | 252 ±107 | Mann-Whitney | 0.4280 |
| | 6 | 7500 ±12421 | | 28071 ±16195 | Mann-Whitney | 0.0253 |
| | 8 | 3042 ±3076 | | 36642 ±67565 | Mann-Whitney | 0.0245 |
| 10µg NS3/no ribavirin | 4 | 180 ±139 | 10 µg NS3/ 3 mg ribavirin | 180± 128 | Mann-Whitney | 0.0736 |
| | 6 | 7500 ±12421 | | 30000± 13887 | Mann-Whitney | 0.0050 |
| | 8 | 3042 ±3076 | | 22500± 34637 | Mann-Whitney | 0.0034 |
| 10µg NS3/no ribavirin | 4 | 180 ±139 | 10 µg NS3/ 10mg ribavirin | 220± 139 | Mann-Whitney | 0.8986 |
| | 6 | 7500 ±12421 | | 18300± 18199 | Mann-Whitney | 0.4346 |
| | 8 | 3042 ±3076 | | 18300± 18199 | Mann-Whitney | 0.2102 |

**TABLE 6**

| Group | Week | Mean±SD | Group | Mean±SD | analysis | p-value |
|---|---|---|---|---|---|---|
| 50µg NS3/no ribavirin | 4 | 100 ±98 | 50 µg NS3/ 1 mg ribavirin | 150 ±124 | Mann-Whitney | 0.1128 |
| | 6 | 15214 ±15380 | | 52500 ±55549 | Mann-Whitney | 0.0210 |
| | 8 | 10757 ±12094 | | 37500 ±62105 | Mann-Whitney | 0.1883 |
| 50µg NS3/no ribavirin | 4 | 100 ±98 | 50 µg NS3/ 3 mg ribavirin | 387 ±513 | Mann-Whitney | 0.1400 |
| | 6 | 15214 ±15380 | | 30000 ±13887 | Mann-Whitney | 0.0679 |
| | 8 | 10757 ±12094 | | 18750 ±15526 | Mann-Whitney | 0.2091 |
| 50µg NS3/no ribavirin | 4 | 100 ±98 | 50 µg NS3/ 10 mg ribavirin | 140 ±124 | Mann-Whitney | 0.4292 |
| | 6 | 15214 ±15380 | | 10929 ±11928 | Mann-Whitney | 0.9473 |
| | 8 | 10757 ±12094 | | 15214 ±15380 | Mann-Whitney | '0.6279 |

| | | | | | | |
|---|---|---|---|---|---|---|
| Significance levels: NS = not significant; * = p<0.05; ** = p<0.01; *** = p<0.001 | | | | | | |

The data above demonstrate that ribavirin facilitates or enhances an immune response to an HCV antigen or HCV epitopes. A potent immune response to rNS3 was elicited after immunization with a vaccine composition comprising as little as 1 mg ribavirin and 10 µg of rNS3 antigen. The data above also provide evidence that the amount of ribavirin that is sufficient to facilitate an immune response to an antigen is between 1 and 3 mg per injection for a 25-30g Balb/c mouse. It should be realized, however, that these amounts are intended for guidance only an should not be interpreted to limit the scope of the invention in any way. Nevertheless, the data shows that vaccine compositions comprising approximately 1 to 3 mg doses of ribavirin induce an immune response that is more than 12 times higher than the immune response elicited in the absence of ribavirin **(TABLES 3** and 4). Thus, ribavirin has a significant adjuvant effect on the humoral immune response of an animal and thereby, enhances or facilitates the immune response to the antigen. The example below describes experiments that were performed to better understand the amount of ribavirin needed to enhance or facilitate an immune response to an antigen.

### EXAMPLE 2

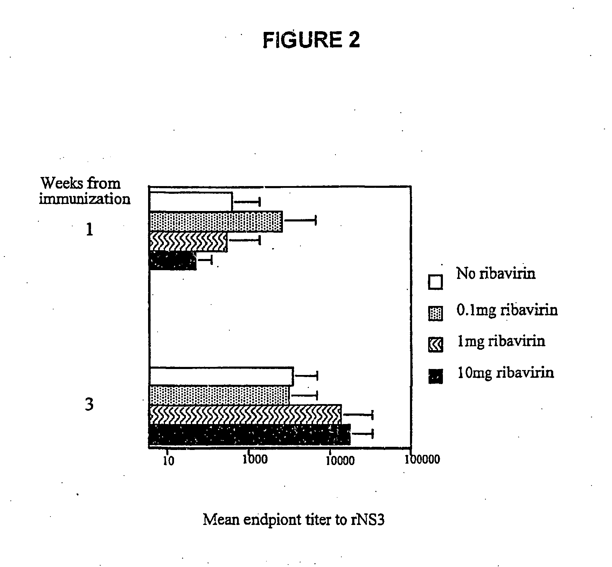

To determine a dose of ribavirin that is sufficient to provide an adjuvant effect, the following experiments were performed. In a first set of experiments, groups of mice (three per group) were immunized with a 20µg rNS3 alone or a mixture of 20µg rNS3 and 0.1mg, 1mg, or 10mg ribavirin. The levels of antibody to the antigen were then determined by EIA. The mean endpoint titers at weeks 1 and 3 were plotted and are shown in **FIGURE 2****.** It was discovered that the adjuvant effect provided by ribavirin had different kinetics depending on the dose of ribavirin provided. For example, even low doses (<1mg) of ribavirin were found to enhance antibody levels at week one but not at week three, whereas, higher doses (1-10mg) were found to enhance antibody levels at week three.

A second set of experiments was also performed. In these experiments, groups of mice were injected with vaccine compositions comprising various amounts of ribavirin and rNS3 and the IgG response in these animals was monitored. The vaccine compositions comprised approximately 100 µl phosphate buffered saline and 20 µg rNS3 with or without 0.1 mg, 1.0 mg, or 10 mg ribavirin (Sigma). The mice were bled at week six and rNS3-specific IgG levels were determined by EIA as described previously. As shown in **TABLE 7,** the adjuvant effects on the sustained antibody levels were most obvious in the dose range of 1 to 10 mg per injection for a 25-30g mouse.

**TABLE 7**

| Immunogen | Amount (mg) ribavirin mixed with the immunogen | Mouse ID | Endpoint titre of rNS3 IgG at indicated week | | |
|---|---|---|---|---|---|
| | | | Week 1 | Week 2 | Week 3 |
| 20 µg rNS3 | None | 1 | 60 | 360 | 360 |
| 20 µg rNS3 | None | 2 | 360 | 360 | 2160 |
| 20 pg rNS3 | None | 3 | 360 | 2160 | 2160 |
| | | Mean | 260±173 | 960±1039 | 1560±1039 |
| 20 µg rNS3 | 0.1 | 4 | 2160 | 12960 | 2160 |
| 20 µg rNS3 | 0.1 | 5 | 60 | 60 | 60 |
| 20 µg rNS3 | 0.1 | 6 | <60 | 2160 | 2160 |
| | | | 1110±1484 | 5060±6921 | 1460±1212 |
| 20 µg rNS3 | 1.0 | 7 | <60 | 60 | 12960 |
| 20 µg rNS3 | 1.0 | 8 | <60 | 2160 | 2160 |
| 20 µg rNS3 | 1.0 | 9 | 360 | 2160 | 2160 |
| | | Mean | 360 | 1460±1212 | 5760±6235 |
| 20 µg rNS3 | 10.0 | 10 | 360 | 12960 | 77760 |
| 20 µg rNS3 | 10.0 | 11 | <60 | 2160 | 12960 |
| 20 µg rNS3 | 10.0 | 12 | 360 | 2160 | 2160 |
| | | Mean | 360 | 5760±6235 | 30960±40888 |

In a third set of experiments, the adjuvant effect of ribavirin after primary and booster injections was investigated. In these experiments, mice were given two intraperitoneal injections of a vaccine composition comprising 10 µg rNS3 with or without ribavirin and the IgG subclass responses to the antigen was monitored, as before. Accordingly, mice were immunized with 100 µl phosphate buffered containing 10 µg recombinant NS3 alone, with or without 0.1 or 1.0 mg ribavirin (Sigma) at weeks 0 and 4. The mice were bled at week six and NS3-specific IgG subclasses were determined by EIA as described previously. As shown in **TABLE 8,** the addition of ribavirin to the immunogen prior to the injection does not change the IgG subclass response in the NS3-specific immune response. Thus, the adjuvant effect of a vaccine composition comprising ribavirin and an antigen can not be explained by a shift in the Th1/Th2-balance. It appears that another mechanism may be responsible for the adjuvant effect of ribavirin.

**TABLE 8**

| Immunogen | Amount (mg) ribavirin mixed with the immunogen | Mouse ID | Endpoint titre of indicated NS3 IgG subclass | | | |
|---|---|---|---|---|---|---|
| | | | IgG1 | IgG2a | IgG2b | IgG3 |
| 10 µg rNS3 | None | 1 | 360 | 60 | <60 | 60 |
| 10 µg rNS3 | None | 2 | 360 | <60 | <60 | 60 |
| 10 µg rNS3 | None | 3 | 2160 | 60 | <60 | 360 |
| | | Mean | 960±1039 | 60 | - | 160±173 |
| 10 µg rNS3 | 0.1 | 4 | 360 | <60 | <60 | 60 |
| 10 µg rNS3 | 0.1 | 5 | 60 | <60 | <60 | <60 |
| 10 µg rNS3 | 0.1 | 6 | 2160 | 60 | 60 | 360 |
| | | | 860±1136 | 60 | 60 | 210±212 |
| 10 µg rNS3 | 1.0 | 7 | 2160 | <60 | <60 | 60 |
| 10 µg rNS3 | 1.0 | 8 | 360 | <60 | <60 | <60 |
| 10 µg rNS3 | 1.0 | 9 | 2160 | <60 | <60 | 60 |
| | | Mean | 1560±1039 | - | - | 60 |

The data presented in this example further verify that ribavirin can be administered as an adjuvant and establish that that the dose of ribavirin can modulate the kinetics of the adjuvant effect. The example below describes another assay that was performed to evaluate the ability of ribavirin to enhance or facilitate an immune response to an antigen.

### EXAMPLE 3

This assay can be used with any ribavirin derivative or combinations of ribavirin derivatives to determine the extent that a particular vaccine formulation modulates a cellular immune response. To determine CD4⁺ T cell responses to a ribavirin-containing vaccine, groups of mice were immunized *s.c.* with either 100µg rNS3 in PBS or 100µg rNS3 and 1mg ribavirin in PBS. The mice were sacrificed ten days post-immunization and their lymph nodes were harvested and drained. *In vitro* recall assays were then performed. (See e.g., Hultgren et al., J Gen Virol. 79:2381-91 (1998) and Hultgren et al., Clin, Diagn. Lab. Immunol. 4:630-632 (1997)). The amount of CD4⁺ T cell proliferation was determined at 96 h of culture by the incorporation of [³H] thymidine.

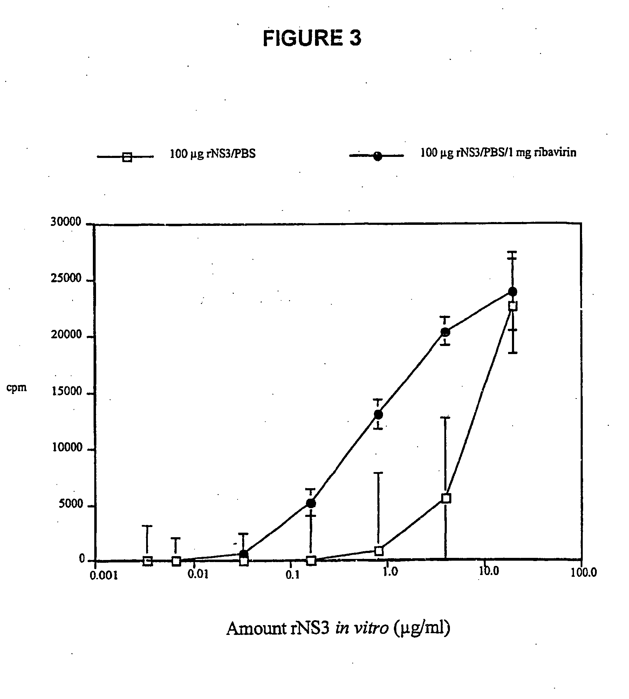

As shown in **FIGURE 3****,** mice that were immunized with 100µg rNS3 mixed with 1mg ribavirin had a much greater T cell proliferative response than mice that were immunized with 100µg rNS3 in PBS. These data provide additional evidence that ribavirin enhances or facilitates a cellular immune response (e.g., by promoting the effective priming of T cells). The section below discusses some of the antigens and epitopes that can be used with the embodiments described herein.

### Antigens and epitopes

Virtually any antigen that can be used to generate an immune response in an animal can be combined with ribavirin so as to prepare the compositions described herein. That is, antigens that can be incorporated into such compositions (e.g., vaccines) comprise bacterial antigens or epitopes, fungal antigens or epitopes, plant antigens or epitopes, mold antigens or epitopes, viral antigens or epitopes, cancer cell antigens or epitopes, toxin antigens or epitopes, chemical antigens or epitopes, and self-antigens or epitopes. Although many of these molecules induce a significant immune response without an adjuvant, ribavirin can be administered in conjunction with or combined with "strong" or "weak" antigens or epitopes to enhance or facilitate the immune response to said antigen or epitope. In addition, the use of ribavirin as an adjuvant may allow for the use of lesser amounts of antigens while retaining immunogenicity.

In addition to peptide antigens, nucleic acid-based antigens can be used in the vaccine compositions described herein. Various nucleic acid-based vaccines are known and it is contemplated that these compositions and approaches to immunotherapy can be augmented by reformulation with ribavirin (See e.g., U.S. Pat. No. 5,589,466 and 6,235,888). By one approach, for example, a gene encoding a polypeptide antigen of interest is cloned into an expression vector capable of expressing the polypeptide when introduced into a subject. The expression construct is introduced into the subject in a mixture of ribavirin or in conjunction with ribavirin (e.g., ribavirin is administered shortly after the expression construct at the same site). Alternatively, RNA encoding a polypeptide antigen of interest is provided to the subject in a mixture with ribavirin or in conjunction with ribavirin.

Where the antigen is to be DNA (e.g., preparation of a DNA vaccine composition), suitable promoters include Simian Virus 40 (SV40), Mouse Mammary Tumor Virus (MMTV) promoter, Human immunodeficiency Virus (HIV) such as the HIV Long Terminal Repeat (LTR) promoter, Moloney virus, ALV, Cytomegalovirus (CMV) such as the CMV immediate early promoter, Epstein Barr Virus (EBV), Rous Sarcoma Virus (RSV) as well as promoters from human genes such as human actin, human myosin, human hemoglobin, human muscle creatine and human metalothionein can be used. Examples of polyadenylation signals useful with some embodiments, especially in the production of a genetic vaccine for humans, include but are not limited to, SV40 polyadenylation signals and LTR polyadenylation signals. In particular, the SV40 polyadenylation signal, which is in pCEP4 plasmid (Invitrogen, San Diego Calif.), referred to as the SV40 polyadenylation signal, is used.

In addition to the regulatory elements required for gene expression, other elements may also be included in a gene construct. Such additional elements include enhancers. The enhancer may be selected from the group including but not limited to: human actin, human myosin, human hemoglobin, human muscle creatine and viral enhancers such as those from CMV, RSV and EBV. Gene constructs can be provided with mammalian origin of replication in order to maintain the construct extrachromosomally and produce multiple copies of the construct in the cell. Plasmids pCEP4 and pREP4 from Invitrogen (San Diego, CA) contain the Epstein Barr virus origin of replication and nuclear antigen EBNA-1 coding region, which produces high copy episomal replication without integration. All forms of DNA, whether replicating or non-replicating, which do not become integrated into the genome, and which are expressible, can be used. The example below describes the use of a composition comprising a nucleic acid-based antigen and ribavirin.

### EXAMPLE 4

The following describes the immunization of an animal with a vaccine comprising a nucleic acid-based antigen and ribavirin. Five to six week old female and male Balb/C mice are anesthetized by intraperitoneal injection with 0.3ml of 2.5% Avertin. A 1.5cm incision is made on the anterior thigh, and the quadriceps muscle is directly visualized. One group of mice are injected with approximately 20:g of an expression construct having the gp-120 gene, driven by a cytomegalovirus (CMV) promotor and second group of mice are injected with approximately 5:g of capped *in vitro* transcribed RNA (e.g., SP6, T7, or T3 (Ambion)) encoding gp-120. These two groups are controls. A third group of mice is injected with approximately 20:g of the expression vector having the gp-120 gene and the CMV promoter mixed with 1mg of ribavirin and a fourth group of mice is injected with approximately 5:g of capped *in vitro* transcribed RNA mixed with . 1mg ribavirin. The vaccines are injected in 0.1ml of solution (PBS) in a 1 cc syringe through a 27 gauge needle over one minute, approximately 0.5cm from the distal insertion site of the muscle into the knee and about 0.2cm deep. A suture is placed over the injection site for future localization, and the skin is then closed with stainless steel clips.

Blood samples are obtained prior to the injection (Day 0) and up to more than 40 days post injection. The serum from each sample is serially diluted and assayed in a standard ELISA technique assay for the detection of antibody, using recombinant gp-120 protein made in yeast as the antigen. Both IgG and IgM antibodies specific for gp-120 will be detected in all samples, however, groups three and four, which contained the ribavirin, will exhibit a greater immune response to the gp-120 as measured by the amount and/or titer of antibody detected in the sera.

The disclosure made comprises ribavirin and a viral antigen or an epitope present on a virus, preferably a hepatitis virus. Compositions comprise, for example, ribavirin and an HAV antigen, HBV antigen, HCV antigen or any combination of these antigens or epitopes present on one or more of these viruses. The hepatitis antigens can be peptides or nucleic acids. Compositions that can be used to vaccinate against HAV infection, for example, comprise ribavirin and an HAV peptide with a length of at least 3-10 consecutive amino acids, 10-50 consecutive amino acids, 50-100 consecutive amino acids, 100-200 consecutive amino acids, 200-400 consecutive amino acids, 400-800 consecutive amino acids, 800-1200 consecutive amino acids, 1200-1600 consecutive amino acids, 1600-2000 consecutive amino acids, and 2000-2227 consecutive amino acids of **SEQ ID. NO.: 12.**

Additionally, compositions comprising ribavirin and a nucleic acid encoding one or more of the HAV peptides, described above, can be used to treat or prevent HAV infection. Preferred nucleic acid-based antigens include a nucleotide sequence of at least 9. consecutive nucleotides of an HAV sequence (e.g., **SEQ. ID. NO.: 15).** That is, a nucleic acid based antigen can comprise at least 9-25 consecutive nucleotides, 25-50 consecutive nucleotides, 50-100 consecutive nucleotides, 100-200 consecutive nucleotides, 200-500 consecutive nucleotides, 500-1000 consecutive nucleotides, 1000-2000 consecutive nucleotides, 2000-4000 consecutive nucleotides, 4000-8000 consecutive nucleotides, and 8000-9416 consecutive nucleotides of **SEQ. ID. NO.: 15** or an RNA that corresponds to these sequences.

Similarly, preferred HBV vaccine embodiments comprise ribavirin and a HBV peptide of at least 3 consecutive amino acids of HBsAg **(SEQ. ID. NO.: 10)** or HBcAg and HBeAg **(SEQ. ID. NO.:** 11). That is, some embodiments have ribavirin and a HBV peptide with a length of at least 3-10 consecutive amino acids, 10-50 consecutive amino acids, 50-100 consecutive amino acids, 100-150 consecutive amino acids, 150-200 consecutive amino acids, and 200-226 consecutive amino acids of either **SEQ. ID. NO.: 10** or **SEQ. ID. NO.: 11.**

Additionally, compositions comprising ribavirin and a nucleic acid encoding one or more of the HBV peptides, described above, can be used to treat or prevent HBV infection. Nucleic acid-based antigens include a nucleotide sequence of at least 9 consecutive nucleotides of an HBV (e.g., **SEQ. ID. NO.:14).** That is, a nucleic acid based antigen can comprise at least 9-25 consecutive nucleotides, 25-50 consecutive nucleotides, 50-100 consecutive nucleotides, 100-200 consecutive nucleotides, 200-500 consecutive nucleotides, 500-1000 consecutive nucleotides, 1000-2000 consecutive nucleotides, 2000-4000 consecutive nucleotides, 4000-8000 consecutive nucleotides, and 8000-9416 consecutive nucleotides of **SEQ. ID. NO.: 14** or an RNA that corresponds to these sequences. The example below describes the use of ribavirin in conjunction with a commercial HBV vaccine preparation.

### EXAMPLE 5

The adjuvant effect of ribavirin was tested when mixed with two doses of a commercially available vaccine containing HBsAg and alum. (Engerix. SKB). Approximately 0.2µg or 2µg of

Engerix vaccine was mixed with either PBS or 1mg ribavirin in PBS and the mixtures were injected intra peritoneally into groups of mice (three per group). A booster containing the same mixture was given on week four and all mice were bled on week six. The serum samples were diluted from 1:60 to 1:37500 and the dilutions were tested by EIA, as described above, except that purified human HBsAg was used as the solid phase antigen. As shown in **TABLE 9**, vaccine formulations having ribavirin enhanced the response to 2µg of an existing vaccine despite the fact that the vaccine already contained alum. That is, by adding ribavirin to a suboptimal vaccine dose (i.e., one that does not induce detectable antibodies alone) antibodies became detectable, providing evidence that the addition of ribavirin allows for the use of lower antigen amounts in a vaccine formulation without compromising the immune response.

**TABLE 9**

| Week | Endpoint antibody titer to HBsAg in EIA | | | | | | | | | | | |
|---|---|---|---|---|---|---|---|---|---|---|---|---|
| | 0.02 µg Engerix | | | | | | 0.2µg Engerix | | | | | |
| | No ribavirin | | | 1mg ribavirin | | | No ribavirin | | | 1mg ribavirin | | |
| | #1 | #2 | #3 | #1 | #2 | #3 | #1 | #2 | #3 | #1 | #2 | #3 |
| 6 | <60 | <60 | <60 | <60 | <60 | <60 | <60 | <60 | <60 | 300 | 60 | <60 |

Some HCV vaccine compositions comprise ribavirin and a HCV peptide of at least 3 consecutive amino acids of **SEQ. ID. NO.: 1** or a nucleic acid encoding said HCV peptide. That is, a vaccine composition can comprise ribavirin and one or more HCV peptides with a length of at least 3-10 consecutive amino acids, 10-50 consecutive amino acids, 50-100 consecutive amino acids, 100-200 consecutive amino acids, 200-400 consecutive amino acids, 400-800 consecutive amino acids, 800-1200 consecutive amino acids, 1200-1600 consecutive amino acids, 1600-2000 consecutive amino acids, 2000-2500 consecutive amino acids, and 2500-3011 consecutive amino acids of **SEQ. ID. NO.:1** or a nucleic acid encoding one or more of said fragments.

HCV compositions may comprise ribavirin and a peptide of at least 3 consecutive amino acids of HCV core protein **(SEQ. ID. NO.: 2),** HCV E1 protein **(SEQ. ID. NO.: 3),** HCV E2 protein **(SEQ. ID. NO.: 4),** HCV NS2 **(SEQ.ID. NO.: 5),** HCV NS3 **(SEQ. ID. NO.: 6),** HCV NS4A **(SEQ. ID. NO.: 7),** HCV NS4B **(SEQ. ID. NO.: 8),** or HCV NS5A/B **(SEQ.ID. NO.: 9)** or peptides consisting of combinations of these domains. HCV vaccines may comprise ribavirin and a peptide with a length of at least 3-10 consecutive amino acids, 10-50 consecutive amino acids, 50-100 consecutive amino acids, 100-200 consecutive amino acids, 200-400 consecutive amino acids, 400-800 consecutive amino acids, and 800-1040 consecutive amino acids of any one or more of **(SEQ. ID. NOs.: 2-9).** These domains correspond to amino acid residues 1-182, 183-379, 380-729, 730-1044, 1045-1657, 1658-1711, 1712-1971, or 1972-3011 **of SEQ. ID. NO.: 1.** The disclosure includes one or more of 1-182, 183-379, 380-729, 730-1044, 1045-1657,1658-1711, 1712-1971, or 1972-3011 of **SEQ. ID. NO.:1** or fragments thereof.

Vaccine compositions comprising ribavirin and a nucleic acid encoding one or more of the peptides described above are also disclosed. Preferred nucleic acid-based antigens include a nucleotide sequence of at least 9 consecutive nucleotides of HCV **(SEQ. ID. NO.: 13).** That is, a nucleic acid based antigen can comprise at least 9-25 consecutive nucleotides, 25-50 consecutive nucleotides, 50-100 consecutive nucleotides, 100-200 consecutive nucleotides, 200-500 consecutive nucleotides, 500-1000 consecutive nucleotides, 1000-2000 consecutive nucleotides, 2000-4000 consecutive nucleotides, 4000-8000 consecutive nucleotides, and 8000-9416 consecutive nucleotides of any one of **SEQ. ID. NOs.: 13** or an RNA that corresponds to these sequences. The section below discusses some compositions containing ribavirin and an antigen.

### Compositions containing ribavirin and an antigen

Compositions (e.g., vaccines) that comprise ribavirin and an antigen or epitope of a pathogen (e.g., virus, bacteria, mold, yeast, and parasite) may contain other ingredients including, but not limited to, adjuvants, binding agents, excipients such as stabilizers (to promote long term storage), emulsifiers, thickening agents, salts, preservatives, solvents, dispersion media, coatings, antibacterial and antifungal agents, isotonic and absorption delaying agents and the like. These compositions are suitable for treatment of animals either as a preventive measure to avoid a disease or condition or as a therapeutic to treat animals already afflicted with a disease or condition.

Many other ingredients can be present in the vaccine. For example, the ribavirin and antigen can be employed in admixture with conventional excipients (e.g., pharmaceutically acceptable organic or inorganic carrier substances suitable for parenteral, enteral (e.g., oral) or topical application that do not deleteriously react with the ribavirin and/or antigen). Suitable pharmaceutically acceptable carriers include, but are not limited to, water, salt solutions, alcohols, gum arabic, vegetable oils, benzyl alcohols, polyetylene glycols, gelatine, carbohydrates such as lactose, amylose or starch, magnesium stearate, talc, silicic acid, viscous paraffin, perfume oil, fatty acid monoglycerides and diglycerides, pentaerythritol fatty acid esters, hydroxy methylcellulose, polyvinyl pyrrolidone, etc. Many more suitable carriers are described in Remmington's Pharmaceutical Sciences, 15th Edition, Easton:Mack Publishing Company, pages 1405-1412 and 1461-1487(1975) and The National Formulary XIV, 14th Edition, Washington, American Pharmaceutical Association (1975).

The gene constructs described herein may be formulated with or administered in conjunction with agents that increase uptake and/or expression of the gene construct by the cells relative to uptake and/or expression of the gene construct by the cells that occurs when the identical genetic vaccine is administered in the absence of such agents. Such agents and the protocols for administering them in conjunction with gene constructs are described in U.S. Ser. No. 08/008,342 filed Jan. 26, 1993, U.S. Ser. No. 08/029,336 filed Mar. 11, 1993, U.S. Ser. No. 08/125,012 filed Sep. 21, 1993, PCT Patent Application Serial Number PCT/US94/00899 filed Jan. 26, 1994, and U.S. Ser. No. 08/221,579 filed Apr. 1, 1994. Examples of such agents include: CaPO₄, DEAE dextran, anionic lipids; extracellular matrix-active enzymes; saponins; lectins; estrogenic compounds and steroidal hormones; hydroxylated lower alkyls; dimethyl sulfoxide (DMSO); urea; and benzoic acid esters anilides, amidines, urethanes and the hydrochloride salts thereof such as those of the family of local anesthetics. In addition, the gene constructs are encapsulated within/administered in conjunction with lipids/polycationic complexes.

Vaccines can be sterilized and if desired mixed with auxiliary agents, e.g., lubricants, preservatives, stabilizers, wetting agents, emulsifiers, salts for influencing osmotic pressure, buffers, coloring, flavoring and/or aromatic substances and the like that do not deleteriously react with ribavirin or the antigen.

The effective dose and method of administration of a particular vaccine formulation can vary based on the individual patient and the type and stage of the disease, as well as other factors known to those of skill in the art. Therapeutic efficacy and toxicity of the vaccines can be determined by standard pharmaceutical procedures in cell cultures or experimental animals, e.g., ED₅₀ (the dose therapeutically effective in 50% of the population). The data obtained from cell culture assays and animal studies can be used to formulate a range of dosage for human use. The dosage of the vaccines lies preferably within a range of circulating concentrations that include the ED₅₀ with no toxicity. The dosage varies within this range depending upon the type of ribavirin derivative and antigen, the dosage form employed, the sensitivity of the patient, and the route of administration.

Since ribavirin has been on the market for several years, many dosage forms and routes of administration are known. All known dosage forms and routes of administration can be provided within the context of the embodiments described herein. Preferably, an amount of ribavirin that is effective to enhance an immune response to an antigen in an animal can be considered to be an amount that is sufficient to achieve a blood serum level of antigen approximately 0.25 - 12.5 µg/ml in the animal, preferably, about 2.8µg/ml. In some embodiments, the amount of ribavirin is determined according to the body weight of the animal to be given the vaccine. Accordingly, the amount of ribavirin in a vaccine formulation can be from about 0.1 - 6.0mg/kg body weight That is, some embodiments have an amount of ribavirin that corresponds to approximately 0.1 - 1.0mg/kg, 1.1 - 2.0mg/kg, 2.1 - 3.0mg/kg, 3.1 - 4.0mg/kg, 4.1 - 5.0mg/kg, 5.1, and 6.0mg/kg body weight of an animal. More conventionally, the vaccines contain approximately 0.25mg - 2000mg of ribavirin. That is, some embodiments have approximately 250µg, 500µg, 1mg, 25mg, 50mg, 100mg, 150mg, 200mg, 250mg, 300mg, 350mg, 400mg, 450mg, 500mg, 550mg, 600mg, 650mg, 700mg, 750mg, 800mg, 850mg, 900mg, 1g, 1.1g, 1.2g, 1.3g, 1.4g, 1.5g, 1.6g, 1.7g, 1.8g, 1.9g, and 2g of ribavirin.

Conventional vaccine preparations can be modified by adding an amount of ribavirin that is sufficient to enhance an immune response to the antigen. That is, existing conventional vaccine formulations can be modified by simply adding ribavirin to the preparation or by administering the conventional vaccine in conjunction with ribavirin (e.g., shortly before or after providing the antigen). As one of skill in the art will appreciate, the amount of antigens in a vaccine can vary depending on the type of antigen and its immunogenicity. The amount of antigens in the vaccines can vary accordingly. Nevertheless, as a general guide, the vaccines can have approximately 0.25mg - 5mg, 5-10mg, 10-100mg, 100-500mg, and upwards of 2000mg of an antigen (e.g., a hepatitis viral antigen).

In some approaches described herein, the exact amount of ribavirin and/or antigen is chosen by the individual physician in view of the patient to be treated. Further, the amounts of ribavirin can be added in combination with or separately from the same or equivalent amount of antigen and these amounts can be adjusted during a particular vaccination protocol so as to provide sufficient levels in light of patient-specific or antigen-specific considerations. In this vein, patient-specific and antigen-specific factors that can be taken into account include, but are not limited to, the severity of the disease state of the patient, age, and weight of the patient, diet, time and frequency of administration, drug combination(s), reaction sensitivities, and tolerance/response to therapy. The next section describes the discovery of a novel HCV gene and the creation of mutant HCV sequences, which can be used with the embodiments described herein.

### EXAMPLE 6 Novel NS3/4A and mutant NS3/4A sequences

A novel nucleic acid and protein corresponding to the NS3/4A domain of HCV was cloned from a patient infected with HCV **(SEQ. ID. NOs.:** 16 and 17). A Genebank search revealed that the cloned sequence had the greatest homology to HCV sequences but was only 93% homologous to the closest HCV relative (accession no AJ 278830). A truncated mutant of the novel NS3/4A peptide and NS3/4A mutants, which lack a proteolytic cleavage site, were also created. It was discovered that these novel peptides and nucleic acids encoding said peptides were potent immunogens that can be mixed with ribavirin so as to make a composition that provides a recipient with a potent immune response to HCV. The cloning of the novel NS3/4A domain and the creation of the various NS3/4A mutants is now described.

The NS3/4A sequence was amplified from the serum of an HCV-infected patient (HCV genotype 1a) using the Polymerase Chain Reaction (PCR). Total RNA was extracted from serum, cDNA synthesis, and PCR was performed according to standard protocols (Chen M et al., J. Med Virol. 43:223-226 (1995)). The cDNA synthesis was initiated using the antisense primer "NS4KR" (5'-CCG TCT AGA TCA GCA CTC TTC CAT TTC ATC-3' **(SEQ. ID. NO.: 18)).** From this cDNA, a 2079 base pair DNA fragment of HCV, corresponding to amino acids 1007 to 1711, which encompasses the NS3 and NS4A genes, was amplified. A high fidelity polymerase (Expand High Fidelity PCR, Boehringer-Mannheim, Mannheim, Germany) was used with the "NS3KF" primer (5'-CCT GAA TTC ATG GCG CCT ATC ACG GCC TAT-3' **(SEQ. ID. NO.: 19)** and the NS4KR primer. The NS3KF primer contained a *EcoRI* restriction enzyme cleavage site and a start codon and the primer NS4KR contained a *XbaI* restriction enzyme cleavage site and a stop codon.

The amplified fragment was then sequenced **SEQ. ID. NO.: 16.** Sequence comparison analysis revealed that the gene fragment was indeed amplified from a viral strain of genotype 1a. A computerized BLAST search against the Genbank database using the NCBI website revealed that the closest HCV homologue was 93% identical in nucleotide sequence.

The amplified DNA fragment was then digested with *EcoRI* and *XbaI,* and was inserted into a pcDNA3.1/His plasmid (Invitrogen) digested with the same enzymes. The NS3/4A-pcDNA3.1 plasmid was then digested with *EcoRI* and *XbaI* and the insert was purified using the QiaQuick kit (Qiagen, Hamburg, Germany) and was ligated to a *EcoRI*/*Xba I* digested pVAX vector (Invitrogen) so as to generatethe NS3/4A-pVAX plasmid.

The rNS3 truncated mutant was obtained by deleting NS4A sequence from the NS3/4A DNA. Accordingly, the NS3 gene sequence of NS3/4A-pVAX was PCR amplified using the primers NS3KF and *3'NotI* (5'-CCA CGC GGC CGC GAC GAC CTA CAG-3' **(SEQ. ID. NO.:** 20)) containing *EcoRI* and *Not I* resection sites, respectively. The NS3 fragment (1850 bp) was then ligated to a *EcoRI* and *Not I* digested pVAX plasmid to generate the NS3-pVAX vector. Plasmids were grown in BL21 *E.coli* cells. The plasmids were sequenced and were verified by restriction cleavage and the results were as to be expected based on the original sequence.

To change the proteolytic cleavage site between NS3 and NS4A, the NS3/4A-pVAX plasmid was mutagenized using the QUICKCHANGE™ mutagenesis kit (Stratagene), following the manufacturer's recommendations. To generate the "TPT" mutation, the plasmid was amplified using the primers 5'-CTGGAGGTCGTCACGCCTACCTGGGTGCTCGTT-3' **(SEQ. ID. NO.:** 21) and 5'-ACCGAGCACCCAGGTAGGCGTGACGACCTCCAG-3' **(SEQ. ID. No.: 22)** resulting in NS3/4A-TPT-pVAX. To generate the "RGT" mutation, the plasmid was amplified using the primers 5'-CTGGAGGTCGTCCGCGGTACCTGGGTGCTCGTT 3' (SEQ. ID. NO.: 23) and 5'-ACCGAGCACCCAGGTACC-GCGGACGACCTCCAG-3' **(SEQ. ID. NO.: 24)** resulting in NS3/4A-RGT-pVAX.

All mutagenized constructs were sequenced to verify that the mutations had been correctly made. Plasmids were grown in competent BL21 E. coli. The plasmid DNA used for in vivo injection was purified using Qiagen DNA purification columns, according to the manufacturers instructions (Qiagen GmbH, Hilden, FRG). The concentration of the resulting plasmid DNA was determined spectrophotometrically (Dynaquant, Pharmacia Biotech, Uppsala, Sweden) and the purified DNA was dissolved in sterile phosphate bluffer saline (PBS) at concentrations of 1 mg/ml. The amino acid sequences of the wild-type and mutated junctions are shown in TABLE 10. The section below describes several nucleic acids that encode HCV peptides.

**TABLE 10**

| Plasmid | Deduced amino acid sequence |
|---|---|
| *NS3/4A-pVAX | TKYMTCMSADLEVVTSTWVLVGGVL **(SEQ. ID. NO.: 25)** |
| NS3/4A-TGT-pVAX | TKYMTCMSADLEVVTGTWVLVGGVL **(SEQ. ID. NO.: 26)** |
| NS3/4A-RGT-pVAX | TKYMTCMSADLEVVRGTWVLVGGVL **(SEQ. ID. NO.: 27)** |
| NS3/4A-TPT-pVAX | TKYMTCMSADLEVVTPTWVLVGGVL **(SEQ. ID. NO.: 33)** |
| NS3/4A-RPT-pVAX | TKYMTCMSADLEWRPTRNLVGGVL **(SEQ. ID. NO.: 34)** |
| NS3/4A-RPA-pVAX | TKYMTCMSADLEVVRPAWVLVGGVL **(SEQ. ID. NO.: 35)** |
| NS3/4A-CST-pVAX | TKYMTCMSADLEVVCSTWVLVGGVL **(SEQ. ID. NO.: 36)** |
| NS3/4A-CCST-pVAX | TKYMTCMSADLEVCCSTWVLVGGVL **(SEQ. ID. NO.: 37)** |
| NS3/4A-SSST-pVAX | TKYMTCMSADLEVSSSTWLVGGVL **(SEQ.ID. NO.: 38)** |
| NS3/4A-SSSSCST-pVAX | TKYMTCMSADSSSSCSTWVLVGGVL **(SEQ. ID. NO.: 39)** |
| NS3A/4A-VVVVTST-pVAX | TKYMTCMSADWWTSTWVLVGGVL **(SEQ. ID. NO.: 40)** |
| NSS-pVAX | ASEDVVCCSMSYTWTG **(SEQ. ID. NO.: 41)** |
| NS5A/B-pVAX | SSEDVVCCSMWLVGGVL **(SEQ. ID. NO.: 42)** |

| | |
|---|---|
| *The wild type sequence for the NS3/4A fragment is NS3/4A-pVAX. The NS3/4A breakpoint is identified by underline, wherein the P1 position corresponds to the first Thr (T) and the P1' position corresponds to the next following amino acid the NS3/4A-pVAX sequence. In the wild type NS3/4A sequence the NS3 protease cleaves between the P1 and P1' positions. | |

### Nucleic acids encoding HCVpeptides

The nucleic acid embodiments include nucleotides encoding the HCV peptides described herein (e.g., **SEQ. ID. NO.:17, 29, 31, 32,** and 43-49) or fragments thereof at least 4, 6, 8,10, 12,15, or 20 amino acids in length (e.g., **SEQ.ID. NOs.: 25-27,** and 33-42). The disclosure made includes genomic DNA, RNA, and cDNA encoding these HCV peptides. The HCV nucleotides not only include the DNA sequences shown in the sequence listing (e.g., SEQ. ID. NO.: 16) but also include nucleotide sequences encoding the amino acid sequences shown in the sequence listing (e.g., SEQ. ID. NO.: 17) and any nucleotide sequence that hybridizes to the DNA sequences shown in the sequence listing under stringent conditions (e.g., hybridization to filter-bound DNA in 0.5 M NaHPO₄, 7.0% sodium dodecyl sulfate (SDS), 1 mM EDTA at 50°C) and washing in 0.2 X SSC/0.2% SDS at 50°C and any nucleotide sequence that hybridizes to the DNA sequences that encode an amino acid sequence provided in the sequence listing (SEQ. ID. NOs.: 17) under less stringent conditions (e.g., hybridization in 0.5 M NaHPO₄, 7.0% sodium dodecyl sulfate (SDS), 1 mM EDTA at 37°C and washing in 0.2X SSC/0.2% SDS at 37C).

The nucleic acid also include fragments, modifications, derivatives, and variants of the sequences described above. Nucleic acids having at least 12 consecutive bases of one of the novel HCV sequences or a sequence complementary thereto may be desired. Fragments include at least 12 consecutive bases of a nucleic acid encoding the NS3/4A molecule of SEQ. ID. NO.: 17 or a sequence complementary thereto.

Nucleic acids can have from 12 to approximately 2079 consecutive nucleotides. Some DNA fragments for example, include nucleic acids having at least 12-15, 15-20, 20-30, 30-50, 50-100, 100-200, 200-500, 500-1000, 1000-1500, 1500-2079 consecutive nucleotides of SEQ. ID. NO.: 16 or a complement thereof. Nucleic acids can also be altered by mutation such as substitutions, additions, or deletions. Due to the degeneracy of nucleotide coding sequences, for example, other DNA sequences that encode substantially the same HCV amino acid sequence as depicted in SEQ. ID. NOs: 17 may be used. These include, but are not limited to, nucleic acid sequences encoding all or portions of NS3/4A (SEQ. ID. NO.: 16) or nucleic acids that complement all or part of this sequence that have been altered by the substitution of different codons that encode a functionally equivalent amino acid residue within the sequence, thus producing a silent change, or a functionally non-equivalent amino acid residue within the sequence, thus producing a detectable change.

By using the nucleic acid sequences described above, probes that complement these molecules can be designed and manufactured by oligonucleotide synthesis. Desirable probes comprise a nucleic acid sequence of (SEQ. ID NO.: 16) that is unique to this HCV isolate. These probes can be used to screen cDNA from patients so as to isolate natural sources of HCV, some of which may be novel HCV sequences in themselves. Screening can be by filter hybridization or by PCR, for example. By filter hybridization, the labeled probe preferably contains at least 15-30 base pairs of the nucleic acid sequence of (SEQ. ID. NO.: 16) that is unique to to this NS3/4A peptide. The hybridization washing conditions used are preferably of a medium to high stringency. The hybridization can be performed in 0.5M NaHPO₄, 7.0% sodium dodecyl sulfate (SDS), 1 mM EDTA at 42°C overnight and washing can be performed in 0.2X SSC/0.2% SDS at 42°C. For guidance regarding such conditions see, for example, Sambrook et al., 1989, Molecular Cloning, A Laboratory Manual, Cold Springs Harbor Press, N.Y.; and Ausubel et al., 1989, Current Protocols in Molecular Biology, Green Publishing Associates and Wiley Interscience, N.Y.

HCV nucleic acids can also be isolated from patients infected with HCV using the nucleic acids described herein. (See also *Example 6*). Accordingly, RNA obtained from a patient infected with HCV is reverse transcribed and the resultant cDNA is amplified using PCR or another amplification technique. The primers are preferably obtained from the NS3/4A sequence (SEQ. ID. NO.: 16).

For a review of PCR technology, see Molecular Cloning to Genetic Engineering, White, B.A. Ed. in Methods in Molecular Biology 67: Humana Press, Totowa (1997) and the publication entitled "PCR Methods and Applications" (1991, Cold Spring Harbor Laboratory Press). For amplification of mRNAs, it is within the scope of the invention to reverse transcribe mRNA into cDNA followed by PCR (RT-PCR); or, to use a single enzyme for both steps as described in U.S. Patent No. 5,322,770. Another technique involves the use of Reverse Transcriptase Asymmetric Gap Ligase Chain Reaction (RT-AGLCR), as described by Marshall R.L. et al. (PCR Methods and Applications 4:80-84, 1994).

Briefly, RNA is isolated, following standard procedures. A reverse transcription reaction is performed on the RNA using an oligonucleotide primer specific for the most 5' end of the amplified fragment as a primer of first strand synthesis. The resulting RNA/DNA hybrid is then "tailed" with guanines using a standard terminal transferase reaction. The hybrid is then digested with RNAse H, and second strand synthesis is primed with a poly-C primer. Thus, cDNA sequences upstream of the amplified fragment are easily isolated. For a review of cloning strategies which can be used, see e.g., Sambrook et al.,1989, supra.

In each of these amplification procedures, primers on either side of the sequence to be amplified are added to a suitably prepared nucleic acid sample along with dNTPs and a thermostable polymerase, such as Taq polymerase, Pfu polymerase, or Vent polymerase. The nucleic acid in the sample is denatured and the primers are specifically hybridized to complementary nucleic acid sequences in the sample. The hybridized primers are then extended. Thereafter, another cycle of denaturation, hybridization, and extension is initiated. The cycles are repeated multiple times to produce an amplified fragment containing the nucleic acid sequence between the primer sites. PCR has further been described in several patents including US Patents 4,683,195, 4,683,202 and 4,965,188.

The primers are selected to be substantially complementary to a portion of the nucleic acid sequence of (SEQ. ID. NO.: 16) that is unique to this NS3/4A molecule, thereby allowing the sequences between the primers to be amplified. Preferably, primers are at least 16-20, 20-25, or 25-30 nucleotides in length. The formation of stable hybrids depends on the melting temperature (Tm) of the DNA. The Tm depends on the length of the primer, the ionic strength of the solution and the G+C content. The higher the G+C content of the primers, the higher is the melting temperature because G:C pairs are held by three H bonds whereas A:T pairs have only two. The G+C content of the amplification primers described herein preferably range between 10 and 75 %, more preferably between 35 and 60 %, and most preferably between 40 and 55 %. The appropriate length for primers under a particular set of assay conditions can be empirically determined by one of skill in the art.

The spacing of the primers relates to the length of the segment to be amplified. Amplified segments carrying nucleic acid sequence encoding HCV peptides can range in size from at least about 25 bp to the entire length of the HCV genome. Amplification fragments from 25-1000 bp are typical, fragments from 50-1000 bp are preferred and fragments from 100-600 bp are highly preferred. It will be appreciated that amplification primers can be of any sequence that allows for specific amplification of the NS3/4A region and can, for example, include modifications such as restriction sites to facilitate cloning.

The PCR product can be subcloned and sequenced to ensure that the amplified sequences represent the sequences of an HCV peptide. The PCR fragment can then be used to isolate a full length cDNA clone by a variety of methods. For example, the amplified fragment can be labeled and used to screen a cDNA library, such as a bacteriophage cDNA library. Alternatively, the labeled fragment can be used to isolate genomic clones via the screening of a genomic library. Additionally, an expression library can be constructed utilizing cDNA synthesized from, for example, RNA isolated from an infected patient. In this manner, HCV geneproducts can be isolated using standard antibody screening techniques in conjunction with antibodies raised against the HCV gene product (For screening techniques, see, for example, Harlow, E. and Lane, eds., 1988, Antibodies: A Laboratory Manual, Cold Spring Harbor Press, Cold Spring Harbor.)

The disclosure also includes (a) DNA vectors that contain any of the foregoing nucleic acid sequences and/or their complements (i.e., antisense); (b) DNA expression vectors that contain any of the foregoing nucleic acid sequences operatively associated with a regulatory element that directs the expression of the nucleic acid; and (c) genetically engineered host cells that contain any of the foregoing nucleic acid sequences operatively associated with a regulatory element that directs the expression of the coding sequences in the host cell. These recombinant constructs are capable of replicating autonomously in a host cell. Alternatively, the recombinant constructs can become integrated into the chromosomal DNA of a host cell. Such recombinant polynucleotides typically comprise an HCV genomic or cDNA polynucleotide of semi-synthetic or synthetic origin by virtue of human manipulation. Therefore, recombinant nucleic acids comprising these sequences and complements thereof that are not naturally occurring are provided.

Although nucleic acids encoding an HCV peptide or nucleic acids having sequences that complement an HCV gene as they appear in nature can be employed, they, will often be altered, e.g., by deletion, substitution, or insertion and can ,be accompanied by sequence not present in humans. As used herein, regulatory elements include, but are not limited to, inducible and non-inducible promoters, enhancers, operators and other elements known to those skilled in the art that drive and regulate expression. Such regulatory elements include, but are not limited to, the cytomegalovirus hCMV immediate early gene, the early or late promoters of SV40 adenovirus, the lac system, the trp system, the TAC system, the TRC system, the major operator and promoter regions of phage A, the control regions of fd coat protein, the promoter for 3-phosphoglycerate kinase, the promoters of acid phosphatase, and the promoters of the yeast -mating factors.

In addition, recombinant HCV peptide-encoding nucleic acid sequences and their complementary sequences can be engineered so as to modify their processing or expression. For example, and not by way of limitation, the HCV nucleic acids described herein can be combined with a promoter sequence and/or ribosome binding site, or a signal sequence can be inserted upstream of HCV peptide-encoding sequences so as to permit secretion of the peptide and thereby facilitate harvesting or bioavailability. Additionally, a given HCV nucleic acid can be mutated *in vitro* or *in vivo*, to create and/or destroy translation, initiation, and/or termination sequences, or to create variations in coding regions and/or form new restriction sites or destroy preexisting ones, or to facilitate further *in vitro* modification. (*See Example 6*). Any technique for mutagenesis known in the art can be used, including but not limited to, *in vitro* site-directed mutagenesis. (Hutchinson et al., J. Biol. Chem., 253:6551 (1978)).

Further, nucleic acids encoding other proteins or domains of other proteins can be joined to nucleic acids encoding an HCV peptide so as to create a fusion protein. Nucleotides encoding fusion proteins can include, but are not limited to, a full length NS3/4A sequence (SEQ. ID. NO.: 16), a truncated NS3/4A sequence or a peptide fragment of an NS3/4A sequence fused to an unrelated protein or peptide, such as for example, poly histidine, hemagglutinin, an enzyme, fluorescent protein, or luminescent protein, as discussed below.

Surprisingly, it was discovered that the NS3-pVAX and NS3/4A-pVAX vectors were capable of eliciting a potent immune response when injected into an immunocompetent mammal. The example below describes these experiments in greater detail.

### EXAMPLE 7

To determine whether a humoral immune response was elicited by the NS3-pVAX and NS3/4A-pVAX vectors, the expression constructs described in *Example 6* were purified using the Qiagen DNA, purification system, according to the manufacturer's instructions and the purified DNA vectors were used to immunize groups of four to ten Balb/c mice. The plasmids were injected directly into regenerating tibialis anterior (TA) muscles as previously described (Davis et al., Human Gene Therapy 4(6):733 (1993)). In brief, mice were injected intramuscularly with 50 µl/TA of 0.01mM cardiotoxin (Latoxan, Rosans, France) in 0.9% sterile NaCl. Five days later, each TA muscle was injected with 50 µl PBS containing either rNS3 or DNA.

Inbred mouse strains C57/BL6 (H-2b) Balb/C (H-2d), and CBA (H-2k) were obtained from the breeding facility at Möllegard Denmark, Charles River Uppsala, Sweden, or B&K Sollentuna Sweden. All mice were female and were used at 4-8 weeks of age. For monitoring of humoral responses, all mice received a booster injection of 50 µl /TA of plasmid DNA every fourth week. In addition, some mice were given recombinant NS3 (rNS3) protein, which was purified as described herein. The mice receiving rNS3 were immunized no more than twice. All mice were bled twice a month.

Enzyme immunosorbent assays (EIAs) were used to detect the presence of murine NS3 antibodies. These assays were performed essentially as described in (Chen et al., Hepatology 28(1): 219 (1998)). Briefly, rNS3 was passively adsorbed overnight at 4°C to 96-well microtiter plates (Nunc, Copenhagen, Denmark) at 1 µg/ml in 50 mM sodium carbonate buffer (pH 9.6). The plates were then blocked by incubation with dilution buffer containing PBS, 2% goat serum, and 1% bovine serum albumin for one hour at 37°C. Serial dilutions of mouse sera starting at 1:60 were then incubated on the plates for one hour. Bound murine serum antibodies were detected by an alkaline phosphatase conjugated goat anti-mouse IgG (Sigma Cell Products, Saint Louis, MO) followed by addition of the substrate pNPP (1 tablet/5ml of 1M Diethanol amine buffer with 0.5 mM MgCl₂). The reaction was stopped by addition of 1M NaOH and absorbency was read at 405 nm.

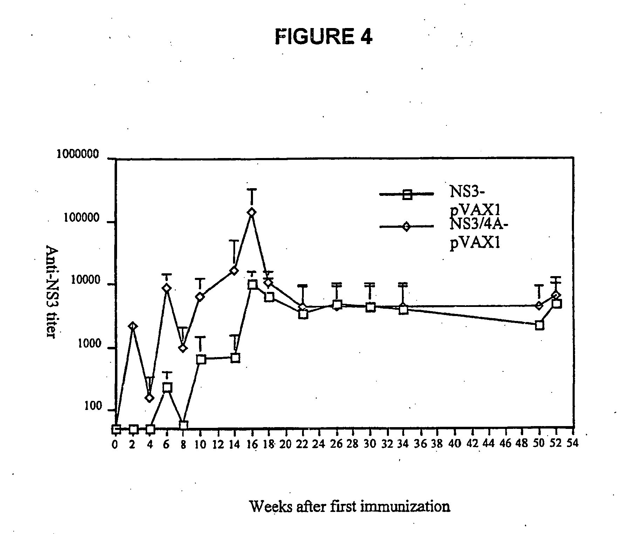

After four weeks, four out of five mice immunized with NS3/4A-pVAX had developed NS3 antibodies, whereas one out of five immunized with NS3-pVAX had developed antibodies (FIGURE 4). After six weeks, four out of five mice immunized with NS3/4A-pVAX had developed high levels (>10⁴) of NS3 antibodies (mean levels 10800±4830) and one had a titer of 2160. Although all mice immunized with NS3-pVAX developed NS3 antibodies, none of them developed levels as high as that produced by the NS3/4A-pVAX construct (mean levels 1800±805). The antibody levels elicited by the NS3/4A fusion construct were significantly higher than those induced by NS3-pVAX at six weeks (mean ranks 7.6 v.s 3.4, p<0.05, Mann-Whitney rank sum test, and p<0.01, Students t-test). Thus, immunization with either NS3-pVAX or NS3/4A-pVAX resulted in the production of anti-NS3 antibodies, but the NS3/4A fusion gene was a more potent immunogen The example below describes experiments that were performed to determine if the NS314A-TPT-pVAX construct could elicit a potent immune response.

### EXAMPLE 8

To test if the enhanced immunogenicity of NS3/4A could be solely attributed to the presence of NS4A, or if the NS3/4A fusion protein in addition had to be cleaved at the NS3/4A junction, new experiments were performed. In a first experiment, the immunogenicity of the NS3-pVAX, NS3/4A-pVAX, and NS3/4A-TPT-pVAX vectors were compared in Balb/c mice. Mice were immunised on week 0 as described above, and, after two weeks, all mice were bled and the presence of antibodies to NS3 at a serum dilution of 1:60 was determined (TABLE 11). Mice were bled again on week 4. Although, the NS3/4A-TPT-pVAX vector was comparable to the NS3-pVAX vector (4/10 vs. 0/10; NS, Fisher's exact test), the NS3/4A-pVAX vector continued to be the most potent immunogen. Thus, all of the HCV constructs that were introduced into mice were capable of eliciting an immune response against NS3, however, the NS4A sequence and a functional proteolytic cleavage site between the NS3 and NS4A sequences provided for a more potent immune response.

**TABLE 11**

| Weeks from 1^{st} immunization | No. of antibody responders.to the respective immunogen after one 100µg *i.m.* immunization | | |
|---|---|---|---|
| | NS3-pVAX | NS3/4A-pVAX | NS3/4A-TPT-pVAX |
| 2 | 0/10 | 17/20 | 4/10 |
| 4 | | 20/20 | 10/10 |
| | 0/10 | (2415±3715) | (390±639) |
| | (<60) | 55% > 10³ | 50% >10² |
| | | 10% > 10⁴ | 10% > 10³ |

During the chronic phase of infection, HCV replicates in hepatocytes, and spreads within the liver. A major factor in combating chronic and persistent viral infections is the cell-mediated immune defense system. CD4+ and CD8+ lymphocytes infiltrate the liver during the chronic phase of HCV infection, but they are incapable of clearing the virus or preventing liver damage. In addition, persistent HCV infection is associated with the onset of hepatocellular carcinoma (HCC). The examples below describe experiments that were performed to determine whether the NS3 and NS3/4A construct were capable of eliciting a T-cell mediated immune response against NS3.

### EXAMPLE 9

To study whether the constructs described above were capable of eliciting a cell-mediated response against NS3, an *in vivo* tumor growth assay was perfomed. To this end, an SP2/0 tumor cell line stably transfected with the NS3/4A gene was made. The peDNA3.1 plasmid containing the NS3/4A gene was linearized by BgIII digestion. A total of 5µg linearized plasmid DNA was mixed with 60µg transfection reagent (Superfect, Qiagen, Germany) and the mixture was added to a 50% confluent layer of SP2/0 cells in a 35 mm dish. The transfected SP2/0 cells (NS3/4ASP2/0) were grown for 14 days in the presence of 800µg/ml geneticin and individual clones were isolated. A stable NS3/4A-expressing SP2/0 clone was identified using PCR and RTPCR. The cloned cell line was maintained in DMEM containing 10% fetal bovine serum, L-glutamine, and penicillin-streptomycin.

The *in vivo* growth kinetics of the SP2/0 and the NS3/4A-SP2/0 cell lines were then evaluated in Balb/c mice. Mice were injected subcutaneously with 2 x 10⁶ tumor cells in the right flank. Each day the size of the tumor was determined through the skin. The growth kinetics of the two cell lines was comparable. For example, the mean tumor sizes did not differ between the two cell lines at any time point. (*See.* TABLE 12). The example below describes experiments that were performed to determine whether mice immunized with the NS3/4A constructs had developed a T-cell response against NS3.

**TABLE 12**

| Mouse ID | Tumor cell line | Maximum *in vivo* tumor size at indicated time point | | | | | | | | |
|---|---|---|---|---|---|---|---|---|---|---|
| | | 5 | 6 | 7 | 8 | 11 | 12 | 13 | 14 | 15 |
| 1 | SP2/0 | 1.6 | 2.5 | 4.5 | 6.0 | 10.0 | 10.5 | 11.0 | 12.0 | 12.0 |
| 2 | SP2/0 | 1.0 | 1.0 | 2.0 | 3.0 | 7.5 | 7.5 | 8.0 | 11.5 | 11.5 |
| 3 | SP2/0 | 2.0 | 5.0 | 7.5 | 8.0 | 11.0 | 11.5 | 12.0 | 12.0 | 13.0 |
| 4 | SP2/0 | 4.0 | 7.0 | 8.0 | 10.0 | 13.0 | 15.0 | 16.5 | 16.5 | 17.0 |
| 5 | SP2/0 | 1.0 | 1.0 | 3.0 | 4.0 | 5.0 | 6.0 | 6.0 | 6.0 | 7.0 |
| Group mean | | 1,92 | 3.3 | 5.0 | 6.2 | 93 | 10.1 | 10.7 | 11.6 | 12.1 |
| 6 | NS3/4A-SP2/0 | 1.0 | 2.0 | 3.0 | 3.5 | 4.0 | 5.5 | 6.0 | 7.0 | 8.0 |
| 7 | NS3/4A-SP2/0 | 2.0 | 2.5 | 3.0 | 5.0 | 7.0 | 9.0 | 9.5 | 9.5 | 11.0 |
| 8 | NS3/4A-SP2/0 - | 1.0 | | 3.5 | 3.5 | 9.5 | 11.0 | 12.0 | 14.0 | 14.0 |
| 9 | NS3/4A-SP2/0 | 1.0 | 1.0 | 2.0 | 6.0 | 11.5 | 13.0 | 14.5 | 16.0 | 18.0 |
| 10 | NS3/4A-SP2/0 | 3.5 | 6.0 | 7.0 | 10.5 | 15.0 | 15.0 | 15.0 | 15.5 | 20.0 |
| Group mean | | 1,7 | 2.7, | 3.7 | 5.7 | 9.4 | 10.7 | 11.4 | 12.4 | 14.2 |
| p-value of student's t-test comparison between group means | | 0,7736 | 0.6918 | 0.4027 | 0.7903 | 0.9670 | 0.7986 | 0.7927 | 0.7508 | 0.4623 |

### EXAMPLE 10

To examine whether a T-cell response is elicited by the NS3/4A immunization, the capacity of an immunized mouse's immune defense system to attack the NS3-expressing tumor cell line was assayed. The protocol for testing for *in vivo* inhibition of tumor growth of the SP2/0 myeloma cell line in Balb/c mice has been described in detail previously (Encke et al., J. Immmol 161:4917 (1998)). Inhibition of tumor growth in this model is dependent on the priming of cytotoxic T lymphocytes (CTLs). Briefly, groups of ten mice were immunized *i.m.* five times with one month intervals with either 100µg NS3-pVAX or 100 µgNS3/4A-pVAX. Two weeks after the last immunization 2 x 10⁶ SP2/0 or NS3/4A-SP2/0 cells were injected into the right flank of each mouse. Two weeks later the mice were sacrificed and the maximum tumor sizes were measured. There was no difference between the mean SP2/0 and NS3/4A-SP2/0 tumor sizes in the NS3-pVAX immunized mice (*See* TABLE 13).

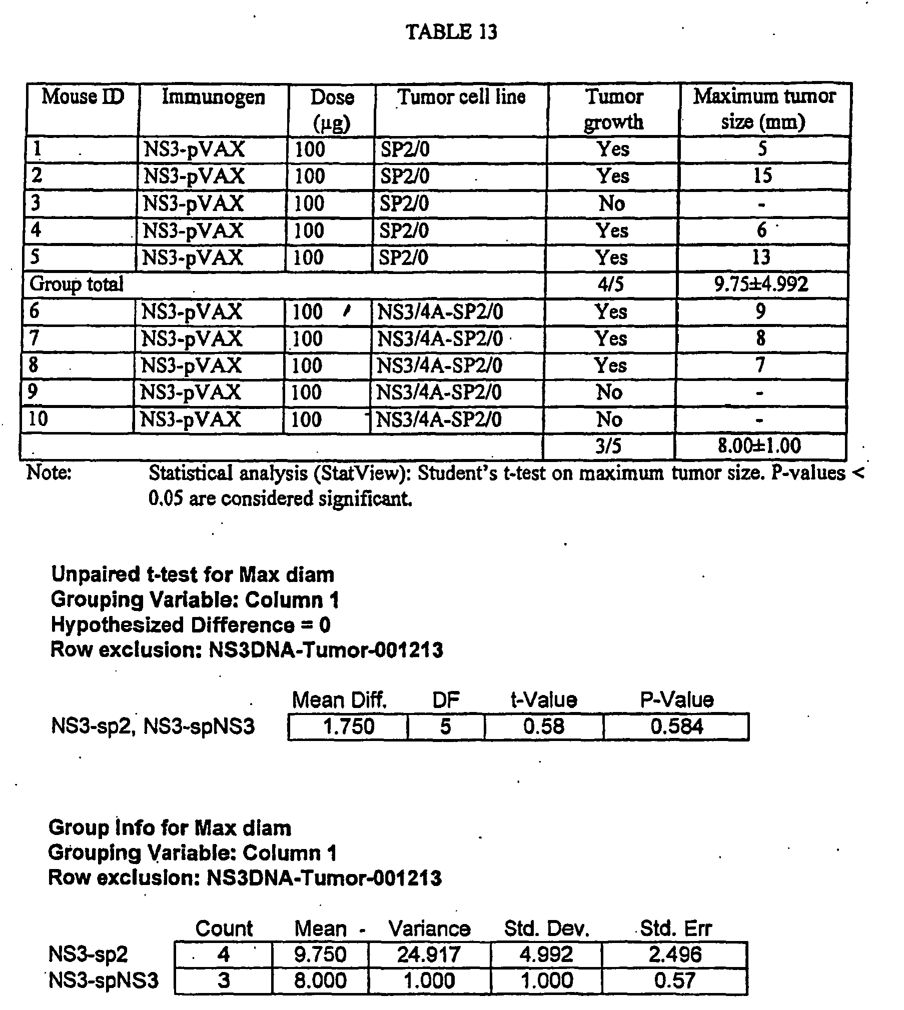

In the next set of experiments, the inhibition of SP2/0 or NS3/4A-SP2/0 tumor growth was evaluated in NS3/4A-pVAX immunized Balb/c mice. In mice immunized with the NS3/4A-pVAX plasmid the growth of NS3/4A-SP2/0 tumor cells was significantly inhibited as compared to growth of the non-transfected SP2/0 cells. (See TABLE 14). Thus, NS3/4A-pVAX immunization elicits CTLs that inhibit growth of cells expressing NS3/4A *in vivo*. The example below describes experiments that were performed to analyze the efficiency of various NS3 containing compositions in eliciting a cell-mediated response to NS3

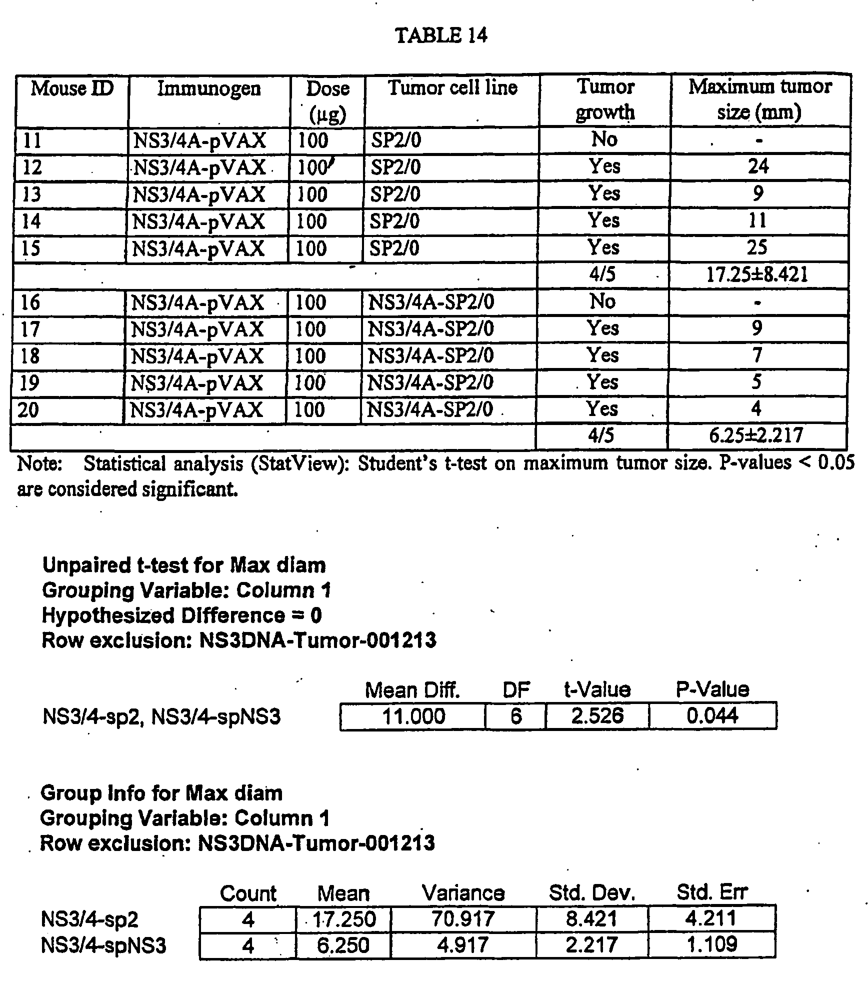

### EXAMPLE 11

To analyze whether administration of different NS3 containing compositions affected the elicitation of a cell-mediated immune response, mice were immunized with PBS, rNS3, irrelevant DNA or the NS3/4A construct, and tumor sizes were determined, as described above. Only the NS3/4A construct was able to elicit a T-cell response sufficient to cause a statistically significant reduction in tumor size (*See* **TABLE 15**). The example below describes experiments that were performed to determine whether the reduction in tumor size can be attributed to the generation of NS3-specific T-lymphocytes.

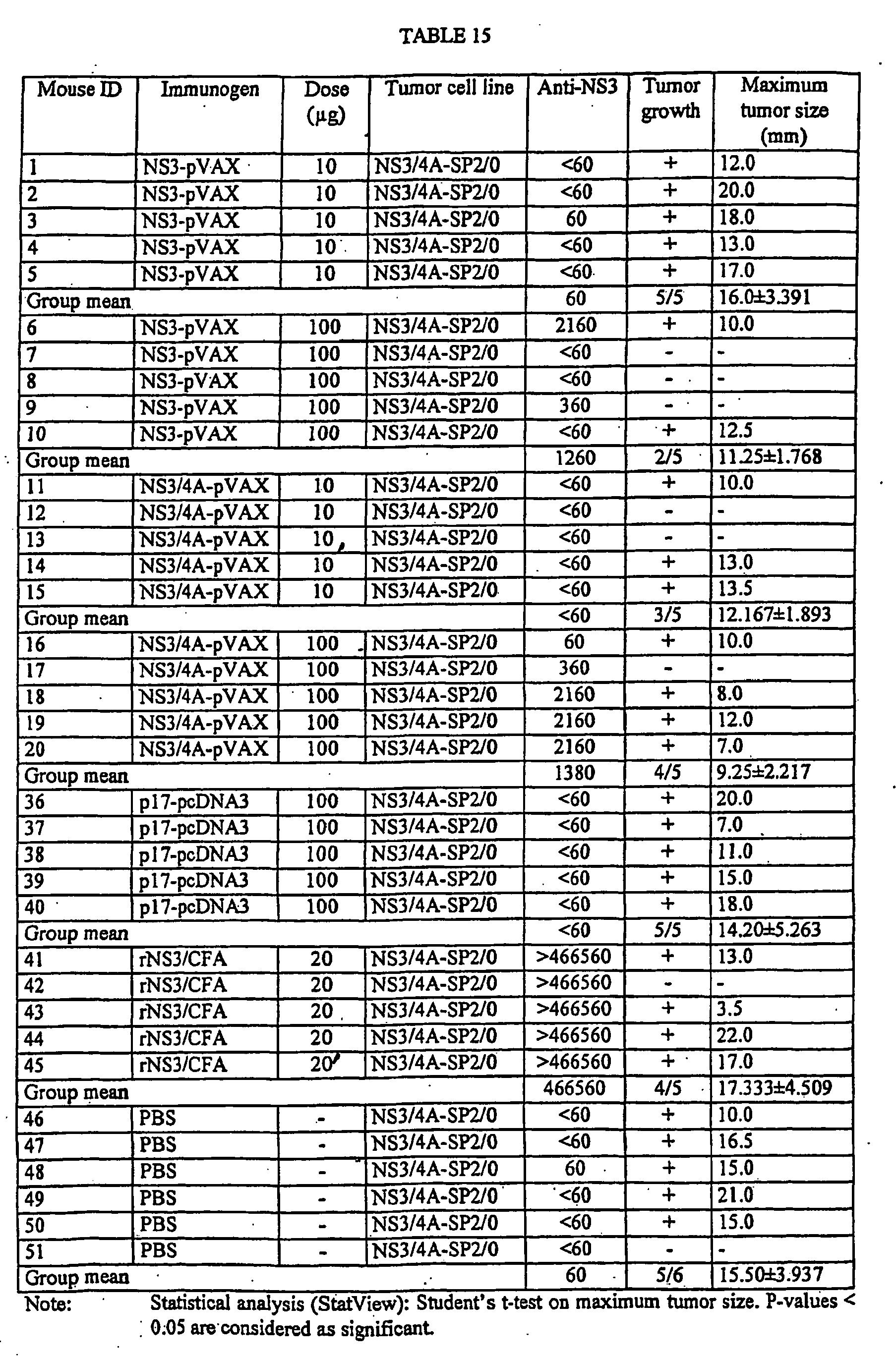

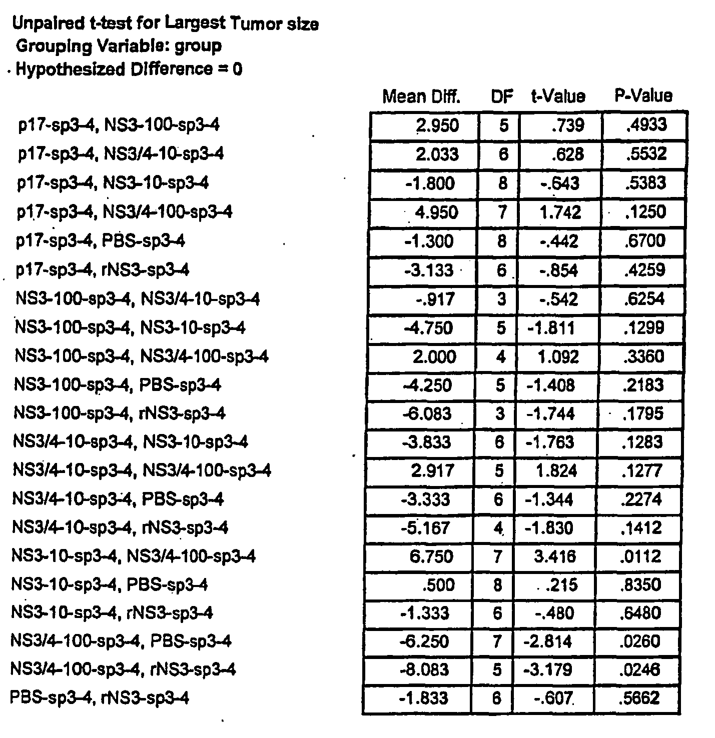

### EXAMPLE 12

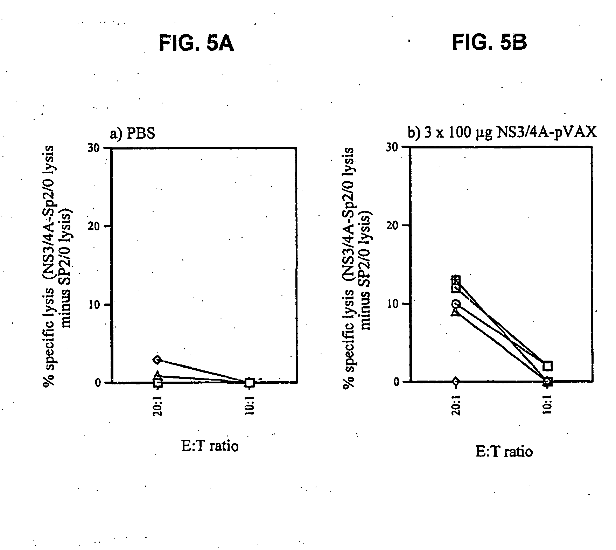

To determine whether NS3-specific T-cells were elicited by the NS3/4A immunizations, an *in vitro* T-cell mediated tumor cell lysis assay was employed. The assay has been described in detail previously (Townsend et al., J. Virol. 71:3365 (1997)). Briefly, groups of five Balb/c mice were immunized three times with 100µg NS3/4A-pVAX *i.m.* Two weeks after the last injection the mice were sacrificed and splenocytes were harvested. Re-stimulation cultures with 3 x 10⁶ splenocytes and 3 x 10⁶ NS3/4A-SP2/0 cells were set. After five days, a standard Cr⁵¹-release assay was performed using NS3/4A-SP2/0 or SP2/0 cells as targets. Percent specific lysis was calculated as the ratio between lysis of NS3/4A-SP210 cells and lysis of SP2/0 cells. Only mice immunized with NS3/4A-pVAX displayed specific lysis over 10% in four out of five tested mice, using an effector to target ratio of 20:1 (*See* FIGURES 5A and B). Accordingly, mice immunized with NS3/4A exhibited a reduction in cancer cell proliferation and/or NS3/4A caused the lysis of cancer cells. The section below describes several of the embodied HCV polypeptides in greater detail.

### HCV peptides

The nucleic acids encoding the HCV peptides, described in the previous section, can be manipulated using conventional techniques in molecular biology so as to create recombinant constructs that express the HCV peptides. HCV peptides or derivatives thereof, include but are not limited to, those containing as a primary amino acid sequence all of the amino acid sequence substantially as depicted in the Sequence Listing (**SEQ. ID. NOs**.: **17, 29- 32** and **43-49**) and fragments thereof at least four amino acids in length (e.g., **SEQ. ID. NOs.: 25-27,** and **33-42**) including altered sequences in which functionally equivalent amino acid residues are substituted for residues within the sequence resulting in a silent change. Fragments of a sequence of **SEQ. ID. NOs**.: **17, 29-32** and **43-49** may be at least four amino acids and comprise amino acid sequence unique to the discovered NS3/4A peptide (**SEQ. ID. NO.: 17**) including altered sequences in which functionally equivalent amino acid residues are substituted for residues within the sequence resulting in a silent change. The HCV peptides can be, for example, at least 12-15, 15-20, 20-25, 25-50, 50-100, 100-150, 150-250, 250-500 or 500-704 amino acids in length. Other fragments (e.g. **SEQ ID. NOs.: 25-27, and 33-42**) are also disclosed herein.

Also disclosed herein are HCV peptides that are substantially identical to those described above. That is, HCV peptides that have one or more amino acid residues within **SEQ. ID. NO**.: **17** and fragments thereof that are substituted by another amino acid of a similar polarity that acts as a functional equivalent, resulting in a silent alteration. Substitutes for an amino acid within the sequence can be selected from other members of the class to which the amino acid belongs. For example, the non-polar (hydrophobic) amino acids include alanine, leucine, isoleucine, valine, proline, phenylalanine, tryptophan, and methionine. The polar neutral amino acids include glycine, serine, threonine, cysteine, tyrosine, asparagine and glutamine. The positively charged (basic) amino acids include arginine, lysine, and histidine. The negatively charged (acidic) amino acids include aspartic acid and glutamic acid. The aromatic amino acids include phenylalanine, tryptophan, and tyrosine.

The HCV peptides described herein can be prepared by chemical synthesis methods (such as solid phase peptide synthesis) using techniques known in the art such as those set forth by Merrifield et al., J. Art Chem. Soc. 85:2149 (1964), Houghten et al., Proc. Natl. Acad Sci. USA, 82:51:32 (1985), Stewart and Young (Solid phase peptide synthesis, Pierce Chem Co., Rockford, IL (1984), and Creighton, 1983, Proteins: Structures and Molecular Principles, W. H. Freeman & Co., N.Y. Such polypeptides can be synthesized with or without a methionine on the amino terminus. Chemically synthesized HCV peptides can be oxidized using methods set forth in these references to form disulfide bridges.

While the HCV peptides described herein can be chemically synthesized, it can be more effective to produce these polypeptides by recombinant DNA technology. Such methods can be used to construct expression vectors containing the HCV nucleotide sequences described above, for example, and appropriate transcriptional and translational control signals. These methods include, for example, *in vitro* recombinant DNA techniques, synthetic techniques, and *in vivo* genetic recombination. Alternatively, RNA capable of encoding HCV nucleotide sequences can be chemically synthesized using, for example, synthesizers. See, for example, the techniques described in Oligonucleotide Synthesis, 1984, Gait, M. J. ed., IRL Press, Oxford. Accordingly, several embodiments concern cell lines that have been engineered to express the embodied HCV peptides. For example, some cells are made to empress the HCV peptides of (**SEQ. ID. NOs.: 17, 29- 32** and 43-49) or fragments of these molecules.

A variety of host-expression vector systems can be utilized to express the embodied HCV peptides. Suitable expression systems include, but are not limited to, microorganisms such as bacteria (e.g., *E*. *coli* or *B. subtilis)* transformed with recombinant bacteriophage DNA, plasmid DNA or cosmid DNA expression vectors containing HCV nucleotide sequences; yeast (e.g., *Saccharomyces, Pichia*) transformed with recombinant yeast expression vectors containing the HCV nucleotide sequences; insect cell systems infected with recombinant virus expression vectors (e.g., baculovirus) containing the HCV sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing HCV sequences; or mammalian cell systems (e.g., COS, CHO, BHK, 293, 3T3) harboring recombinant expression constructs containing promoters derived from the genome of mammalian cells (e.g., metallothionein promoter) or from mammalian viruses (e.g., the adenovirus late promoter; the vaccinia virus 7.5K promoter).

In bacterial systems, a number of expression vectors can be advantageously selected depending upon the use intended for the HCV gene product being expressed. For example, when a large quantity of such a protein is to be produced, for the generation of pharmaceutical compositions of HCV peptide or for raising antibodies to the HCV peptide, for example, vectors which direct the expression of high levels of fusion protein products that are readily purified can be desirable. Such vectors include, but are not limited, to the *E. coli* expression vector pUR278 (Ruther et al., EMBO J., 2:1791 (1983), in which the HCV coding sequence can be ligated individually into the vector in frame with the lacZ coding region so that a fusion protein is produced; pIN vectors (Inouye & Inouye, Nucleic Acids Res., 13:3101-3109 (1985); Van Heeke & Schuster, J. Biol. Chem., 264:5503-5509 (1989)); and the like. pGEX vectors can also be used to express foreign polypeptides as fusion proteins with glutathione S-transferase (GST). In general, such fusion proteins are soluble and can be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

In an insect *system, Autographa californica* nuclear polyhedrosis virus (AcNPV) is used as a vector to express foreign genes. The virus grows in *Spodoptera frugiperda* cells. The HCV coding sequence can be cloned individually into non-essential regions (for example the polyhedrin gene) of the virus and placed under control of an AcNPV promoter (for example the polyhedrin promoter). Successful insertion of an HCV gene coding sequence will result in inactivation of the polyhedrin gene and production of non-occluded recombinant virus, (i.e., virus lacking the proteinaceous coat coded for by the polyhedrin gene). These recombinant viruses are then used to infect *Spodoptera frugiperda* cells in which the inserted gene is expressed. (See e.g., Smith et al., . J. Virol. 46: 584 (1983); and Smith, U.S. Pat. No. 4,215,051).

In mammalian host cells, a number of viral-based expression systems can be utilized. In cases where an adenovirus is used as an expression vector, the HCV nucleotide sequence of interest can be ligated to an adenovirus transcription/translation control complex, e.g., the late promoter and tripartite leader sequence. This chimeric gene can then be inserted in the adenovirus genome by in *vitro* or *in* vivo recombination. Insertion in a non-essential region of the viral genome (e.g., region E1 or E3) will result in a recombinant virus that is viable and capable of expressing the HCV gene product in infected hosts. (See e.g., Logan & Shenk, Proc. Natl. Acad Sci. USA 81:3655-3659 (1984)). Specific initiation signals can also be required for efficient translation of inserted HCV nucleotide sequences. These signals include the ATG initiation codon and adjacent sequences.

However, in cases where only a portion of the HCV coding sequence is inserted, exogenous translational control signals, including, perhaps, the ATG initiation codon, can be provided. Furthermore, the initiation codon can be in phase with the reading frame of the desired coding sequence to ensure translation of the entire insert. These exogenous translational control signals and initiation codons can be of a variety of origins, both natural and synthetic. The efficiency of expression can be enhanced by the inclusion of appropriate transcription enhancer elements, transcription terminators, etc. (See Bittner et al., Methods in Enzymol., 153:516-544 (1987)).

In addition, a host cell strain can be chosen which modulates the expression of the inserted sequences, or modifies and processes the gene product in the specific fashion desired. Such modifications (e.g., glycosylation) and processing (e.g., cleavage) of protein products are important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells that possess the cellular machinery for proper processing of the primary transcript, glycosylation, and phosphorylation of the gene product can be used. Such mammalian host cells include, but are not limited to, CHO, VERO, BHK, HeLa, COS, MDCK, 293, 3T3, and WI38.

For long-term, high-yield production of recombinant proteins, stable expression is preferred. For example, cell lines that stably express the HCV peptides described above can be engineered. Rather than using expression vectors that contain viral origins of replication, host cells can be transformed with DNA controlled by appropriate expression control elements (e.g., promoter, enhancer sequences, transcription terminators, polyadenylation sites, etc.), and a selectable marker. Following the introduction of the foreign DNA, engineered cells are allowed to grow for 1-2 days in an enriched media; and then are switched to a selective media. The selectable marker in the recombinant plasmid confers resistance to the selection and allows cells to stably integrate the plasmid into their chromosomes and grow to form foci which in turn are cloned and expanded into cell lines. This method is advantageously used to engineer cell lines which express the HCV gene product

A number of selection systems can be used, including but not limited to the herpes simplex virus thymidine kinase (Wigler, et al., Cell 11:223 (1977), hypoxanthine-guanine phosphoribosyltransferase (Szybalska & Szybalski, Proc. Natl. Acad. Sci. USA 48:2026 (1962), and adenine phosphoribosyltransferase (Lowy, et al., Cell 22:817 (1980) genes can be employed in tk-, hgprt- or aprt- cells, respectively. Also, antimetabolite resistance can be used as the basis of selection for the following genes:-dhfr, which confers resistance to methotrexate (Wigler, et al., Proa Natl. Acad Sci. USA 77:3567 (1980); O'Hare, et al., Proc. Natl. Acad Sci USA 78:1527 (1981); gpt, which confers resistance to mycophenolic acid (Mulligan & Berg, Proc. Natl. Acad Sci. USA 78:2072 (1981); neo, which confers resistance to the aminoglycoside G-418 (Colberre-Garapin, et al., J. Mol. Biol. 150:1 (1981); and hygro, which confers resistance to hygromycin (Santerre, et al., Gene 30:147 (1984)).

Alternatively, any fusion protein can be readily purified by utilizing an antibody specific for the fusion protein being expressed. For example, a system described by Janknecht et al. allows for the ready purification of non-denatured fusion proteins expressed in human cell lines. (Janknecht, et al., Proc. Natl. Acad Sci. USA 88: 8972-8976 (1991)). In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺nitriloacetic acid-agarose columns and histidine-tagged proteins are selectively eluted with imidazole-containing buffers. The example below describes a method that was used to express the HCV peptides encoded by the embodied nucleic acids.

### EXAMPLE 13

To characterize the NS3/4A fusion protein, and the truncated and mutated versions thereof, the vector constructs, described in *Example* 6, were transcribed and translated *in vitro,* and the resulting polypeptides were visualized by sodium dodecyl sulfate-polyacrylamide gel electrophoresis (SDS-PAGE). *In vitro* transcription and translation were performed using the T7 coupled reticulocyte lysate system (Promega, Madison, WI) according to the manufacturer's instructions. All *in vitro* translation reactions of the expression constructs were carried out at 30°C with ³⁵S-labeled methionine (Amersham International, Plc, Buckinghamshire, UK). The labeled proteins were separated on 12% SDS-PAGE gels and visualized by exposure to X-ray film (Hyper Film-MP, Amersham) for 6-18 hours.

The *in vitro* analysis revealed that all proteins were expressed to high amounts from their respective expression constructs. The rNS3 construct (NS3-pVAX vector) produced a single peptide of approximately 61kDa, whereas, the TPT construct (NS3/4A-TPT-pVAX) and the RGT construct (NS3/4A-RGT-pVAX) produced a single polypeptide of approximately 67 kDa, which is identical to the molecular weight of the uncleaved NS3/4A peptide produced from the NS3/4A-pVAX construct. The cleaved product produced from the expressed NS3/4A peptide was approximately 61 kDa, which was identical in size to the rNS3 produced from the NS3-pVAX vector. These results demonstrated that the expression constructs were functional, the NS3/4A construct was enzymatically active, the rNS3 produced a peptide of the predicted size, and the TPT and RGT mutations completely abolished cleavage at the NS3-NS4A junction.

The sequences, constructs, vectors, clones, and other materials comprising the embodied HCV nucleic acids and peptides can be in enriched or isolated form. As used herein, "enriched" means that the concentration of the material is at least about 2, 5, 10, 100, or 1000 times its natural concentration (for example), advantageously 0.01%, by weight, preferably at least about 0.1% by weight. Enriched preparations from about 0.5%, 1%, 5%, 10%, and 20% by weight are also contemplated. The term "isolated" requires that the material be removed from its original environment (e.g., the natural environment if it is naturally occurring). For example, a naturally-occurring polynucleotide present in a living animal is not isolated, but the same polynucleotide, separated from some or all of the coexisting materials in the natural system, is isolated. It is also advantageous that the sequences be ip purified form. The term "purified" does not require absolute purity; rather, it is intended as a relative definition. Isolated proteins have been conventionally purified to electrophoretic homogeneity by Coomassie staining, for example. Purification of starting material or natural material to at least one order of magnitude, preferably two or three orders, and more preferably four or five orders of magnitude is expressly contemplated.

The HCV gene products described herein can also be expressed in plants, insects, and animals so as to create a transgenic organism. Desirable transgenic plant systems having an HCV peptide include *Arabadopsis,* maize, and *Chlamydomonas.* Desirable insect systems having an HCV peptide include, but are not limited to, *D. melanogaster* and *C*. *elegans.* Animals of any species, including, but not limited to, amphibians, reptiles, birds, mice, hamsters, rats, rabbits, guinea pigs, pigs, micro-pigs, goats, dogs, cats, and non-human primates, e.g., baboons, monkeys, and chimpanzees can be used to generate transgenic animals having an embodied HCV molecule. These transgenic organisms desirably exhibit germline transfer of HCV peptides described herein.

Any technique known in the art is preferably used to introduce the HCV transgene into animals to produce the founder lines of transgenic animals or to knock out or replace existing HCV genes. Such techniques include, but are not limited to pronuclear microinjection (Hoppe, P. C. and Wagner, T. E., 1989, U.S. Pat. No. 4,873,191); retrovirus mediated gene transfer into germ lines (Van der Putten et al., Proc. Natl. Acad. Sci., USA 82:6148-6152 (1985); gene targeting in embryonic stem cells (Thompson et al., Cell 56:313-321 (1989); electroporation of embryos (Lo, Mol Cell. Biol. 3:1803-1814 (1983); and sperm-mediated gene transfer (Lavitrano et al., Cell 57:717-723 (1989); see also Gordon, Transgenic Animals, Intl. Rev. Cytol. 115:171-229 (1989). The section below describes the manufacture of antibodies that interact with the HCV peptides described herein.

### Anti-HCV antibodies

Following synthesis or expression and isolation or purification of the HCV peptides, the isolated or purified peptide can be used to generate antibodies. Depending on the context, the term "antibodies" can encompass polyclonal, monoclonal, chimeric, single chain, Fab fragments and fragments produced by a Fab expression library. Antibodies that recognize the HCV peptides have many uses including, but not limited to, biotechnological applications, therapeutic/prophylactic applications, and diagnostic applications.

For the production of antibodies, various hosts including goats, rabbits, rats, mice, and humans etc. can be immunized by injection with an HCV peptide. Depending on the host species, various adjuvants can be used to increase immunological response. Such adjuvants include, but are not limited to, ribavirin, Freund's, mineral gels such as aluminum hydroxide, and surface active substances such as lysolecithin, pluronic polyols, polyanions, peptides, oil emulsions, keyhole limpet hemocyanin, and dinitrophenol. BCG (Bacillus Calmette-Guerin) and Corynebacterium parvum are also potentially useful adjuvants.

Peptides used to induce specific antibodies can have an amino acid sequence consisting of at least four amino acids, and preferably at least 10 to 15 amino acids. By one approach, short stretches of amino acids encoding fragments of NS3/4A are fused with those of another protein such as keyhole limpet hemocyanin such that an antibody is produced against the chimeric molecule. Additionally, a composition comprising ribavirin and NS3/4A (**SEQ. ID. NO**.: **17**), a fragment thereof at least 4, 6, 8, 10, 12, 15, or 20 amino acids in length, or a nucleic acid encoding one or more of these moleucles is administered to an animal. While antibodies capable of specifically recognizing HCV can be generated by injecting synthetic 3-mer, 10-mer, and 15-mer peptides that correspond to an HCV peptide into mice, a more diverse set of antibodies can be generated by using recombinant HCV peptides, prepared as decribed above.

To generate antibodies to an HCV peptide, substantially pure peptide is isolated from a transfected or transformed cell. The concentration of the peptide in the final preparation is adjusted, for example, by concentration on an Amicon filter device, to the level of a few micrograms/ml. Monoclonal or polyclonal antibody to the peptide of interest can then be prepared as follows:

Monoclonal antibodies to an HCV peptide can be prepared using any technique that provides for the production of antibody molecules by continuous cell lines in culture. These include, but are not limited to, the hybridoma technique originally described by Koehler and Milstein (Nature 256:495-497 (1975); the human B-cell hybridoma technique (Kosbor et al. Immunol Today 4:72 (1983); Cote et al Proc Natl Acad Sci 80:2026-2030 (1983), and the EBV-hybridoma technique Cole et al. Monoclonal Antibodies and Cancer Therapy, Alan R. Liss Inc, New York N.Y., pp 77-96 (1985). In addition, techniques developed for the production of "chimeric antibodies", the splicing of mouse antibody genes to human antibody genes to obtain a molecule with appropriate antigen specificity and biological activity can be used. (Morrison et al. Proc Natl Acad Sci 81:6851-6855 (1984); Neuberger et al. Nature 312:604-608(1984); Takeda et al. Nature 314:452-454(1985). Alternatively, techniques described for the production of single chain antibodies (U.S. Pat No. 4,946,778) can be adapted to produce HCV-specific single chain antibodies. Antibodies can also be produced by inducing *in vivo* production in the lymphocyte population or by screening recombinant immunoglobulin libraries or panels of highly specific binding reagents as disclosed in Orlandi et al., Proc Natl Acad Sci 86: 3833-3837 (1989), and Winter G. and Milstein C; Nature 349:293-299 (1991).

Antibody fragments that contain specific binding sites for an HCV peptide can also be generated. For example, such fragments include, but are not limited to, the F(ab')₂ fragments that can be produced by pepsin digestion of the antibody molecule and the Fab fragments that can be generated by reducing the disulfide bridges of the F(ab')₂ fragments. Alternatively, Fab expression libraries can be constructed to allow rapid and easy identification of monoclonal Fab fragments with the desired specificity. (Huse W. D. et al. Science 256:1275-1281 (1989)).

By one approach, monoclonal antibodies to an HCV peptide are made as follows. Briefly, a mouse is repetitively inoculated with a few micrograms of the selected protein or peptides derived therefrom over a period of a few weeks. The mouse is then sacrificed, and the antibody producing cells of the spleen isolated. The spleen cells are fused in the presence of polyethylene glycol with mouse myeloma cells, and the excess unfused cells destroyed by growth of the system on selective media comprising aminopterin (HAT media). The successfully fused cells are diluted and aliquots of the dilution placed in wells of a microtiter plate where growth of the culture is continued. Antibody-producing clones are identified by detection of antibody in the supernatant fluid of the wells by immunoassay procedures, such as ELISA, as originally described by Engvall, E., *Meth. Enzymol* 70:419 (1980), and derivative methods thereof. Selected positive clones can be expanded and their monoclonal antibody product harvested for use. Detailed procedures for monoclonal antibody production are described in Davis, L. et al. Basic Methods in Molecular Biology Elsevier, New York. Section 21-2.

Polyclonal antiserum containing antibodies to heterogenous epitopes of a single protein can be prepared by immunizing suitable animals with the expressed protein or peptides derived therefrom described above, which can be unmodified or modified to enhance immunogenicity. Effective polyclonal antibody production is affected by many factors related both to the antigen and the host species. For example, small molecules tend to be less immunogenic than others and can require the use of carriers and adjuvant Also, host animals vary in response to site of inoculations and dose, with both inadequate or excessive doses of antigen resulting in low titer antisera. Small doses (ng level) of antigen administered at multiple intradermal sites appears to be most reliable. An effective immunization protocol for rabbits can be found in Vaitukaitis, J. et al. J. Clin Endocrinol. Metab. 33:988-991 (1971).

Booster injections are given at regular intervals, and antiserum harvested when antibody titer thereof, as determined semi-quantitatively, for example, by double immunodiffusion in agar against known concentrations of the antigen, begins to fall. See, for example, Ouchterlony, O. et al., Chap. 19 in: Handbook of Experimental Immunology D. Wier (ed) Blackwell (1973). Plateau concentration of antibody is usually in the range of 0.1 to 0.2 mg/ml of serum (about 12µM). Affinity of the antisera for the antigen is determined by preparing competitive binding curves, as described, for example, by Fisher, D., Chap. 42 in: Manual of Clinical Immunology, 2d Ed. (Rose and Friedman, Eds.) Amer. Soc. For Microbiol., Washington, D.C. (1980). Antibody preparations prepared according to either protocol are useful in quantitative immunoassays that determine concentrations of antigen-bearing substances in biological samples; they are also used semi-quantitatively or qualitatively (e.g., in diagnostic embodiments that identify the presence of HCV in biological samples). The section below describes some of the diagnostic embodiments in greater detail.

### Diagnostics

Generally, diagnostics are classified according to whether a nucleic acid or protein-based assay is used. Some diagnostic assays detect the presence or absence of an embodied HCV nucleic acid sequence in a sample obtained from a patient, whereas, other assays seek to identify whether an embodied HCV peptide is present in a biological sample obtained from a patient Additionally, the manufacture of kits that incorporate the reagents and methods described herein that allow for the rapid detection and identification of HCV are also embodied. These diagnostic kits can include, for example, an embodied nucleic acid probe or antibody, which specifically detects HCV. The detection component of these kits will typically be supplied in combination with one or more of the following reagents. A support capable of absorbing or otherwise binding DNA, RNA, or protein will often be supplied. Available supports include membranes of nitrocellulose, nylon or derivatized nylon that can be characterized by bearing an array of positively charged substituents. One or more restriction enzymes, control reagents, buffers, amplification enzymes, and non-human polynucleotides like calf-thymus or salmon-sperm DNA can be supplied in these kits.

Useful nucleic acid-based diagnostics include, but are not limited to, direct DNA sequencing, Southern Blot analysis, dot blot analysis, nucleic acid amplification, and combinations of these approaches. The starting point for these analysis is isolated or purified nucleic acid from a biological sample obtained from a patient suspected of contracting HCV or a patient at risk of contracting HCV. The nucleic acid is extracted from the sample and can be amplified by RT-PCR and/or DNA amplification using primers that correspond to regions flanking the embodied HCV nucleic acid sequences (e.g., NS3/4A (SEQ. ID, NO.: 16)).

Nucleic acid probes that specifically hybridize with HCV sequences are attached to a support in an ordered array, wherein the nucleic acid probes are attached to distinct regions of the support that do not overlap with each other. Preferably, such an ordered array is designed to be "addressable" where the distinct locations of the probe are recorded and can be accessed as part of an assay procedure. These probes are joined to a support in different known locations. The knowledge of the precise location of each nucleic acid probe makes these "addressable" arrays particularly useful in binding assays. The nucleic acids from a preparation of several biological samples are then labeled by conventional approaches (e.g., radioactivity or fluorescence) and the labeled samples are applied to the array under conditions that permit hybridization.

If a nucleic acid in the samples hybridizes to a probe on the array, then a signal will be detected at a position on the support that corresponds to the location of the hybrid. Since the identity of each labeled sample is known and the region of the support on which the labeled sample was applied is known, an identification of the presence of the polymorphic variant can be rapidly determined. These approaches are easily automated using technology known to those of skill in the art of high throughput diagnostic or detection analysis.

Additionally, an opposite approach to that presented above can be employed. Nucleic acids present in biological samples can be disposed on a support so as to create an addressable array. Preferably, the samples are disposed on the support at known positions that do not overlap. The presence of HCV nucleic acids in each sample is determined by applying labeled nucleic acid probes that complement nucleic acids, which encode HCV peptides, at locations on the array that correspond to the positions at which the biological samples were disposed. Because the identity of the biological sample and its position on the array is known, the identification of a patient that has been infected with HCV can be rapidly determined. These approaches are also easily automated using technology known to those of skill in the art of high throughput diagnostic analysis.

Any addressable array technology known in the art can be employed. One particular embodiment of polynucleotide arrays is known as Genechips™, and has been generally described in US Patent 5,143,854; PCT publications WO 90/15070 and 92/10092. These arrays are generally produced using mechanical synthesis methods or light directed synthesis methods, which incorporate a combination of photolithographic methods and solid phase oligonucleotide synthesis. (Fodor et al., Science, 251:767-777, (1991)). The immobilization of arrays of oligonucleotides on solid supports has been rendered possible by the development of a technology generally identified as "Very Large Scale Immobilized Polymer Synthesis" (VLSPIS™) in which, typically, probes are immobilized in a high density array on a solid surface of a chip. Examples of VLSPIS™ technologies are provided in US Patents 5,143,854 and 5,412,087 and in PCT Publications WO 90/15070, WO 92/10092 and WO 95/11995, which describe methods for forming oligonucleotide arrays through techniques such as light-directed synthesis techniques. In designing strategies aimed at providing arrays of nucleotides immobilized on solid supports, further presentation strategies were developed to order and display the oligonucleotide arrays on the chips in an attempt to maximize hybridization patterns and diagnostic information. Examples of such presentation strategies are disclosed in PCT Publications WO 94/12305, WO 94/11530, WO 97/29212, and WO 97/31256.

A wide variety of labels and conjugation techniques are known by those skilled in the art and can be used in various nucleic acid assays. There are several ways to produce labeled nucleic acids for hybridization or PCR including, but not limited to, oligolabeling, nick translation, end-labeling, or PCR amplification using a labeled nucleotide. Alternatively, a nucleic acid encoding an HCV peptide can be cloned into a vector for the production of an mRNA probe. Such vectors are known in the art, are commercially available, and can be used to synthesize RNA probes *in vitro* by addition of an appropriate RNA polymerase such as T7, T3 or SP6 and labeled nucleotides. A number of companies such as Pharmacia Biotech (Piscataway N.J.), Promega (Madison Wis.), and U.S. Biochemical Corp (Cleveland Ohio) supply commercial kits and protocols for these procedures. Suitable reporter molecules or labels include those radionuclides, enzymes, fluorescent, chemiluminescent, or chromogenic agents, as well as, substrates, cofactors, inhibitors, magnetic particles and the like.

The presence of an HCV peptide in a protein sample obtained from a patient can be detected by using conventional assays, or by means disclosed herein. For example, antibodies that are immunoreactive with the disclosed HCV peptides can be used to screen biological samples for the presence of HCV infection. Antibodies that are reactive to the embodied HCV peptides are used to immunoprecipitate the disclosed HCV peptides from biological samples or are used to react with proteins obtained from a biological sample on Western or Immunoblots. Favored diagnostic embodiments also include enzyme-linked immunosorbant assays (ELISA), radioimmunoassays (RIA), immunomdiometric assays (IRMA) and immunoenzymatic assays (IEMA), including sandwich assays using monoclonal and/or polyclonal antibodies specific for the disclosed HCV peptides. Exemplary sandwich assays are described by David et al., in U.S. Patent Nos.4,376,110 and 4,486,530. Other embodiments employ aspects of the immune-strip technology disclosed in U.S. Patent Nos. 5,290,678; 5,604,105; 5,710,008; 5,744,358; and 5,747,274.

In another protein-based diagnostic, the antibodies described herein are attached to a support in an ordered array, wherein a plurality of antibodies are attached to distinct regions of the support that do not overlap with each other. As with the nucleic acid-based arrays, the protein-based arrays are ordered arrays that are designed to be "addressable" such that the distinct locations are recorded and can be accessed as part of an assay procedure. These probes are joined to a support in different known locations. The knowledge of the precise location of each probe makes these "addressable" arrays particularly useful in binding assays. For example, an addressable array can comprise a support having several regions to which are joined a plurality of antibody probes that specifically recognize HCV peptides present in a biological sample and differentiate the isotype of HCV identified herein.

By one approach, proteins are obtained from biological samples and are then labeled by conventional approaches (e.g., radioactivity, colorimetrically, or fluorescently). The labeled samples are then applied to the array under conditions that permit binding. If a protein in the sample binds to an antibody probe on the array, then a signal will be detected at a position on the support that corresponds to the location of the antibody-protein complex. Since the identity of each labeled sample is known and the region of the support on which the labeled sample was applied is known, an identification of the presence, concentration, and/or expression level can be rapidly determined. That is, by employing labeled standards of a known concentration of HCV peptide, an investigator can accurately determine the protein concentration of the particular peptide in a tested sample and can also assess the expression level of the HCV peptide. Conventional methods in densitometry can also be used to more accurately determine the concentration or expression level of the HCV peptide. These approaches are easily automated using technology known to those of skill in the art of high throughput diagnostic analysis.

An opposite approach to that presented above can be employed. Proteins present in biological samples can be disposed on a support so as to create an addressable array. Preferably, the protein samples are disposed on the support at known positions that do not overlap. The presence of an HCV peptide in each sample is then determined by applying labeled antibody probes that recognize epitopes specific for the HCV peptide. Because the identity of the biological sample and its position on the array is known, an identification of the presence, concentration, and/or expression level of an HCV peptide can be rapidly determined.

That is, by employing labeled standards of a known concentration of HCV peptide, an investigator can accurately determine the concentration of peptide in a sample and from this information can assess the expression level of the peptide. Conventional methods in densitometry can also be used to more accurately determine the concentration or expression level of the HCV peptide. These approaches are also easily automated using technology known to those of skill in the art of high throughput diagnostic analysis. As detailed above, any addressable array technology known in the art can be employed. The section below describes some of the compositions that can have one or more of the embodied HCV nucleic acids or HCV peptides.

### Compositions comprising the embodied HCV nucleic acids or peptides

At least one of the HCV nucleic acids or peptides may be joined to a support. Preferably, these supports are manufactured so as to create a multimeric agent. These multimeric agents provide the HCV peptide or nucleic acid in such a form or in such a way that a sufficient affinity to the molecule is achieved. A multimeric agent having an HCV nucleic acid or peptide can be obtained by joining the desired molecule to a macromolecular support A "support" can be a termed a carrier, a protein, a resin, a cell membrane, or any macromolecular structure used to join or immobilize such molecules. Solid supports include, but are not limited to, the walls of wells of a reaction tray, test tubes, polystyrene beads, magnetic beads, nitrocellulose strips, membranes, microparticles such as latex particles, animal cells, Duracyte®, artificial cells, and others. An HCV nucleic acid or peptide can also be joined to inorganic carriers, such as silicon oxide material (e.g., silica gel, zeolite, diatomaceous earth or aminated glass) by, for example, a covalent linkage through a hydroxy, carboxy or amino group and a reactive group on the carrier.

In several multimeric agents, the macromolecular support has a hydrophobic surface that interacts with a portion of the HCV nucleic acid or peptide by a hydrophobic non-covalent interaction. In some cases, the hydrophobic surface of the support is a polymer such as plastic or any other polymer in which hydrophobic groups have been linked such as polystyrene, polyethylene or polyvinyl. Additionally, HCV nucleic acid or peptide can be covalently bound to carriers including proteins and oligo/polysaccarides (e.g. cellulose, starch, glycogen, chitosane or aminated sepharose). In these later multimeric agents, a reactive group on the molecule, such as a hydroxy or an amino group, is used to join to a reactive group on the carrier so as to create the covalent bond. Additional multimeric agents comprise a support that has other reactive groups that are chemically activated so as to attach the HCV nucleic acid or peptide. For example, cyanogen bromide activated matrices, epoxy activated matrices, thio and thiopropyl gels, nitrophenyl chloroformate and N-hydroxy succinimide chlorformate linkages, or oxirane acrylic supports are used. (Sigma).

Carriers for use in the body, (i.e. for prophylactic or therapeutic applications) are desirably physiological, non-toxic and preferably, non-immunoresponsive. Suitable carriers for use in the body include poly-L-lysine, poly-D, L-alanine, liposomes, and Chromosorb^{®} (Johns-Manville Products, Denver Co.). Ligand conjugated Chromosorb^{®} (Synsorb-Pk) has been tested in humans for the prevention of hemolytic-uremic syndrome and was reported as not presenting adverse reactions. (Armstrong et al. J. Infectious Diseases 171:1042-1045 (1995*)).* For some embodiments, a "naked" carrier (i.e., lacking an attached HCV nucleic acid or peptide) that has the capacity to attach an HCV nucleic acid or peptide in the body of a organism is administered. By this approach, a "prodrug-type" therapy is envisioned in which the naked carrier is administered separately from the HCV nucleic acid or peptide and, once both are in the body of the organism, the carrier and the HCV nucleic acid or peptide are assembled into a multimeric complex.

The insertion of linkers, such as linkers (e.g., "λ linkers" engineered to resemble the flexible regions of λ phage) of an appropriate length between the HCV nucleic acid or peptide and the support are also contemplated so as to encourage greater flexibility of the HCV peptide, hybrid, or binding partner and thereby overcome any steric hindrance that can be presented by the support. The determination of an appropriate length of linker that allows for an optimal cellular response or lack thereof, can be determined by screening the HCV nucleic acid or peptide with varying linkers in the assays detailed in the present disclosure.

A composite support comprising more than one type of HCV nucleic acid or peptide is also envisioned. A "composite support" can be a carrier, a resin, or any macromolecular structure used to attach or immobilize two or more different HCV nucleic acids or peptides. As above, the insertion of linkers, such as λ linker, of an appropriate length between the HCV nucleic acid or peptide and the support is also contemplated so as to encourage greater flexibility in the molecule and thereby overcome any steric hindrance that can occur. The determination of an appropriate length of linker that allows for an optimal cellular response or lack thereof, can be determined by screening the HCV nucleic acid or peptide with varying linkers in the assays detailed in the present disclosure_{.}

The multimeric and composite supports discussed above can have attached multimerized HCV nucleic acids or peptides so as to create a "multimerized-multimeric support" and a "multimerized-composite support", respectively. A multimerized ligand can, for example, be obtained by coupling two or more HCV nucleic acids or peptides in tandem using conventional techniques in molecular biology. The multimerized form of the HCV nucleic acid or peptide can be advantageous for many applications because of the ability to obtain an agent with a higher affinity, for example. The incorporation of linkers or spacers, such as flexible λ, linkers, between the individual domains that make-up the multimerized agent can also be advantageous for some embodiments. The insertion of λ linkers of an appropriate length between protein binding domains, for example, can encourage greater flexibility in the molecule and can overcome steric hindrance. Similarly, the insertion of linkers between the multimerized HCV nucleic acid or peptide and the support can encourage greater flexibility and limit steric hindrance presented by the support. The determination of an appropriate length of linker can be determined by screening the HCV nucleic acids or peptides in the assays detailed in this disclosure.

The disclosure includes genetic vaccines, as described above. Preferably these compositions contain ribavirin and a nucleic acid encoding NS3/4A (**SEQ. ID. NO**.: **17**), NS3 (**SEQ. ID. NO**.: **29**), or a mutant (e.g., **SEQ. ID. NOs**.: **30 - 32 and 43-49**) or a fragment thereof (e.g., **SEQ. ID. NOs**.: **25-27, and 33-42**). The following example describes the preparation of a genetic vaccine suitable for use in humans.

### EXAMPLE 14

An HCV expression plasmid is designed to express the NS3/4A peptide. The NS3/4A coding sequence of NS3/4A-pVAX is removed by digestion with *EcoRI* and *XbaI,* and the isolated fragment is inserted into plasmid A so that it is under the transcriptional control of the CMV promoter and the RSV enhancer element. (*See* U.S. Pat. No. 6,235,888 to Pachuk, et al.). Plasmid backbone A is 3969 base pairs in length; it contains a PBR origin of replication for replicating in *E. coli* and a kanamycin resistance gene. Inserts such as the NS3/4A, are cloned into a polylinker region, which places the insert between and operably linked to the promoter and polyadenylation signal. Transcription of the cloned inserts is under the control of the CMV promoter and the RSV enhancer elements. A polyadenylation signal is provided by the presence of an SV40 poly A signal situated just 3' of the cloning site. An NS3/4A containing vaccine composition is then made by mixing 500µg of the rNS3/4A construct with 1mg of ribavirin.

Said vaccine composition can be used to raise antibodies in a mammal (e.g., mice or rabbits) or can be injected intramuscularly into a human so as to to raise antibodies, preferably a human that is chronically infected with the HCV virus. The recipient preferably receives three immunization boosts of the mixture at 4-week intervals, as well. By the third boost, the titer of antibody specific for HCV will be significantly increased. Additionally, at this time, said subject will experience an enhanced antibody and T-cell mediated immune response against NS3, as evidenced by an increased fraction of NS3 specific antibodies as detected by EIA, and a reduction in viral load as detected by RT-PCR.

NS3/4A fusion proteins or nucleic acids encoding these molecules are disclosed herein. Production and purification of recombinant protein may be facilitated by the addition of auxiliary amino acids to form a "tag". Such tags include, but are not limited to, His-6, Flag, Myc and GST. The tags may be added to the C-terminus, N-terminus, or within the NS3/4A amino acid sequence. Further aspects of the disclosure include NS3/4A fusion proteins with amino or carboxy terminal truncations, or internal deletions, or with additional polypeptide sequences added to the amino or carboxy terminal ends, or added internally. Other aspects include NS3/4A fusion proteins, or truncated or mutated versions thereof, where the residues of the NS3/4A proteolytic cleavage site have been substituted. Such substitutions include, but are not limited to, sequences where the P1' site is a Ser, Gly, or Pro, or the P1 position is an Arg, or where the P8 to P4' sequence is Ser-Ala-Asp-Leu-Glu-Val-Val-Thr-Ser-Thr-Trp-Val (**SEQ. ID.**

**NO**.: **28**).

Other aspects concern an immunogen comprising the NS3/4A fusion protein, or a truncated or modified version thereof, capable of eliciting an enhanced immune response against NS3. The immunogen can be provided in a substantially purified form, which means that the immunogen has been rendered substantially free of other proteins, lipids, carbohydrates or other compounds with which it naturally associates. Disclosure is made of vaccine compositions comprising the NS3/4A fusion protein (**SEQ. ID. NO**.: **17**), or a truncated or mutated version thereof (e.g., **SEQ. ID. NOS**.: **29- 32 and 43-49**) or a fragment thereof (e.g., **SEQ. ID. NOs**.: **25-**27, and 33-42), and an adjuvant, such as ribavirin. The following example describes one approach to prepare a vaccine composition comprising the NS3/4A fusion protein and an adjuvant

### Example 15

To generate a tagged NS3/4A construct, the NS3/4A coding sequence of NS3/4A-pVAX is removed by digestion with *EcoRI* and *XbaI,* and the isolated fragment is inserted into an Xpress vector (Invitrogen). The Xpress vector allows for the production of a recombinant fusion protein having a short N-terminal leader peptide that has a high affinity for divalent cations. Using a nickel-chelating resin (Invitrogen), the recombinant protein can be purified in one step and the leader can be subsequently removed by cleavage with enterokinase. A preferred vector is the pBlueBacHis2 Xpress. The pBlueBacHis2 Xpress vector is a Baculovirus expression vector containing a multiple cloning site, an ampicillin resistance gene, and a *lac z* gene. Accordingly, the digested amplification fragment is cloned into the pBlueBacHis2 Xpress vector and SF9 cells are infected. The expression protein is then isolated or purified according to the maufacturer's instructions. An NS3/4A containing vaccine composition is then made by mixing 100µg of the rNS3/4A with 1mg of ribavirin.

Said vaccine composition can be used to raise antibodies in a mammal (e.g., mice or rabbits) or can be injected intramuscularly into a human so as to to raise antibodies, preferably a human that is chronically infected with the HCV virus. The recipient preferably receives three immunization boosts of the mixture at 4-week intervals. By the third boost, the titer of antibody specific for HCV will be significantly increased. Additionally, at this time, said subject will experience an enhanced antibody and T-cell mediated immune response against NS3, as evidenced by an increased fraction of NS3 specific antibodies as detected by EIA, and a reduction in viral load as detected by RT-PCR. The section below provides more explanation concerning the methods of using the compositions described herein.

### Methods of using compositions comprising ribavirin and an antigen

Routes of administration of the vaccines described herein include, but are not limited to, transdermal, parenteral, gastrointestinal, transbronchial, and transalveolar. Transdermal administration can be accomplished by application of a cream, rinse, gel, or other compounds capable of allowing ribavirin and antigen to penetrate the skin. Parenteral routes of administration include, but are not limited to, electrical or direct injection such as direct injection into a central venous line, intravenous, intramuscular, intraperitoneal, intradermal, or subcutaneous injection. Gastrointestinal routes of administration include, but are not limited to, ingestion and rectal. Transbronchial and transalveolar routes of administration include, but are not limited to; inhalation, either via the mouth or intranasally.

Compositions having ribavirin and an antigen that are suitable for transdermal administration include, but are not limited to, pharmaceutically acceptable suspensions, oils, creams, and ointments applied directly to the skin or incorporated into a protective carrier such as a transdermal device ("transdermal patch"). Examples of suitable creams, ointments, etc. can be found, for instance, in the Physician's Desk Reference. Examples of suitable transdermal devices are described, for instance, in U.S. Patent No. 4,818,540 issued April 4, 1989 to Chinen, et al.

Compositions having ribavirin and an antigen that are suitable for parenteral administration include, but are not limited to, pharmaceutically acceptable sterile isotonic solutions. Such solutions include, but are not limited to, saline, phosphate buffered saline and oil preparations for injection into a central venous line, intravenous, intramuscular, intraperitoneal, intradermal, or subcutaneous injection.

Compositions having ribavirin and an antigen that are suitable for transbronchial and transalveolar administration include, but not limited to, various types of aerosols for inhalation. Devices suitable for transbronchial and transalveolar administration of these are also embodiments. Such devices include, but are not limited to, atomizers and vaporizers. Many forms of currently available atomizers and vaporizers can be readily adapted to deliver vaccines having ribavirin and an antigen.

Compositions having ribavirin and an antigen that are suitable for gastrointestinal administration include, but not limited to, pharmaceutically acceptable powders, pills or liquids for ingestion and suppositories for rectal administration.

The gene constructs described herein, in particular, may be administered by means including, but not limited to, traditional syringes, needleless injection devices, or "microprojectile bombardment gene guns". Alternatively, the genetic vaccine may be introduced by various means into cells that are removed from the individual. Such means include, for example, *ex vivo* transfection, electroporation, microinjection and microprojectile bombardment. After the gene construct is taken up by the cells, they are reimplanted into the individual. It is contemplated that otherwise non-immunogenic cells that have gene constructs incorporated therein can be implanted into the individual even if the vaccinated cells were originally taken from another individual.

According to some aspects of the disclosure, the gene construct is administered to an individual using a needleless injection device. According to some embodiments, the gene construct is simultaneously administered to an individual intradermally, subcutaneously and intramuscularly using a needleless injection device. Needleless injection devices are well known and widely available. One having ordinary skill in the art can, following the teachings herein, use needleless injection devices to deliver genetic material to cells of an individual. Needleless injection devices are well suited to deliver genetic material to all tissue. They are particularly useful to deliver genetic material to skin and muscle cells. In some embodiments, a needleless injection device may be used to propel a liquid that contains DNA molecules toward the surface of the individual's skin. The liquid is propelled at a sufficient velocity such that upon impact with the skin the liquid penetrates the surface of the skin, permeate the skin and muscle tissue therebeneath. Thus, the genetic material is simultaneously administered intradermally, subcutaneously and intramuscularly. In some aspects, a needleless injection device may be used to deliver genetic material to tissue of other organs in order to introduce a nucleic acid molecule to cells of that organ.

The vaccines containing ribavirin and an antigen can be used to treat and prevent a vast spectrum of diseases and can enhance the immune response of an animal to an antigen. As one of skill in the art will appreciate, conventional vaccines have been administered to subjects in need of treatment or prevention of bacterial diseases, viral diseases, fungal diseases, and cancer. Because the vaccines described herein include conventional vaccines, which have been modified by the addition of ribavirin, the methods described herein include the treatment and prevention of a disease using a vaccine that comprises an antigen and ribavirin.

Preferred disclosed aspects concern methods of treating or preventing hepatitis infection. In these aspects, an animal in need is provided a hepatitis antigen (e.g., a peptide antigen or nucleic acid-based antigen) and an amount of ribavirin sufficient to exhibit an adjuvant activity in said animal. Accordingly, an animal can be identified as one in need by using currently available diagnostic testing or clinical evaluation. The range of hepatitis viral antigens that can be used with these embodiments is diverse. Preferred hepatitis viral antigens include an HBV antigen, an HAV antigen, an HCV antigen, nucleic acids encoding these antigens, or any combination thereof. Highly preferred embodiments include an HBV antigen selected from the group consisting of hepatitis B surface antigen (HBsAg), hepatitis core antigen (EIBcAg), and hepatitis E antigen (HBeAg), in particular, the peptide and nucleic acid-based antigens described *supra.* The ribavirin and antigen can be provided separately or in combination, and other adjuvants (e.g., oil, alum, or other agents that enhance an immune response) can also be provided to the animal in need. Thus, preferred embodiments include methods of treating or preventing hepatitis in an animal (e.g., HBV) by identifying an infected animal or an animal at risk of infection and providing said animal a hepatitis antigen (e.g., HBsAg, HBcAg, and HBeAg) and an amount of ribavirin sufficient to exhibit adjuvant activity

Other disclosed aspects include methods of enhancing an immune response to an antigen by providing an animal in need with an amount of ribavirin that is effective to enhance said immune response. In these aspects, an animal in need of an enhanced immune response to an antigen is identified by using currently available diagnostic testing or clinical evaluation. Oftentimes these individuals will be suffering from a disease (e.g., bacterial, fungal, mold, viral, or cancer) or are at risk from contracting the disease. However, an animal in need of an enhanced immune response can be an animal that has been poisoned (e.g., bit by a poisonous insect or animal) or that has been exposed to a toxin or other toxic compound. Once identified, these animals are provided an appropriate antigen and an amount of-ribavirin effective to enhance an immune response in the animal.

As above, the hepatitis viral antigens that can be used with these aspects of the disclosure include, but are not limited to, an HBV antigen, an HAV antigen, an HCV antigen, a nucleic acid encoding these molecules, or any combination thereof. Highly preferred aspects include, an HBV antigen selected from the group consisting of hepatitis B surface antigen (HBsAg), hepatitis core antigen (HBcAg), and hepatitis E antigen (HBeAg), in particular, the peptide and nucleic acid-based antigens described *supra*. The ribavirin and antigen can be provided separately or in combination, and other adjuvants (e.g., oil, alum, or other agents that enhance an immune response) can also be provided to the animal in need. Thus, preferred aspects include methods of enhancing an immune response to a hepatitis antigen (e.g., HBV) by identifying an animal in need and providing the animal a hepatitis antigen (e.g., HBsAg, HBcAg, and HBeAg) and an amount of ribavirin that is effective to enhance an immune response in the animal..

By one approach, for example, an uninfected individual is provided with the above mentioned vaccine compositions in an amount sufficient to elicit a cellular and humoral immune response to NS3 so as to protect said individual from becoming infected with HCV. In another aspect, an HCV-infected individual is identified and provided with a vaccine composition comprising ribavirin and NS3 in an amount sufficient to enhance the cellular and humoral immune response against NS3 so as to reduce or eliminate the HCV infection. Such individual may be in the chronic or acute phase of the infection. In yet another aspect, an HCV-infected individual suffering from HCC is provided with a composition comprising ribavirin and the NS3/4A fusion gene in an amount sufficient to elicit a cellular and humoral immune response against NS3-expressing tumor cells.

The disclosure made herein can be characterized by the following items:
1. A composition comprising ribavirin and the nucleic acid of SEQ. ID. NO.: 16.
2. A composition comprising ribavrin and the peptide of SEQ. ID. NO.: 17.
3. A composition comprising ribavrin and the nucleic acid of SEQ. ID. NO.: 13 or a fragment thereof at least 18 consecutive nucleotides in length.
4. A composition comprising ribavirin and the peptide of SEQ. ID. NO.:1 or a fragment thereof at least 6 consecutive amino acids in length.
5. A composition comprising ribavirin and an antigen.
6. The composition of 5, wherein said antigen is a nucleic acid.
7. The composition of 5, wherein said antigen is a peptide.
8. The composition of 6, wherein said nucleic acid is derived from a virus selected from the group consisting of hepatitis A virus (HAV), hepatitis B virus (HBV), and hepatitis C virus (HCV).
9. The composition of 7, wherein said peptide is derived from a virus selected from the group consisting of hepatitis A virus(HAV), hepatitis B virus (HBV), and hepatitis C virus (HCV).
10. The composition of 5, wherein said antigen is a nucleic acid or a peptide corresponding to an antigen selected from the group consisting of hepatitis B surface antigen (HBsAg), hepatitis core antigen (HBcAg), and hepatitis E antigen (HBeAg).
11. The composition of 7, wherein said peptide comprises at least three consecutive amino acids of a sequence selected from the group consisting of SEQ. ID. NOs.: 1-12.
12. The composition of 6, wherein said nucleic acid comprises at least 9 consecutive nucleotides of a sequence selected from the group consisting of SEQ. ID. NOs.: 1315.
13. A method of enhancing an immune response to a hepatitis C antigen comprising: identifying an animal in need of an enhanced immune response to a hepatitis C antigen; and providing to said animal a composition comprising ribavirin and the nucleic acid of SEQ. ID. NO.: 16.
14. A method of enhancing an immune response to a hepatitis C antigen comprising: identifying an animal in need of an enhanced immune response to a hepatitis C antigen; and providing to said animal a composition comprising ribavirin and the peptide of SEQ. ID. NO:: 17.
15. A method of enhancing an immune response to a hepatitis C antigen comprising: identifying an animal in need of an enhanced immune response to a hepatitis C antigen; and providing to said animal a composition comprising ribavirin and the nucleic acid of SEQ. ID. NO.: 13 or a fragment thereof at least 18 consecutive nucleotides in length.
16. A method of enhancing an immune response to a hepatitis C antigen comprising: identifying an animal in need of an enhanced immune response to a hepatitis C antigen; and providing to said animal a composition comprising ribavirin and the peptide of SEQ. ID. NO.: 1 or a fragment thereof at least 6 consecutive amino acids in length.
17. A method of making a vaccine comprising: providing ribavirin; providing the nucleic acid of SEQ. ID. NO.: 16; and mixing said ribavirin and said nucleic acid so as to formulate said vaccine.
18. A method of making a vaccine comprising: providing ribavirin; providing the peptide of SEQ. ID. NO.: 17; and mixing said ribavirin and said peptide so as to formulate said vaccine.
19. A method of making vaccine comprising: providing ribavirin; providing the nucleic acid of SEQ. ID. NO.: 13 or a fragment thereof at least at least 18 consecutive nucleotides in length; and mixing said ribavirin and said nucleic acid so as to formulate said vaccine.
20. A method of making a vaccine comprising: providing ribavirin; providing the nucleic acid of SEQ. ID. NO.: 1 or a fragment thereof at least 6 consecutive amino acids in length; and mixing said ribavirin and said nucleic acid so as to formulate said vaccine.

## Claims

1. A purified or isolated nucleic acid consisting of the sequence of SEQ ID. NO.: 16 or the complement thereof

2. A purified or isolated nucleic acid encoding the peptide sequence of SEQ ID. NO.: 17.

3. A purified or isolated nucleic acid comprising SEQ ID. NO.: 16.

4. A purified or isolated nucleic acid comprising a nucleic acid encoding the peptide sequence of SEQ ID. No.: 17.

5. A purified or isolated nucleic acid comprising at least 100,200, 500, or 1000 consecutive nucleotides of the purified or isolated nucleic acid of anyone of claims 1- 2.

6. A vector comprising the purified or isolated nucleic acid of any one of claims 1-5.

7. An isolated cell comprising the purified or isolated nucleic acid of anyone of claims 1-5.

8. An immunogenic composition comprising the purified or isolated nucleic acid of anyone of claims 1-5.

9. A composition comprising the purified or isolated nucleic acid of any one of claims 1-5.

10. An isolated cell comprising the vector of claim 6.

11. An immunogenic composition comprising the vector of claim 6.

12. A composition comprising the vector of claim 6.

13. The immunogenic composition of claim 8 or 11, further comprising an adjuvant.

## Patentansprüche

1. Gereinigte oder isolierte Nukleinsäure, bestehend aus der Sequenz von SEQ. ID. Nr.: 16 oder dem Komplement derselben.

2. Gereinigte oder isolierte Nukleinsäure, welche die Peptidsequenz von SEQ. ID. Nr.: 17 codiert.

3. Gereinigte oder isolierte Nukleinsäure, die SEQ. ID. Nr.: 16 umfasst.

4. Gereinigte oder isolierte Nukleinsäure, die eine Nukleinsäure umfasst, welche die Peptidsequenz von SEQ. ID. Nr.: 17 codiert.

5. Gereinigte oder isolierte Nukleinsäure, die wenigstens 100, 200, 500 oder 1000 aufeinanderfolgende Nukleotide der gereinigten oder isolierten Nukleinsäure gemäß einem der Ansprüche 1 - 2 umfasst.

6. Vektor, der die gereinigte oder isolierte Nukleinsäure gemäß einem der Ansprüche 1- 5 umfasst.

7. Isolierte Zelle, welche die gereinigte oder isolierte Nukleinsäure gemäß einem der Ansprüche 1 - 5 umfasst.

8. Immunogene Zusammensetzung, welche die gereinigte oder isolierte Nukleinsäure gemäß einem der Ansprüche 1 - 5 umfasst.

9. Zusammensetzung, welche die gereinigte oder isolierte Nukleinsäure gemäß einem der Ansprüche 1 - 5 umfaßt.

10. Isolierte Zelle, die den Vektor gemäß Anspruch 6 umfasst.

11. Immunogene Zusammensetzung, die den Vektor gemäß Anspruch 6 umfasst.

12. Zusammensetzung, die den Vektor gemäß Anspruch 6 umfasst.

13. Immunogene Zusammensetzung gemäß Anspruch 8 oder 11, die weiterhin ein Adjuvans umfasst.

## Revendications

1. Un acide nucléique purifié ou isolé constitué de la séquence de SEQ.ID.No.:16 ou le complément de celle-ci.

2. Un acide nucléique purifié ou isolé encodant la séquence peptidique de SEQ.ID.No.:17.

3. Un acide nucléique purifié ou isolé comprenant SEQ.ID.No.:16.

4. Un acide nucléique purifié ou isolé comprenant un acide nucléique encodant la séquence peptidique de SEQ.ID.No.:17.

5. Un acide nucléique purifié ou isolé comprenant au moins 100, 200, 500 ou 1000 nucléotides consécutifs de l'acide nucléique purifié ou isolé de l'une des revendications 1-2.

6. Un vecteur comprenant l'acide nucléique purifié ou isolé de l'une des revendications 1-5.

7. Une cellule isolée comprenant l'acide nucléique purifié ou isolé de l'une des revendications 1-5.

8. Une composition immunogène comprenant l'acide nucléique purifié ou isolé de l'une des revendications 1-5.

9. Une composition comprenant l'acide nucléique purifié ou isolé de l'une des revendications 1-5.

10. Une cellule isolée comprenant le vecteur de la revendication 6.

11. Une composition immunogène comprenant le vecteur de la revendication 6.

12. Une composition comprenant le vecteur de la revendication 6.

13. La composition immunogène de la revendication 8 ou 11, comprenant de plus un adjuvant.
